# EUROPÄISCHE PATENTSCHRIFT

(11) **EP 2 678 035 B1**
(45) Veröffentlichungstag und Bekanntmachung des Hinweises auf die Patenterteilung: **10.04.2019**
(21) Anmeldenummer: 12706540.7
(22) Anmeldetag: 23.02.2012
(51) Int. Cl.: A61K 41/00, C07C 217/10, C07D 213/20, C07C 45/75, C07C 49/813, C07C 271/16, C07C 271/20, C07C 279/10, C07C 279/12, C07C 279/24, C07C 225/16, C07D 295/116, C07D 211/58, A61K 31/137, A61K 31/14, A61K 8/41, A61K 8/43, A61Q 11/02, A61L 2/08, A61L 2/10

(54) **PHENALEN-1-ON-DERIVATE, VERFAHREN ZUR HERSTELLUNG UND VERWENDUNG DERSELBEN**
PHENALENE-1-ONE DERIVATIVES, METHOD FOR PRODUCING SAME AND USE THEREOF
DÉRIVÉS DE PHÉNALÈNE-1-ONE, LEUR PROCÉDÉ DE PRODUCTION ET LEUR UTILISATION

(30) Priorität: 24.02.2011 DE 102011012343
(43) Veröffentlichungstag der Anmeldung: 01.01.2014
(73) Patentinhaber: TriOptoTec GmbH, 93053 Regensburg (DE)
(72) Erfinder: BÄUMLER, Wolfgang, 84085 Langquaid (DE); FELGENTRÄGER, Ariane, 93059 Regensburg (DE); LEHNER, Karin, A-6060 Hall in Tirol (AT); MAISCH, Tim, 90425 Nürnberg (DE); REGENSBURGER, Johannes, 93092 Barbing (DE); SANTARELLI, Francesco, 51105 Köln (DE); SPÄTH, Andreas, 93073 Neutraubling (DE)
(74) Vertreter: Walcher, Armin
(86) Internationale Anmeldenummer: PCT/EP2012/053062
(87) Internationale Veröffentlichungsnummer: WO 2012/113860

(56) Entgegenhaltungen:
- EP-A1- 0 341 018
- DE-A1- 2 224 371
- GB-A- 2 337 530
- US-A1- 2007 110 672
- CRISTINA FLORS ET AL: "Light and Singlet Oxygen in Plant Defense Against Pathogens: Phototoxic Phenalenone Phytoalexins +", ACCOUNTS OF CHEMICAL RESEARCH, Bd. 39, Nr. 5, 1. Mai 2006 (2006-05-01), Seiten 293-300, XP55035752, ISSN: 0001-4842, DOI: 10.1021/ar0402863
- NASSER K. THALLAJ ET AL: "The Design of Metal Chelates with a Biologically Related Redox-Active Part: Conjugation of Riboflavin to Bis(2-pyridylmethyl)amine Ligand and Preparation of a Ferric Complex", EUROPEAN JOURNAL OF INORGANIC CHEMISTRY, Bd. 2007, Nr. 1, 1. Januar 2007 (2007-01-01), Seiten 44-47, XP55034009, ISSN: 1434-1948, DOI: 10.1002/ejic.200600789
- MICHAEL R. DETTY ET AL: "Current Clinical and Preclinical Photosensitizers for Use in Photodynamic Therapy", JOURNAL OF MEDICINAL CHEMISTRY, Bd. 47, Nr. 16, 1. Juli 2004 (2004-07-01), Seiten 3897-3915, XP55034032, ISSN: 0022-2623, DOI: 10.1021/jm040074b
- ESTHER OLIVEROS ET AL: "Photochemistry of the singlet oxygen [O2(1[Delta]g)] sensitizer perinaphthenone (phenalenone) in N,N'-dimethylacetamide and 1,4-dioxane", NEW JOURNAL OF CHEMISTRY, Bd. 23, Nr. 1, 1. Januar 1999 (1999-01-01) , Seiten 85-93, XP55035753, ISSN: 1144-0546, DOI: 10.1039/a804054k

## Beschreibung

Die vorliegende Erfindung betrifft 1H-Phenalen-1-on-Derivate, deren Herstellung und Verwendung.

Das aktive oder passive Eindringen, Anhaften und Vermehren von Krankheitserregern in einen Wirt wird als Infektion bezeichnet. Quellen für infektiöse Partikel treten überall auf. So wird beispielsweise der menschliche Körper von einer großen Anzahl von Mikroorganismen besiedelt, die im Regelfall durch den normalen Metabolismus und ein intaktes Immunsystem unter Kontrolle gehalten werden. Allerdings kann es, beispielsweise bei einer Schwächung des Immunsystems, zu einer starken Vermehrung der Krankheiterreger kommen und je nach Art des Erregers zu unterschiedlichen Krankheitssymptomen. Die Medizin hält für viele Erreger-bedingte Krankheiten spezifische Gegenmittel bereit, beispielsweise Antibiotika gegen Bakterien oder Antimikotika gegen Pilze oder Virustatika gegen Viren. Allerdings ist bei der Verwendung dieser Gegenmittel vermehrt das Auftreten von resistenten Krankheitserregern zu beobachten, die teilweise gleichzeitig gegen mehrere Gegenmittel Resistenzen aufweisen. Durch das Auftreten dieser resistenten oder multiresistenten Krankheitserreger hat sich die Therapie von Infektionserkrankungen zunehmend erschwert. Die klinische Konsequenz der Resistenz zeigt sich durch ein Versagen der Behandlung, vor allem bei immunsupprimierten Patienten.

Neue Ansatzpunkt zur Bekämpfung von resistenten bzw. multiresistenten Krankheitskeimen sind daher einerseits die Suche nach neuen Gegenmitteln, beispielsweise Antibiotika oder Antimikotika, und andererseits die Suche nach alternativen Inaktivierungsmöglichkeiten.

Als alternatives Verfahren hat sich die photodynamische Inaktivierung von Mikroorganismen bewährt. Bei der photodynamischen Inaktivierung von Mikroorganismen spielen zwei verschiedene photooxidative Prozesse eine entscheidende Rolle. Voraussetzung für den Ablauf einer photooxidativen Inaktivierung ist einerseits das Vorhandensein einer ausreichenden Menge Sauerstoff und andererseits die Lokalisierung eines so genannten Photosensibilisators, welcher durch Licht einer entsprechenden Wellenlänge angeregt wird. Der angeregte Photosensibilisator kann die Bildung von reaktiven Sauerstoffspezies (ROS) bewirken, wobei einerseits Radikale, beispielsweise Superoxidanionen, Wasserstoffperoxid oder Hydroxylradikale, und/oder andererseits angeregter molekularer Sauerstoff, beispielsweise Singulett-Sauerstoff, gebildet werden können.

Bei beiden Reaktionen steht die Photooxidation von spezifischen Biomolekülen, die sich in direkter Nachbarschaft zu den reaktiven Sauerstoffspezies (ROS) befindet, im Vordergrund. Dabei findet insbesondere eine Oxidation von Lipiden und Proteinen statt, die beispielsweise als Bestandteile der Zellmembran von Mikroorganismen vorkommen. Durch die Zerstörung der Zellmembran wiederum kommt es zur Inaktivierung der betreffenden Mikroorganismen. Für Viren und Pilze wird ein ähnlicher Eliminationsprozess angenommen.

Beispielsweise werden durch Singulett-Sauerstoff alle Moleküle angegriffen.

Besonders anfällig für eine Schädigung sind allerdings ungesättigte Fettsäuren in den Membranen von Bakterien. Gesunde, körpereigene Zellen verfügen über eine zelluläre Abwehr gegen Angriffe von freien Radikalen, die so genannten Katalasen oder Superoxiddismutasen. Daher können gesunde, körpereigene Zellen eine Schädigung durch reaktive Sauerstoffspezies (ROS), beispielsweise Radikale oder Singulett-Sauerstoff, entgegen wirken.

Aus dem Stand der Technik, beispielsweise Maisch, T. ("A new strategy to destroy antibiotic resistant microorganisms: antimicrobial photodynamic treatment", Mini Rev. Med. Chem. 2009, 9(8), Seiten 974 bis 983), sind zahlreiche Photosensibilisatoren bekannt, die beispielsweise aus der Gruppe der Porphyrine und deren Derivate oder Phthalocyanine und deren Derivate oder Fullerene und deren Derivate oder Derivate der Phenothiazininium-Struktur, wie beispielsweise Methylenblau oder Toluidinblau, oder Vertreter der Phenoxazinium-Reihe, wie beispielsweise Nile blue, stammen. Die in der WO 93/21992 A1 beschriebene Photodynamik von Methylenblau bzw. Toluidinblau gegenüber Bakterien wird beispielsweise bereits in der Zahnheilkunde eingesetzt.

Bei den aus dem Stand der Technik bekannten Photosensibilisatoren handelt es sich zumeist um Substanzen mit einer relativ komplexen Molekülstruktur und daher aufwendigen Herstellungsverfahren.

Es ist bekannt, dass 1H-Phenalen-1-on und sulfoniertes 1H-Phenalen-1-on hohe Ausbeuten an Singulett-Sauerstoff haben, wobei jedoch die Affinität zu Mikroorganismen gering ist. Es ist weiterhin bekannt, dass Singulett-Sauerstoff lediglich über eine geringe Entfernung diffundieren kann, bevor er reagiert oder abgebaut wird. Daher ist die Inaktivierung von Mikroorganismen durch 1H-Phenalen-1-on und sulfoniertem 1H-Phenalen-1-on unzureichend.

Die US 2007/0110672 A1 offenbart unter Anderem Borsäure-haltige Phenalen-1-on-Verbindungen als Nachweismolekül zur Verwendung in einem in-vivo Glukose-Biosensor.

Die Nichtpatentliteratur Flors, C. und Nonell, S. (Acc. Chem. Res. 39(5), 2006, Seiten 293 bis 300) betrifft die Bereitstellung von phototoxischen Phenalenonen als Phytoalexine.

Die EP 0 341 018 A1 betrifft Nachweisreagenzien für Oxidase-Tests.

Aus der GB 2 337 530 A sind Haarfärbemittel auf Basis von Phenalenonderivaten bekannt, bei denen die Ringkohlenstoffatome direkt mit einer Aminodialkylgruppe substituiert sein können.

Die DE 2 224 371 offenbart eine photoempfindliche Zubereitung.

Die Nichtpatentliteratur Thallaj, N.K. et al. (Eur. J. Inorg. Chem. 2007, Seiten 44 bis 47) betrifft Riboflavinderivate bei der Herstellung von Eisenkomplexen.

Die Nichtpatentliteratur Detty, M.R. et al. (J. Med. Chem. Vol. 47(16), 2004, Seiten 3897-3915) betrifft die Verwendung von Photosensibilisatoren bei der photodynamischen Therapie.

Die Nichtpatentliteratur Oliveros, E. et al. (New J. Chem. 1999, Seiten 85 bis 93) beschreibt die photophysikalischen Eigenschaften von Phenalen-1-on in N,N'-dimethylacetamid und 1,4-Dioxan.

Aufgabe der vorliegenden Erfindung ist es daher, neue Photosensibilisatoren bereitzustellen, die Mikroorganismen effizienter inaktivieren.

Die Aufgabe der vorliegenden Erfindung wird gelöst durch die Bereitstellung einer Verbindung nach Anspruch 1 mit der Formel (1): 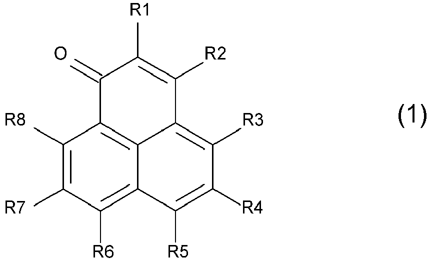 wobei R2 bis R8 Wasserstoff bedeutet und wobei R1 den Rest -(CH₂)ₖ-X bedeutet, wobei k eine ganze Zahl von 1 bis 4, weiter bevorzugt 1, ist und wobei X ein organischer Rest, der a) wenigstens ein neutrales, protonierbares Stickstoffatom und/oder b) wenigstens ein positiv geladenes Stickstoffatom enthält, ist.

Bei der erfindungsgemäßen Verbindung mit der Formel (1) handelt es sich um ein 1H-Phenalen-1-on-Derivat, das im folgenden auch so bezeichnet wird.

Des Weiteren wird die Aufgabe gelöst durch die Bereitstellung eines Verfahrens nach Anspruch 12 zur Herstellung der Verbindung nach Formel (1), wobei das Verfahren folgende Schritte umfasst:
(A1) Umsetzen von 1H-Phenalen-1-on mit wenigstens einem Alkylierungsagens der Formel Y-(CH₂)ₕ-Z, wobei Y aus der Gruppe, die aus H, Cl, Br, I, p-Toluolsulfonyl (OTs), Methansulfonyl (OMs), OH und Alkyl₂S⁺, vorzugsweise H, Cl, Br und I, besteht, wobei Alkyl gleich oder unabhängig voneinander verschieden sein kann und vorzugsweise Methyl, Ethyl, Propyl oder Butyl bedeutet, ausgewählt wird, optional in Gegenwart eines Katalysators, vorzugsweise Lewis-Säure oder Broenstedt-Säure, unter Erhalt einer Verbindung mit der Formel (58): 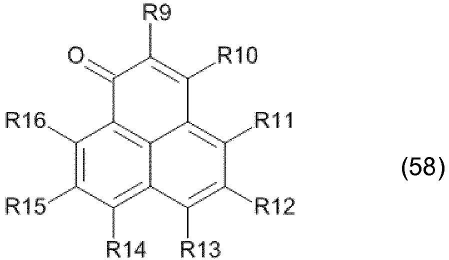 wobei R10 bis R16 jeweils Wasserstoff bedeuten und wobei R9 den Rest -(CH₂)ₕ-Z, wobei h eine ganze Zahl von 1 bis 4, weiter bevorzugt 1, bedeutet und wobei Z aus der Gruppe, die aus Cl, Br, I, OTs, OMs, OH und Alkyl₂S⁺ besteht, wobei Alkyl gleich oder unabhängig voneinander verschieden sein kann und vorzugsweise Methyl, Ethyl, Propyl oder Butyl bedeutet, ausgewählt wird, bedeutet, oder
(A2) Umsetzen von 1H-Phenalen-1-on mit Formaldehyd und wenigstens einer Halogenwasserstoffsäure, optional in Gegenwart eines Katalysators, unter Erhalt einer Verbindung mit der Formel (58): 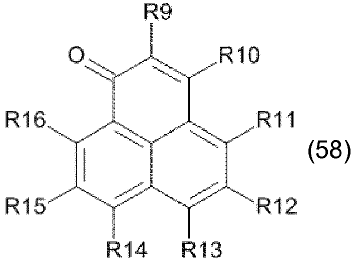 wobei R10 bis R16 jeweils Wasserstoff bedeuten und wobei R9 den Rest -CH₂-W bedeutet und wobei W aus der Gruppe, die aus Cl, Br und I besteht, ausgewählt wird,
(B) Umsetzen der in Schritt (A1) oder (A2) erhaltenen Verbindung mit der Formel (58) mit einer organischen Verbindung, die a) wenigstens ein neutrales, protonierbares Stickstoffatom und/oder b) wenigstens ein positiv geladenes Stickstoffatom enthält, optional in Gegenwart einer Base, und optional
(C) Entfernen vorhandener Aminoschutzgruppen unter Erhalt des erfindungsgemäßen 1H-Phenalen-1-on-Derivats der Formel (1).

Bei der in Schritt (A2) verwendeten Halogenwasserstoffsäure handelt es sich vorzugsweise um HCl, HBr, HI oder Mischungen davon. Als sehr geeignete Halogenwasserstoffsäure hat sich HCl erwiesen. Vorzugsweise handelt es sich bei Schritt (A2) bei dem Rest -CH₂-W um -CH₂-Cl.

Vorzugsweise wird das Alkylierungsagens gemäß Schritt (A2) in situ erzeugt. Hierbei wird der Rest R beispielsweise durch Chlormethylierung, wahlweise mit Reagenzien wie Phosphorsäure, Chlorwasserstoffsäure und Formaldehyd bei erhöhter Temperatur eingeführt. Weiterhin wird die Aufgabe gelöst durch die Bereitstellung einer pharmazeutischen Zusammensetzung, enthaltend wenigstens eine Verbindung mit der Formel (1) oder einem pharmakologisch verträglichen Salz und/oder Ester und/oder Komplex davon, sowie durch die Bereitstellung eines beschichteten Gegenstandes, dessen Oberfläche wenigstens eine Verbindung mit der Formel (1) aufweist.

Weiterhin wird die Aufgabe gelöst durch die Verwendung der Verbindung mit der Formel (1) bei der Inaktivierung von Mikroorganismen.

Weitere bevorzugte Ausführungsformen der vorliegenden Erfindung sind in den abhängigen Ansprüchen beschrieben.

Als "Photosensibilisator" werden erfindungsgemäß Verbindungen verstanden, die elektromagnetische Strahlung, vorzugsweise sichtbares Licht, UV-Licht und/oder Infrarotlicht, absorbieren und sodann reaktive Sauerstoffspezies (ROS), vorzugsweise freie Radikale und/oder Singulett-Sauerstoff, aus Triplett-Sauerstoff erzeugen.

Unter dem Begriff "photodynamische Therapie" wird erfindungsgemäß die lichtinduzierte Inaktivierung von Zellen oder Mikroorganismen verstanden.

Unter dem Begriff "Inaktivierung" wird erfindungsgemäß die Reduzierung der Lebensfähigkeit oder die Zerstörung eines Mikroorganismus, vorzugsweise dessen Zerstörung, verstanden. Eine lichtinduzierte Inaktivierung kann beispielsweise durch Verringerung der Anzahl von Mikroorganismen nach Bestrahlung einer definierten Ausgangsmenge dieser Mikroorganismen in Gegenwart wenigstens einer erfindungsgemäßen Verbindung mit der Formel (1) bestimmt werden.

Erfindungsgemäß wird unter einer Reduzierung der Lebensfähigkeit verstanden, dass die Anzahl der Mikroorganismen um wenigstens 99,0 %, vorzugsweise um wenigstens 99,9 %, weiter bevorzugt um wenigstens 99,99 %, weiter bevorzugt um wenigstens 99,999 %, noch weiter bevorzugt um wenigstens 99,9999 %, verringert wird. Äußerst bevorzugt wird die Anzahl der Mikroorganismen um mehr als 99,9 bis 100 %, vorzugsweise um mehr als 99,99 bis 100 %, verringert.

Vorzugsweise wird die Reduzierung der Anzahl der Mikroorganismen gemäß Boyce, J.M. und Pittet, D. ("Guidelines for hand hygiene in healthcare settings. Recommendations of the Healthcare Infection Control Practices Advisory Committee and the HIPAC/SHEA/APIC/IDSA Hand Hygiene Task Force", Am.J.Infect.Control 30 (8), 2002, Seite 1 - 46) als log₁₀-Reduktionsfaktor angegeben.

Erfindungsgemäß wird unter dem Begriff "log₁₀-Reduktionsfaktor" die Differenz zwischen dem dekadischen Logarithmus der Anzahl der Mikroorganismen vor und dem dekadischen Logarithmus der Anzahl der Mikroorganismen nach einer Bestrahlung dieser Mikroorganismen mit elektromagnetischer Strahlung in Gegenwart wenigstens einer erfindungsgemäßen Verbindung mit der Formel (1) verstanden.

Geeignete Methoden zur Bestimmung des log₁₀-Reduktionsfaktors sind beispielsweise in der DIN EN 14885:2007-01 "Chemische Desinfektionsmittel und Antiseptika - Anwendung Europäischer Normen für chemische Desinfektionsmittel und Antiseptika" oder in Rabenau, H.F. und Schwebke, I. ("Leitlinie der Deutschen Vereinigung zur Bekämpfung der Viruskrankheiten (DVV) e.V. und des Robert Koch-Instituts (RKI) zur Prüfung von chemischen Desinfektionsmitteln auf Wirksamkeit gegen Viren in der Humanmedizin" Bundesgesundheitsblatt, Gesundheitsforschung, Gesundheitsschutz 51(8), (2008), Seiten 937 - 945) beschrieben.

Vorzugsweise beträgt der log₁₀-Reduktionsfaktor nach einer Bestrahlung von Mikroorganismen mit elektromagnetischer Strahlung in Gegenwart wenigstens einer erfindungsgemäßen Verbindung mit der Formel (1) mindestens 2 log₁₀, vorzugsweise mindestens 3 log₁₀, weiter bevorzugt mindestens 4 log₁₀, weiter bevorzugt mindestens 4,5 log₁₀, weiter bevorzugt mindestens 5 log₁₀, weiter bevorzugt mindestens 6 log₁₀, noch weiter bevorzugt mindestens 7 log₁₀, noch weiter bevorzugt mindestens 7,5 log₁₀.

Beispielsweise bedeutet eine Reduktion der Anzahl der Mikroorganismen nach einer Bestrahlung dieser Mikroorganismen mit elektromagnetischer Strahlung in Gegenwart wenigstens einer erfindungsgemäßen Verbindungen mit der Formel (1) um 2 Zehnerpotenzen, bezogen auf die Ausgangsmenge dieser Mikroorganismen, einen log₁₀-Reduktionsfaktor von 2 log₁₀.

Weiter bevorzugt wird die Anzahl der Mikroorganismen nach einer Bestrahlung dieser Mikroorganismen mit elektromagnetischer Strahlung in Gegenwart wenigstens einer erfindungsgemäßen Verbindung mit der Formel (1) um mindestens 1 Zehnerpotenz, weiter bevorzugt um mindestens 2 Zehnerpotenzen, vorzugsweise um mindestens 4 Zehnerpotenzen, weiter bevorzugt um mindestens 5 Zehnerpotenzen, weiter bevorzugt um mindestens 6 Zehnerpotenzen, noch weiter bevorzugt um mindestens 7 Zehnerpotenzen, jeweils bezogen auf die Ausgangsmenge dieser Mikroorganismen, verringert.

Unter dem Begriff "Mikroorganismen" werden im Sinne der Erfindung insbesondere Viren, Archäeen, prokaryote Mikroorganismen, wie Bakterien und Bakteriensporen, und eukaryote Mikroorganismen, wie Pilze, Protozoen, Pilzsporen, einzellige Algen, verstanden. Die Mikroorganismen können dabei einzellig oder mehrzellig, beispielsweise als Pilzmycel, auftreten.

Erfindungsgemäß weist das 1H-Phenalen-1-on-Derivat die Formel (1) auf.

Bei der erfindungsgemäßen Verbindung mit der Formel (1) gemäß Anspruch 1 sind R2 bis R8 Wasserstoff und R1 ist -(CH₂)ₖ-X, wobei k eine ganze Zahl von 1 bis 4, weiter bevorzugt 1, ist und wobei X ein organischer Rest, der a) wenigstens ein neutrales, protonierbares Stickstoffatom und/oder b) wenigstens ein positiv geladenes Stickstoffatom enthält, ist, ausgewählt wird.

Vorzugsweise wird der organische Rest X, der a) wenigstens ein neutrales, protonierbares Stickstoffatom und/oder b) wenigstens ein positiv geladenes Stickstoffatom enthält, ausgewählt aus der Gruppe, die aus gesättigten oder ungesättigten Alkylresten, gesättigten oder ungesättigten Heteroalkylresten, gesättigten oder ungesättigten Cycloalkylresten, gesättigten oder ungesättigten heterocyclischen Alkylresten, Arylresten und Heteroarylresten besteht. Vorzugsweise weisen die Arylreste jeweils höchstens 4, weiter bevorzugt höchstens 3, weiter bevorzugt höchstens 2, anellierte Ringe auf. Noch weiter bevorzugt weisen die Arylreste jeweils 1 Ring auf. Vorzugsweise weisen die Heteroarylreste jeweils höchstens 4, weiter bevorzugt höchstens 3, weiter bevorzugt höchstens 2, anellierte Ringe auf. Noch weiter bevorzugt weisen die Heteroarylreste jeweils 1 Ring auf.

Bei einer weiteren bevorzugten Ausführungsform weisen die Heteroarylreste jeweils 5 bis 20, vorzugsweise 5 bis 13, weiter bevorzugt 5 bis 7, Ringatome, die mindestens ein 1 Kohlenstoffatom und 1 bis 4 Stickstoffatome sowie optional 1 oder 2 Sauerstoff- oder Schwefelatom umfassen, auf. Bei einer weiteren bevorzugten Ausführungsform weisen die gesättigten oder ungesättigten heterocyclischen Alkylreste jeweils 5 bis 20, vorzugsweise 5 bis 13, weiter bevorzugt 5 bis 7, Ringatome, die mindestens ein 1 Kohlenstoffatom und 1 bis 4 Stickstoffatome sowie optional 1 oder 2 Sauerstoff- oder Schwefelatom umfassen, auf.

Bei einer weiteren bevorzugten Ausführungsform werden die gesättigten oder ungesättigten Alkylreste jeweils aus der Gruppe, die aus Methyl, Ethyl, n-Propyl, n-Butyl, n-Pentyl, n-Hexyl, n-Heptyl und n-Octyl besteht, ausgewählt.

Bei einer weiteren bevorzugten Ausführungsform weist das erfindungsgemäße 1H-Phenalen-1-on-Derivat ein Molekulargewicht von weniger als 1100 g/mol, vorzugsweise weniger als 990 g/mol, weiter bevorzugt weniger als 810 g/mol, weiter bevorzugt weniger als 690 g/mol, noch weiter bevorzugt weniger als 610 g/mol, noch weiter bevorzugt weniger als 600 g/mol, noch weiter bevorzugt weniger als 570 g/mol, auf.

Chiralitätszentren können, wenn nicht anders angegeben, in der R- oder in der S-Konfiguration vorliegen. Die Erfindung betrifft sowohl die optisch reinen Verbindungen als auch Stereoisomerengemische, wie Enantiomerengemische und Diasteromerengemische, in jedem Verhältnis.

Die Erfindung betrifft vorzugsweise auch Mesomere und/oder Tautomere der Verbindung mit der Formel (1), sowohl die reinen Verbindungen als auch Isomerengemische in jedem Verhältnis.

Bei einer bevorzugten Ausführungsform wird X durch die Formel (2) repräsentiert: 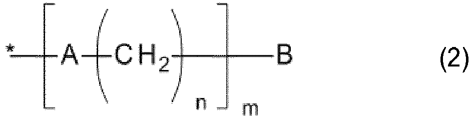 wobei A ein Sauerstoff- oder ein Schwefelatom oder ein Stickstoffatom, das neutral oder positiv geladen sein kann, ist und wobei n eine ganze Zahl von 1 bis 3, und m eine ganze Zahl von 0 bis 10, ist und wobei B aus der Gruppe, die aus den Resten der Formel (3), (4) und (5): 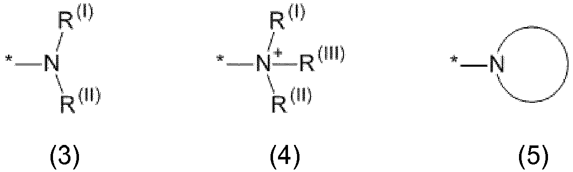 besteht, ausgewählt wird, und wobei jeder der Reste R^{(I)}, R^{(II)} und R^{(III)} unabhängig voneinander ausgewählt wird aus Wasserstoff, Amidino, Guanidino, Monosaccharid oder C1 bis C20 Alkyl, vorzugsweise C2 bis C18 Alkyl, das geradkettig oder verzweigt, vorzugsweise geradkettig, sein kann, wobei die vorgenannten Alkylreste unsubstituiert sind oder mit wenigstens einem Rest, der aus der Gruppe, die aus Amino, Methylamino, Dimethylamino, Trimethylammonio, Imino, Methylimino, Amidino, Hydroxy und Guanidino besteht, ausgewählt wird, substituiert sein können.

Vorzugsweise haben geeignete Monosaccharide 3 bis 7 Kohlenstoffatome, vorzugsweise 5 bis 6 Kohlenstoffatome, und weisen eine Carbonylgruppe, vorzugsweise Aldehydgruppe oder Ketogruppe, sowie mindestens eine Hydroxylgruppe auf und können offenkettig oder cyclisch, vorzugsweise als Furanose oder Pyranose, vorliegen.

Bevorzugt werden geeignete Monosaccharide aus der Gruppe, die aus D-Glycerinaldehyd, L-Glycerinaldehyd, D-Erythrose, L-Erythrose, D-Threose, L-Threose, D-Ribose, L-Ribose, D-Arabinose, L-Arabinose, D-Xylose, L-Xylose, D-Lyxose, L-Lyxose, D-Allose, L-Allose, D-Altrose, L-Altrose, D-Glucose, L-Glucose, D-Mannose, L-Mannose, D-Gulose, L-Gulose, D-Idose, L-Idose, D-Galactose, L-Galactose, D-Talose, L-Talose, Dihydroxyaceton, D-Erythrulose, L-Erythrulose, D-Ribulose, L-Ribulose, D-Xylulose, L-Xylulose, D-Psicose, L-Psicose, D-Fructose, L-Fructose, D-Sorbose, L-Sorbose, D-Tagatose und L-Tagatose besteht, ausgewählt. Weiter bevorzugt werden geeignete Monosaccharide aus der Gruppe, die aus D-Ribose, L-Ribose, D-Arabinose, L-Arabinose, D-Xylose, L-Xylose, D-Lyxose, L-Lyxose, D-Allose, L-Allose, D-Altrose, L-Altrose, D-Glucose, L-Glucose, D-Mannose, L-Mannose, D-Gulose, L-Gulose, D-Idose, L-Idose, D-Galactose, L-Galactose, D-Talose, L-Talose, D-Ribulose, L-Ribulose, D-Xylulose, L-Xylulose, D-Psicose, L-Psicose, D-Fructose, L-Fructose, D-Sorbose, L-Sorbose, D-Tagatose und L-Tagatose besteht, ausgewählt.

Bei einer weiteren bevorzugten Ausführungsform werden die Reste R^{(I)} R^{(II)} und R^{(III)} unabhängig voneinander aus der Gruppe, die aus Wasserstoff oder Alkylgruppen der allgemeinen Formel -(CH₂)ₙ-CH₃, wobei n eine ganze Zahl von 0 bis 19, vorzugsweise von 1 bis 17, ist, besteht, ausgewählt.

Bei einer weiteren bevorzugten Ausführungsform werden die Reste R^{(I)} R^{(II)} und R^{(III)} unabhängig voneinander ausgewählt aus der Gruppe, die aus Wasserstoff, Methyl, Ethyl, Prop-1-yl, Prop-2-yl, But-1-yl, But-2-yl, 2-Methylprop-1-yl, 2-Methyl-prop-2-yl, Pent-1-yl, Pent-2-yl, Pent-3-yl, 2-Methylbut-1-yl, 2-Methylbut-2-yl, 2-Methylbut-3-yl, 2-Methylbut-4-yl, 2,2-Dimethylprop-1-yl, Hex-1-yl, Hex-2-yl, Hex-3-yl, Hept-1-yl, Oct-1-yl, 2-Methylpent-1-yl, 2-Methylpent-2-yl, 2-Methylpent-3-yl, 2-Methylpent-4-yl, 2-Methylpent-5-yl, 3-Methylpent-1-yl, 3-Methylpent-2-yl, 3-Methylpent-3-yl, 2,2-Dimethylbut-1-yl, 2,2-dimethylbut-3-yl, 2,2-Dimethylbut-4-yl, 2,3-Dimethylbut-1-yl und 2,3-Dimethylbut-2-yl besteht, ausgewählt. Bei einer besonders bevorzugten Ausführungsform werden die Reste R^{(I)} R^{(II)} und R^{(III)} unabhängig voneinander aus der Gruppe, die aus Methyl, Ethyl, Prop-1-yl, But-1-yl, Pent-1-yl, Hex-1-yl, Hept-1- und Oct-1-yl besteht, ausgewählt.

Bei einer weiteren bevorzugten Ausführungsform werden die Reste R^{(I)} R^{(II)} und R^{(III)} unabhängig voneinander aus der Gruppe, die aus Wasserstoff oder den Resten der Formel (6) bis (9): 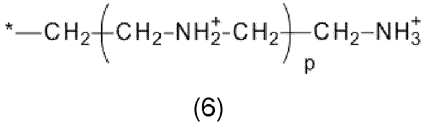 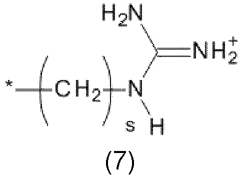 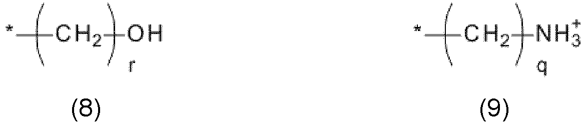 besteht, wobei p eine ganze Zahl von 1 bis 10, vorzugsweise von 1 bis 7, weiter bevorzugt von 1 bis 3, bedeutet und wobei s, r, q jeweils unabhängig voneinander eine ganze Zahl von 1 bis 20, vorzugsweise von 1 bis 8, weiter bevorzugt von 1 bis 4, bedeutet, ausgewählt.

Bei einer weiteren bevorzugten Ausführungsform bedeutet der Rest mit der Formel (5): 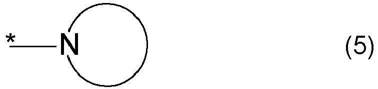 einen substituierten oder unsubstituierten heterocyclischen Rest mit 5 bis 7 Ringatomen, die mindestens ein 1 Kohlenstoffatom und 1 bis 4 Stickstoffatome sowie optional 1 oder 2 Sauerstoff- oder Schwefelatom umfassen, wobei der heterocyclische Rest gesättigt oder ungesättigt ist.

Unter dem Begriff "heterocyclisch" werden erfindungsgemäß cyclische Verbindungen mit ringbildenden Atomen aus mindestens zwei verschiedenen chemischen Elementen, vorzugsweise Kohlenstoff, Stickstoff, Sauerstoff oder Schwefel, verstanden.

Bei einer weiteren bevorzugten Ausführungsform wird der heterocyclische Rest mit 5 bis 7 Ringatomen ausgewählt aus der Gruppe, die aus Azolylen, Azolinylen, Azolidinylen, Diazolylen, Diazolinylen, Diazolidinylen, Triazolylen, Triazolinylen, Triazolidinylen, Tetrazolylen, Thiazolylen, Thiadiazolylen, Oxazolylen, Oxazolinylen, Oxazolidinylen, Oxadiazolylen, Azinylen, Dihydroazinylen, Tetrahydroazinylen, Diazinylen, Dihydrodiazinylen, Tetrahydrodiazinylen, Tetrahydrodiazinylen, Triazinylen, Tetrazinylen, Oxazinylen, Dihydrooxazinylen, Tetrahydrooxazinylen, Thiazinylen, Azepanylen, Azepinylen, Diazepinylen oder Thiaazepinylen besteht, ausgewählt, wobei die vorgenannten heterocyclischen Reste unsubstituiert sind oder mit wenigstens einem Rest, der aus der Gruppe, die aus Phenyl, Benzyl, geradkettigen oder verzweigten Alkyl mit 1 bis 20 C-Atomen, Amino, Methylamino, und Dimethylamino besteht, ausgewählt wird, substituiert sein können.

Bei einer weiter bevorzugten Ausführungsform wird der heterocyclische Rest aus Verbindungen mit 5 bis 7 Ringatomen gebildet, wobei diese Verbindungen aus der Gruppe, die aus Pyrrolidin, Pyrrol, Imidazol, Imidazolin, Imidazolidin, Pyrazol, Pyrazolin, Pyrazolidin, Triazol, Tetrazol, Oxazol, Oxazolin, Oxazolidin, Isoxazol, Isoxazolin, Isoxazolidin, Thiazol, Thiazolin, Thiazolidin, Isothiazol, Isothiazolin, Isothiazolidin, Oxadiazolen, Thiadiazolen, Piperidin, Pyridin, Piperazin, Pyrazine, Pyrimidin, Pyridazin, Oxazinen, Dihydrooxazinen, Tetrahydrooxazinen, vorzugsweise Morpholin, Thiazin, Triazinen, Azepinen, Azepan, Diazepinen und Thiazepinen besteht, ausgewählt wird, wobei die vorgenannten Verbindungen unsubstituiert sind oder mit wenigstens einem Rest, der aus der Gruppe, die aus Phenyl, Benzyl, geradkettigen oder verzweigten Alkyl mit 1 bis 20 C-Atomen, Amino, Methylamino, und Dimethylamino besteht, ausgewählt wird, substituiert sein können.

Bei einer besonders bevorzugten Ausführungsform wird der heterocyclische Rest mit 5 bis 7 Ringatomen ausgewählt aus der Gruppe, die aus Piperazinium-1-yl, 4-Methylpiperazinium-1-yl, 4-Morpholinium, Pyrrolidinium-1-yl, Pyridinium-1-yl und 4-Aminopyridinium-1-yl besteht, ausgewählt.

Bei einer weiteren bevorzugten Ausführungsform bedeutet der Rest mit der Formel (5): 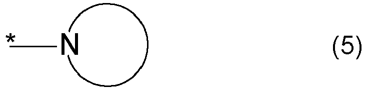 einen substituierten oder unsubstituierten Heteroarylrest mit 5 bis 7 Ringatomen, die mindestens ein 1 Kohlenstoffatom und 1 bis 4 Stickstoffatome sowie optional 1 oder 2 Sauerstoff- oder Schwefelatom umfassen, oder einen substituierten oder einen unsubstituierten cyclischen Heteroalkylrest mit 5 bis 7 Ringatomen, die mindestens ein 1 Kohlenstoffatom und 1 bis 4 Stickstoffatome sowie optional 1 oder 2 Sauerstoff- oder Schwefelatom umfassen, oder einen substituierten oder unsubstituierten cyclischen Heteroalkenylrest mit 5 bis 7 Ringatomen, die mindestens ein 1 Kohlenstoffatom und 1 bis 4 Stickstoffatome sowie optional 1 oder 2 Sauerstoff- oder Schwefelatom umfassen, darstellt.

Bei einer weiteren bevorzugten Ausführungsform bedeutet der Rest mit der Formel (5): 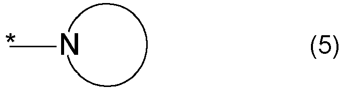 einen substituierten oder unsubstituierten heterocyclischen Rest mit 5 bis 7 Ringatomen, der aus der Gruppe, die aus den Resten der Formel (60) bis (84c) besteht, ausgewählt wird: 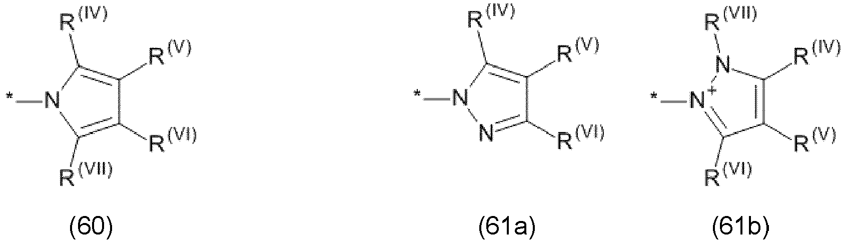 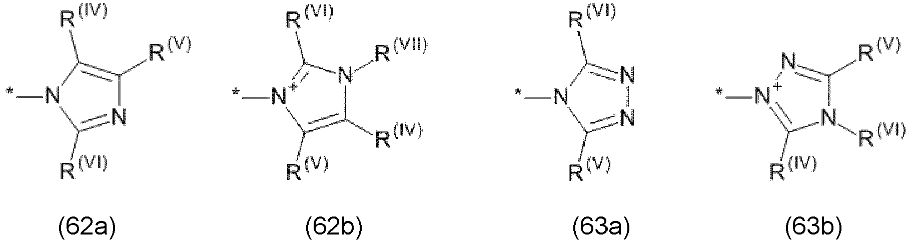 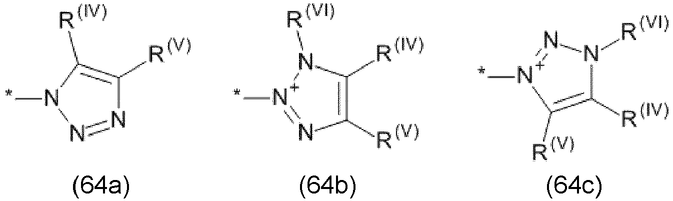 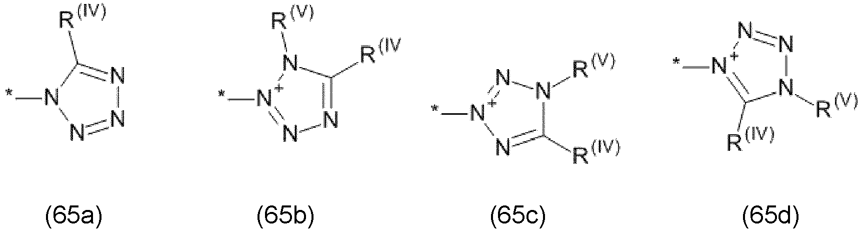 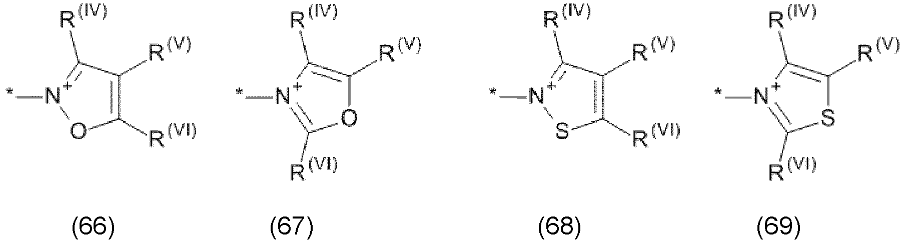 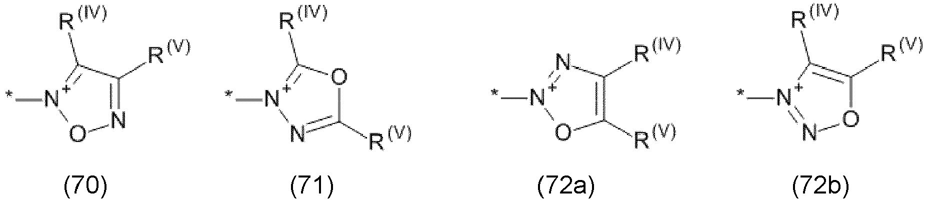 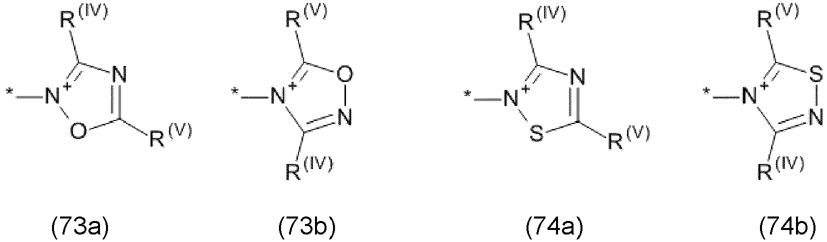 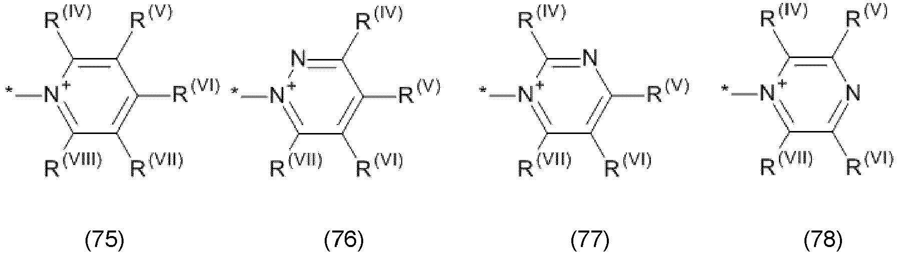 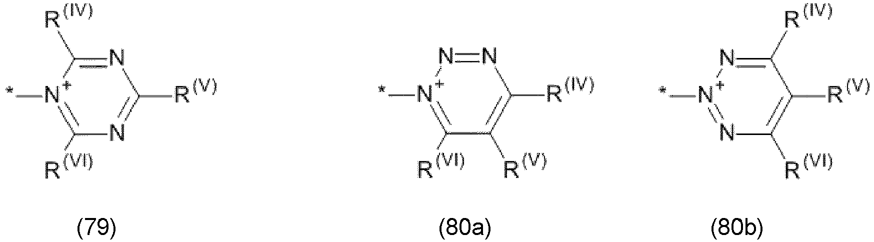 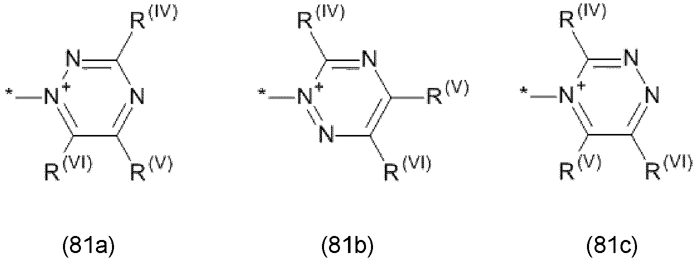 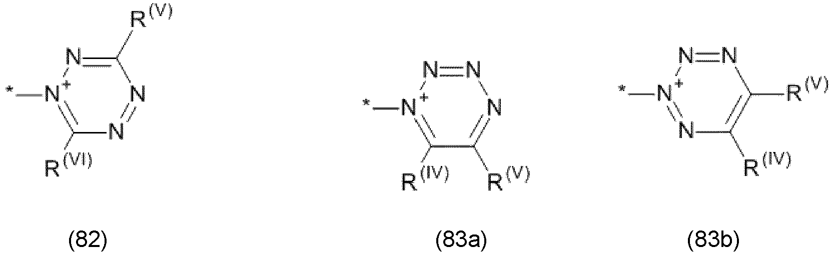 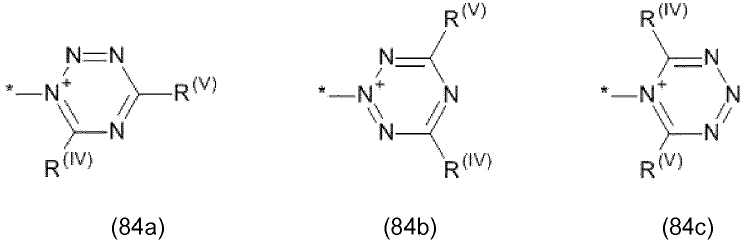 und wobei jeder der Reste R^{(IV)}, R^{(V)}, R^{(VI)}, R^{(VII)} und R^{(VIII)} unabhängig voneinander jeweils aus der Gruppe ausgewählt wird, die aus Wasserstoff, Phenyl, Benzyl, C1 bis C20 Alkyl, das geradkettig oder verzweigt sein kann, Amino, Methylamino, und Dimethylamino besteht.

Bei einer weiteren bevorzugten Ausführungsform der Verbindung mit der Formel (1) sind R2 bis R8 Wasserstoff und R1 ist -(CH₂)ₖ-X , wobei k eine ganze Zahl von 1 bis 4, weiter bevorzugt 1, ist und wobei X ein Rest mit der Formel (5): 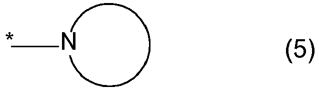 ist, der einen substituierten oder unsubstituierten heterocyclischen Rest mit 5 bis 7 Ringatomen bedeutet, der aus der Gruppe, die aus den Resten der Formel (60) bis (84c) besteht, ausgewählt wird.

Bei einer weiteren bevorzugten Ausführungsform des 1H-Phenalen-1-on-Derivats die Formel (1) wird X aus der Gruppe, die aus den Resten der Formeln (10) bis (33): 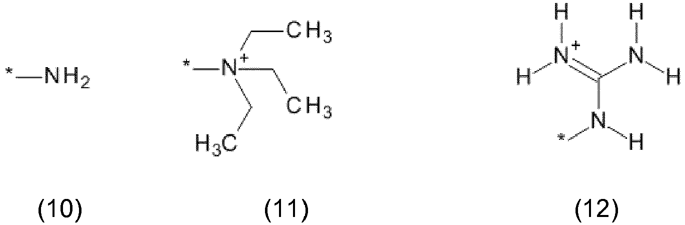 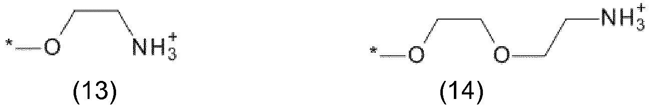 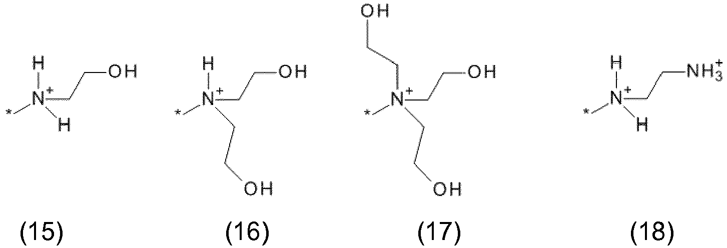 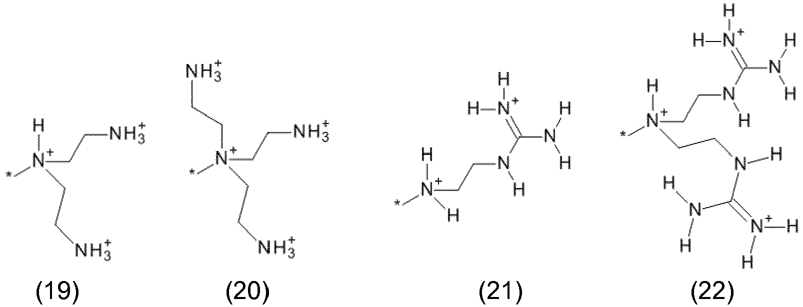 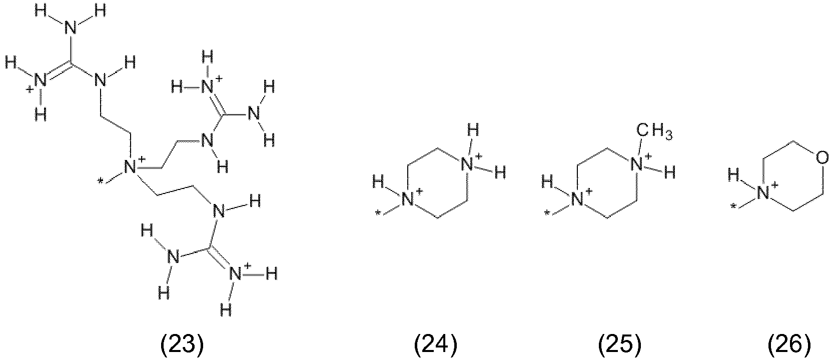 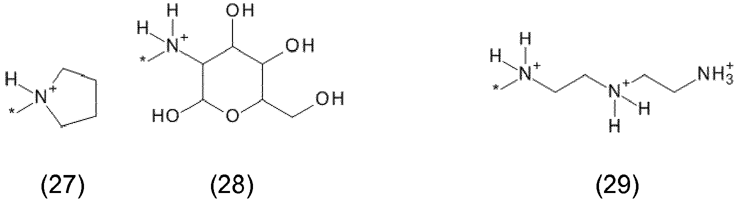 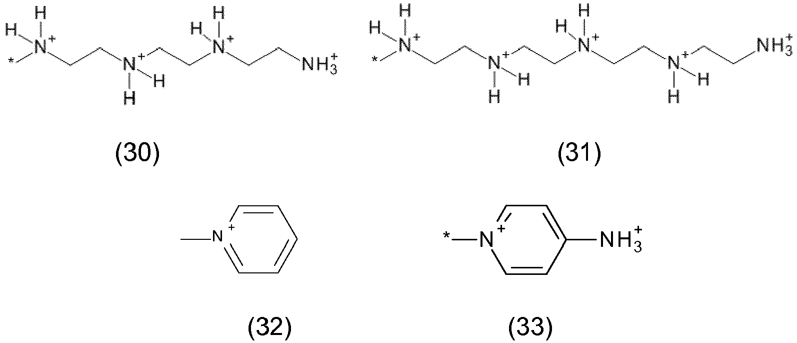 besteht, ausgewählt.

Bei einer weiteren bevorzugten Ausführungsform sind R2 bis R8 Wasserstoff und R1 ist -(CH₂)ₖ-X, wobei X aus der Gruppe, die aus den Resten der Formel (9) bis (33) besteht, ausgewählt wird. Bei dieser bevorzugten Ausführungsform wird das erfindungsgemäße 1H-Phenalen-1-on-Derivat mithin aus der Gruppe, die aus den Verbindung mit den Formeln (34) bis (57) besteht, ausgewählt:

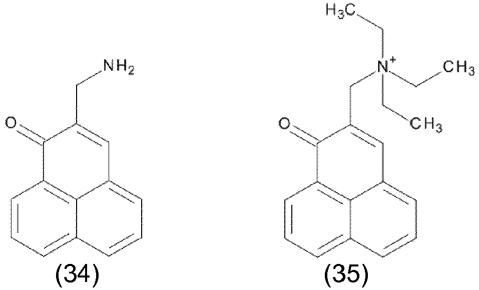

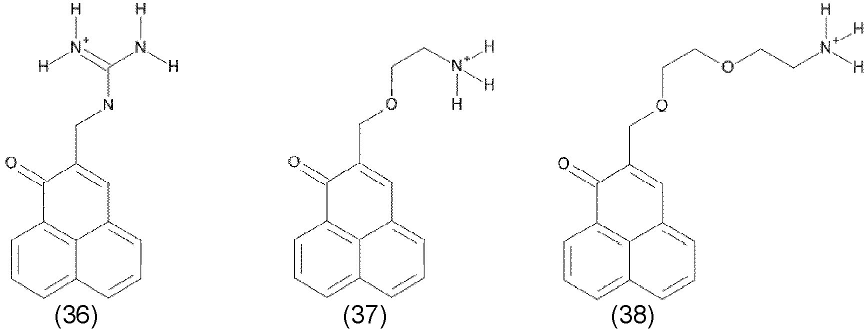

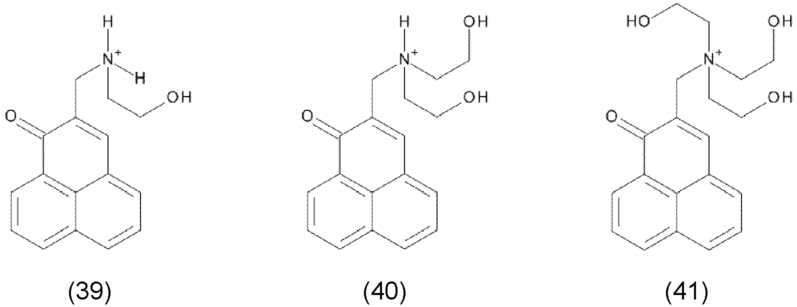

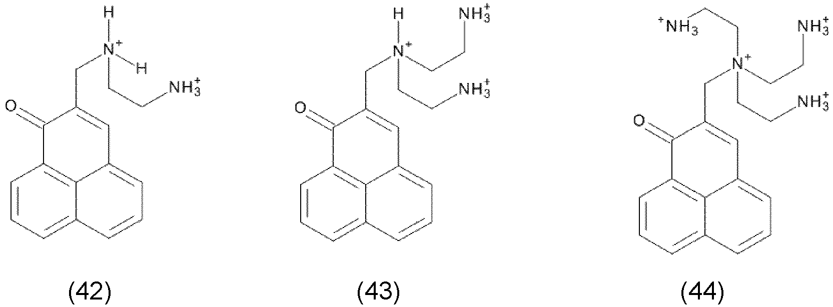

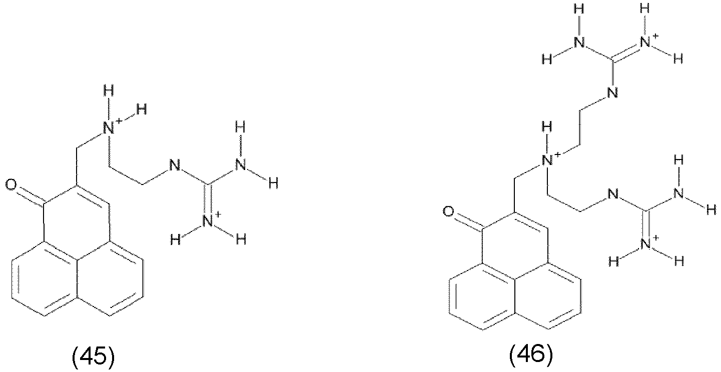

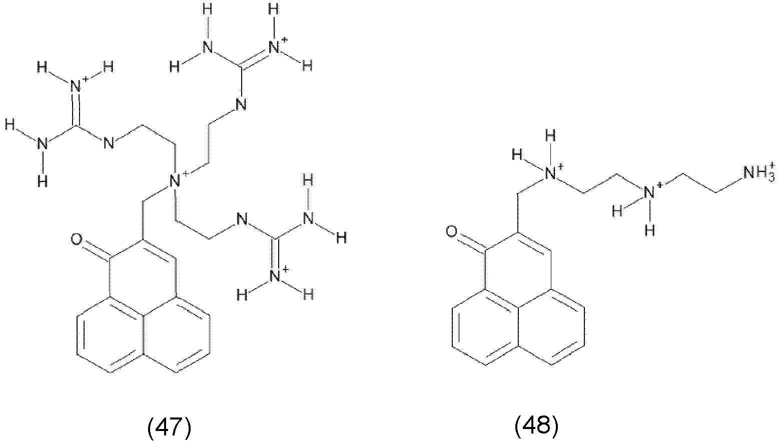

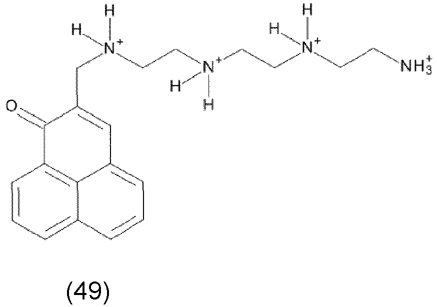

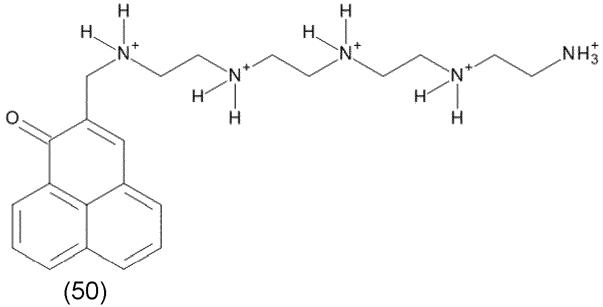

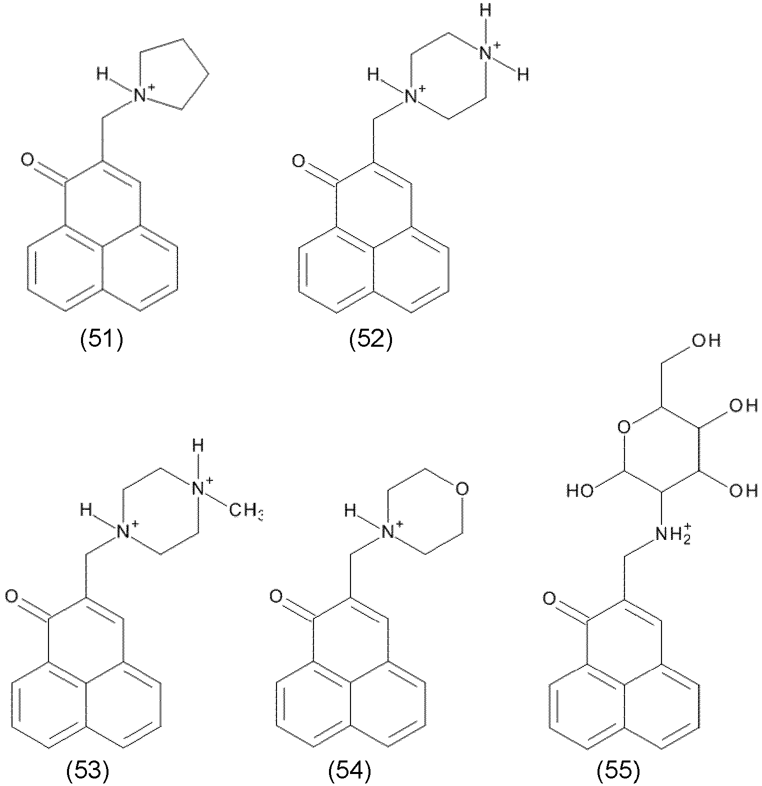

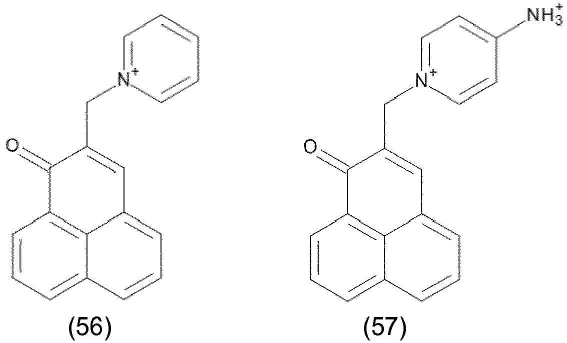

Bei einer weiteren bevorzugten Ausführungsform weist das 1H-Phenalen-1-on-Derivat die Formel (1) auf, wobei das Molekulargewicht des 1H-Phenalen-1-on-Derivats der Formel (1) weniger als 1000 g/mol, vorzugsweise weniger als 890 g/mol, weiter bevorzugt weniger als 750 g/mol, weiter bevorzugt weniger als 660 g/mol, noch weiter bevorzugt weniger als 600 g/mol, noch weiter bevorzugt weniger als 570 g/mol, beträgt.

Das erfindungsgemäße Verfahren zur Herstellung eines 1H-Phenalen-1-on-Derivats der Formel (1), umfasst folgende Schritte:
(A1) Umsetzen von 1H-Phenalen-1-on mit wenigstens einem Alkylierungsagens der Formel Y-(CH₂)ₕ-Z, wobei Y aus der Gruppe, die aus H, Cl, Br, I, p-Toluolsulfonyl (OTs), welches auch als Tosyl bezeichnet wird, Methansulfonyl (OMs), welches auch als Mesyl bezeichnet wird, OH und Alkyl₂S⁺, vorzugsweise H, Cl, Br und I, besteht, wobei Alkyl gleich oder unabhängig voneinander verschieden sein kann und vorzugsweise Methyl, Ethyl, Propyl oder Butyl bedeutet, ausgewählt wird, optional in Gegenwart eines Katalysators, vorzugsweise Lewis-Säure oder Broenstedt-Säure, unter Erhalt einer Verbindung mit der Formel (58): 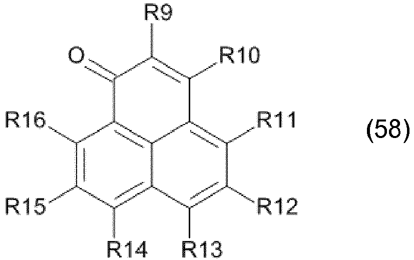 wobei R10 bis R16 jeweils Wasserstoff bedeuten und wobei R9 den Rest -(CH₂)ₕ-Z bedeutet, wobei h eine ganze Zahl von 1 bis 4, weiter bevorzugt 1, bedeutet und wobei Z aus der Gruppe, die aus Cl, Br, I, OTs, OMs, OH und Alkyl₂S⁺ besteht, wobei Alkyl gleich oder unabhängig voneinander verschieden sein kann und vorzugsweise Methyl, Ethyl, Propyl oder Butyl bedeutet, ausgewählt wird, oder
(A2) Umsetzen von 1H-Phenalen-1-on mit Formaldehyd und wenigstens einer Halogenwasserstoffsäure, optional in Gegenwart eines Katalysators, unter Erhalt einer Verbindung mit der Formel (58): 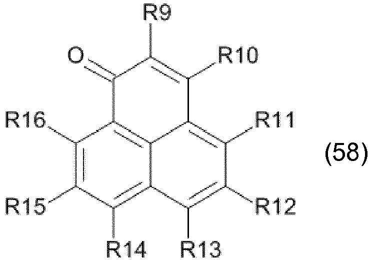 wobei R10 bis R16 jeweils Wasserstoff bedeuten und wobei R1 den Rest -CH₂-W bedeutet und wobei W aus der Gruppe, die aus Cl, Br und I besteht, ausgewählt wird,
(B) Umsetzen der in Schritt (A1) oder (A2) erhaltenen Verbindung mit der Formel (58) mit einer organischen Verbindung, die a) wenigstens ein neutrales, protonierbares Stickstoffatom und/oder b) wenigstens ein positiv geladenes Stickstoffatom enthält, optional in Gegenwart einer Base, und optional
(C) Entfernen vorhandener Aminoschutzgruppen unter Erhalt des erfindungsgemäßen 1H-Phenalen-1-on-Derivats der Formel (1).

Bei der in Schritt (A2) verwendeten Halogenwasserstoffsäure handelt es sich vorzugsweise um HCl, HBr, HI oder Mischungen davon. Als sehr geeignete Halogenwasserstoffsäure hat sich HCl erwiesen. Vorzugsweise handelt es sich bei Schritt (A2) bei dem Rest -CH₂-W um -CH₂-Cl.

Bei einer weiteren bevorzugten Ausführungsform des erfindungsgemäßen Verfahrens wird in Schritt (A1) eine Verbindung der Formel (58) erhalten: 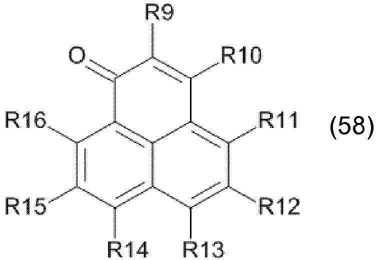 wobei R9 den Rest -(CH₂)ₕ-Z bedeutet, wobei h eine ganze Zahl von 1 bis 4, vorzugsweise 1, bedeutet und wobei Z aus der Gruppe, die aus Cl, Br, I, OTs, OMs, OH und Alkyl₂S⁺ besteht, wobei Alkyl gleich oder unabhängig voneinander verschieden sein kann und vorzugsweise Methyl, Ethyl, Propyl oder Butyl bedeutet, ausgewählt wird, und R10 bis R16 Wasserstoff bedeuten.

Bei einer weiteren bevorzugten Ausführungsform der Erfindung wird in Schritt (A2) eine Verwendung der Formel (58) erhalten, wobei R9 den Rest -CH₂-W bedeutet, wobei W aus der Gruppe, die aus Cl, Br und I besteht, ausgewählt wird. Vorzugsweise bedeutet R9 den Rest -CH₂-Cl.

Die Alkylierung von 1H-Phenalen-1-on kann beispielsweise mittels Friedel-Crafts-Alkylierung erfolgen, die die Alkylierung von Aromaten ermöglicht.

Bei einer bevorzugten Ausführungsform umfasst das Verfahren folgende Schritte:
(A1) Umsetzen von 1H-Phenalen-1-on mit wenigstens einem Haloalkylierungsagens, optional in Gegenwart eines Katalysators, vorzugsweise Lewis-Säure oder Broenstedt-Säure, unter Erhalt einer Verbindung der Formel (58): 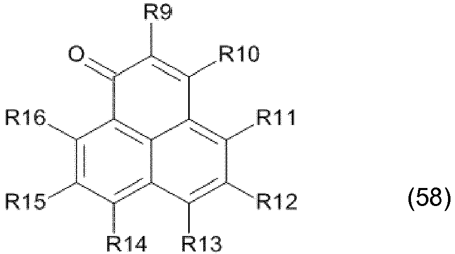 wobei R10 bis R16 Wasserstoff bedeuten und wobei R9 den Rest -(CH₂)ᵢ-Hal bedeutet, wobei i eine ganze Zahl von 1 bis 4, bedeutet und wobei Hal ein Halogenatom, das aus der Gruppe ausgewählt wird, die aus Cl, Br und I besteht, bedeutet, oder
(A2) Umsetzen von 1H-Phenalen-1-on mit Formaldehyd und wenigstens einer Halogenwasserstoffsäure, optional in Gegenwart eines Katalysators, unter Erhalt einer Verbindung mit der Formel (58): 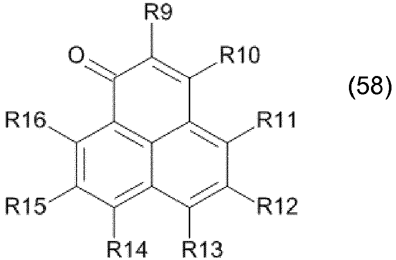 wobei R10 bis R16 Wasserstoff bedeutet und wobei R9 den Rest -CH₂-W bedeutet und wobei W aus der Gruppe, die aus Cl, Br und I besteht, ausgewählt wird,.
(B) Umsetzen der in Schritt (A1) oder (A2) erhaltenen Verbindung mit der Formel (58) mit einer organischen Verbindung, die a) wenigstens ein neutrales, protonierbares Stickstoffatom und/oder b) wenigstens ein positiv geladenes Stickstoffatom enthält, optional in Gegenwart einer Base, und optional
(C) Entfernen vorhandener Aminoschutzgruppen unter Erhalt des erfindungsgemäßen 1H-Phenalen-1-on-Derivats der Formel (1).

Bei der in Schritt (A2) verwendeten Halogenwasserstoffsäure handelt es sich vorzugsweise um HCl, HBr, HI oder Mischungen davon. Als sehr geeignete Halogenwasserstoffsäure hat sich HCl erwiesen. Vorzugsweise handelt es sich bei Schritt (A2) bei dem Rest -CH₂-W um -CH₂-Cl.

Ein geeignetes Alkylierungsagens ist aus dem Stand der Technik bekannt und überträgt den Rest -(CH₂)ₕ-Z, wobei h eine ganze Zahl von 1 bis 4 vorzugsweise 1, bedeutet und wobei Z aus der Gruppe, die aus Cl, Br, I, OTs, OMs, OH und Alkyl₂S⁺ besteht, wobei Alkyl vorzugsweise gleich oder unabhängig voneinander Methyl, Ethyl, Propyl oder Butyl bedeutet, ausgewählt wird.

Ein geeignetes Haloalkylierungsagens ist aus dem Stand der Technik bekannt und wird aus der Gruppe, die aus Aldehyden und Halogenwasserstoff, Haloalkylethern, Haloalkylsulfiden, Acetalen und Halogenwasserstoff, Di- und Polyhaloalkanen, Haloalkenen, Haloalkoholen, Haloalkylsulfaten, Haloalkyl-p-tosylaten, Haloalkylmesylaten und Haloalkylestern anorganischer Säuren besteht, ausgewählt, wobei ein geeignetes Haloalkylierungsagens den Rest -(CH₂)ᵢ-Hal, wobei i eine ganze Zahl von 1 bis 4, vorzugsweise 1, bedeutet und wobei Hal ein Halogenatom, vorzugsweise Cl, Br oder I, bedeutet, überträgt.

Vorzugsweise wird das Haloalkylierungsagens aus der Gruppe ausgewählt, die aus Aldehyden der allgemeinen Formel H-(CH₂)₍ᵢ₋₁₎-CHO oder Acetalen davon und Halogenwasserstoff, Haloalkylethern der allgemeinen Formel Hal-(CH₂)ᵢ-O-R¹⁷, Haloalkane der allgemeinen Formel Hal-(CH₂)ᵢ-F, Haloalkohole der allgemeinen Formel Hal-(CH₂)ᵢ-OH, Haloalkylsulfate der allgemeinen Formel Hal-(CH₂)ᵢ-OSO₃H, Haloalkyl-mesylate der allgemeinen Formel Hal-(CH₂)ᵢ-OMs, und Haloalkyl-p-tosylaten der allgemeinen Formel Hal-(CH₂)ᵢ-OTs besteht, ausgewählt, wobei i eine ganze Zahl von 1 bis 4, vorzugsweise 1, bedeutet und wobei Hal ein Halogenatom, das aus der Gruppe, die aus Cl, Br und I besteht, ausgewählt wird, bedeutet, und wobei R¹⁷ ein Alkylrest mit 1 bis 20 C-Atomen bedeutet.

Bei einer bevorzugten Ausführungsform findet die Haloalkylierung von 1H-Phenalen-1-on in Schritt (B) des erfindungsgemäßen Verfahrens in Gegenwart wenigstens eines Katalysators, vorzugsweise Lewis-Säure und/oder Broenstedt-Säure, statt.

Geeignete Katalysatoren sind aus dem Stand der Technik bekannt. Als Katalysator geeignete Lewis-Säuren sind beispielsweise AlCl₃, FeCl₃, FeBr₃, ZnCl₂, SnCl₄, BF₃ oder TiCl₄. Als Katalysator geeignete Broenstedt-Säuren sind beispielsweise H₂SO₄, H₃PO₄, H₂SnCl₆×6H₂O, HCOOH oder CH₃COOH.

Die in Schritt (B) erhaltene Verbindung mit der Formel (58) kann anschließend in Schritt (C) mit einer organischen Verbindung, die a) wenigstens ein neutrales, protonierbares Stickstoffatom und/oder b) wenigstens ein positiv geladenes Stickstoffatom enthält, unter Erhalt des erfindungsgemäßen 1H-Phenalen-1-on-Derivats der Formel (1) umgesetzt werden, optional in Gegenwart einer Base, beispielsweise NaOH, LiOH, KOH, Na₂CO₃, K₂CO₃ oder Cs₂CO₃.

Bei einer bevorzugten Ausführungsform wird die organischen Verbindung, die a) wenigstens ein neutrales, protonierbares Stickstoffatom und/oder b) wenigstens ein positiv geladenes Stickstoffatom enthält, aus der Gruppe, die aus den Verbindungen der Formel (2a), (2b) und (2c) besteht: 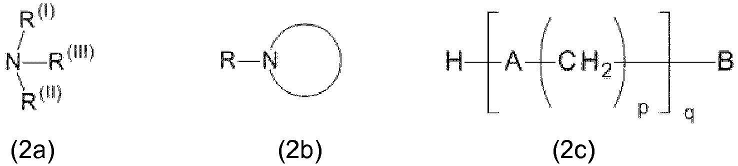 ausgewählt,
wobei der Rest R ausgewählt wird aus Wasserstoff, einer Schutzgruppe PG oder einem freien Elektronenpaar und wobei A ein Sauerstoff- oder ein Schwefelatom oder ein Stickstoffatom, das neutral oder positiv geladen sein kann, ist und wobei p eine ganze Zahl von 1 bis 3 und q eine ganze Zahl von 1 bis 10 ist und wobei B aus der Gruppe, die aus den Resten der Formel (3), (4) und (5): 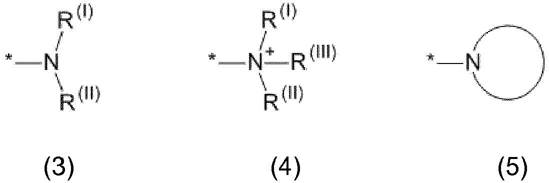 besteht, ausgewählt wird, und wobei jeder der Reste R^{(I)}, R^{(II)} und R^{(III)} unabhängig voneinander ausgewählt wird aus PG, Monosaccharid, vorzugsweise Pentose oder Hexose, oder C1 bis C20 Alkyl, vorzugsweise C2 bis C18 Alkyl, das geradkettig oder verzweigt, vorzugsweise geradkettig, sein kann, wobei die vorgenannten Alkylreste unsubstituiert sind oder mit wenigstens einem Rest, der aus der Gruppe, die aus -NH(PG), -N(PG)₂, -N(PG)CH₃, Dimethylamino, Trimethylammonio, =N(PG), Methylimino, geschütztes Amidino, Hydroxy und geschütztes Guanidino besteht, ausgewählt wird, substituiert sein können, wobei der Rest PG eine Amino-Schutzgruppe bedeutet.

Geeignete Amino-Schutzgruppen PG sind aus dem Stand der Technik bekannt und umfassen beispielsweise die Reste 1-(1-Adamantyl)-1-methylethoxycarbonyl (Adpoc), Allyloxycarbonyl (Alloc), Benzyloxycarbonyl (Cbz), Di-tert-butyloxycarbonyl (Boc), 9-Fluorenylmethoxycarbonyl (Fmoc), Trifluoracetyl (Tfa) oder Triphenylmethyl (Trt).

Bei einer weiteren bevorzugten Ausführungsform bedeutet der Rest mit der Formel (2a): 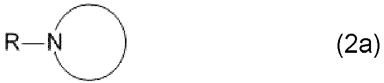 einen substituierten oder unsubstituierten heterocyclischen Rest mit 5 bis 7 Ringatomen, die mindestens ein 1 Kohlenstoffatom und 1 bis 4 Stickstoffatome sowie optional 1 oder 2 Sauerstoff- oder Schwefelatom umfassen, wobei der heterocyclische Rest gesättigt oder ungesättigt ist und wobei der Rest R ausgewählt wird aus Wasserstoff, PG oder einem freien Elektronenpaar.

Bei einer weiteren bevorzugten Ausführungsform wird der substituierte oder unsubstituierte heterocyclische Rest mit 5 bis 7 Ringatomen aus den zuvor aufgeführten heterocyclischen Resten ausgewählt.

Bei einer weiteren bevorzugten Ausführungsform werden die Reste R^{(I)} R^{(II)} und R^{(III)} unabhängig voneinander aus der Gruppe, die aus PG und den Resten der Formel (6a), (7a), (8), (9a): 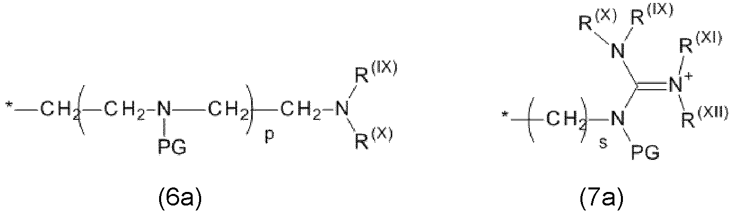 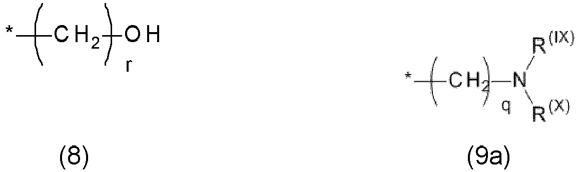 besteht, wobei p eine ganze Zahl von 1 bis 10, vorzugsweise von 1 bis 7, weiter bevorzugt von 1 bis 3, bedeutet und wobei s, r, q jeweils unabhängig voneinander eine ganze Zahl von 1 bis 20, vorzugsweise von 1 bis 8, weiter bevorzugt von 1 bis 4, vorzugsweise 1, bedeutet und wobei die Reste R^{(IX)}, R^{(X)}, R^{(XI)} und R^{(XII)} unabhängig voneinander PG oder Methyl oder Wasserstoffatom bedeuten und wobei PG eine Amino-Schutzgruppe bedeutet, ausgewählt.

Bei einer weiteren bevorzugten Ausführungsform wird die organische Verbindung, die a) wenigstens ein neutrales, protonierbares Stickstoffatom und/oder b) wenigstens ein positiv geladenes Stickstoffatom enthält, aus der Gruppe, die aus Verbindungen der Formeln (10a) bis (33a) besteht: 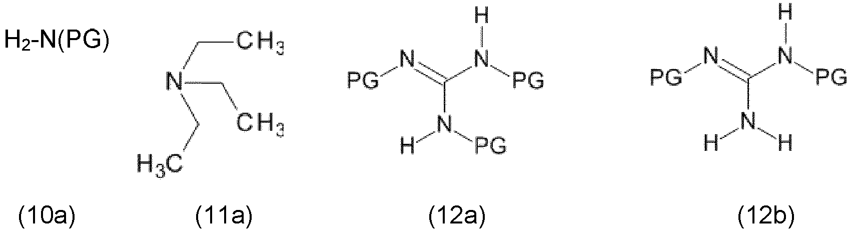 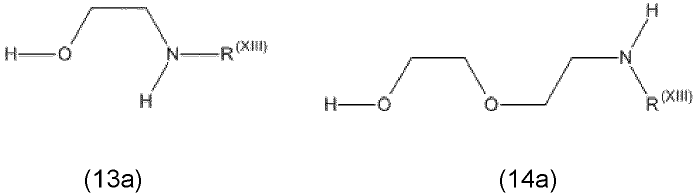 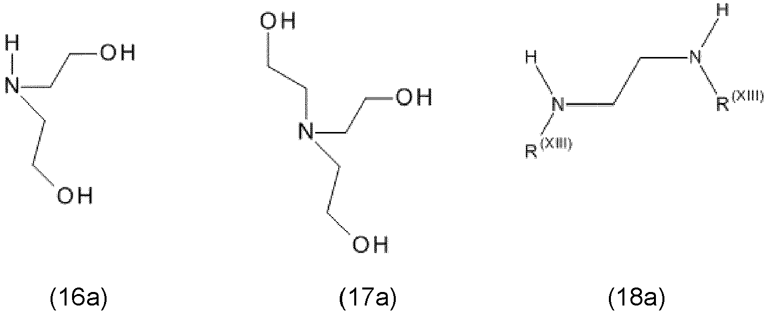 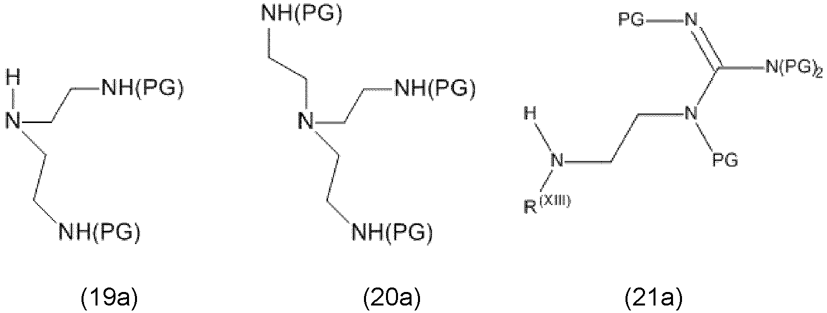 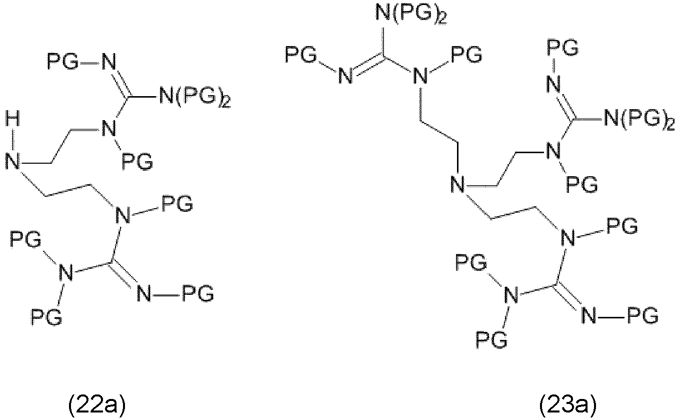 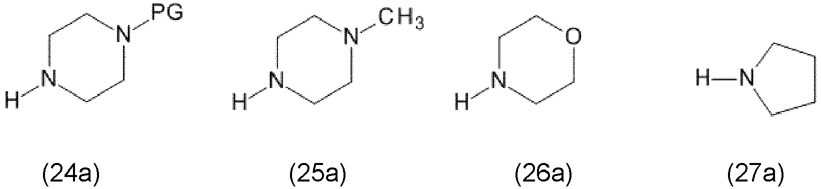 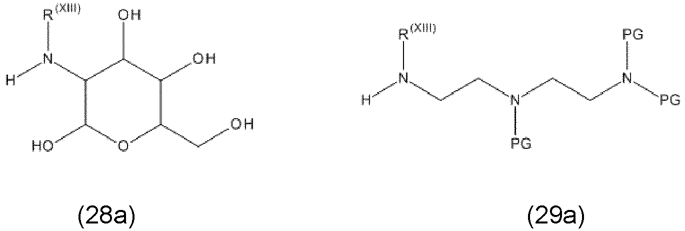 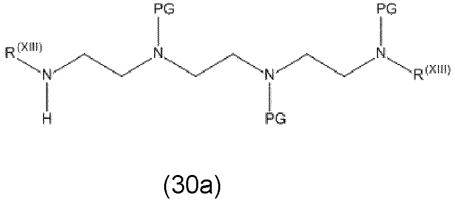 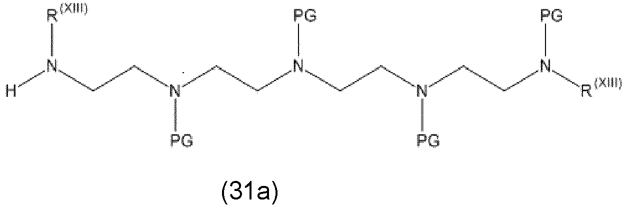 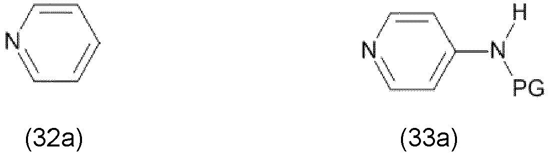 ausgewählt, wobei der Rest R^{(XIII)} PG oder Wasserstoff ist und der Rest PG eine Amino-Schutzgruppe, vorzugsweise Benzyloxycarbonyl (Cbz) oder Di-tert-butyloxycarbonyl (Boc), bedeutet.

Geeignete Methoden zur Entfernung der Amino-Schutzgruppe PG sind aus dem Stand der Technik bekannt. Beispielsweise kann Benzyloxycarbonyl (Cbz) durch katalytische Hydrierung unter hydrogenolytischer Spaltung der Benzyl-Heteroatom-Bindung mit anschließender Decarboxylierung der so entstehenden instabilen Carbaminsäure oder Behandlung mit Säuren wieder entfernt werden. Di-tert-butyloxycarbonyl (Boc) kann beispielsweise durch saure Hydrolyse entfernt werden. Allyloxycarbonyl (Alloc) kann beispielsweise durch Einwirkung von Tetrakis(triphenylphosphan)palladium(0) und einem Nukleophil abgespalten werden.

Bei der Verwendung unterschiedlicher Amino-Schutzgruppe PG in einer Synthese, ergibt sich die Möglichkeit der orthogonalen Schutzgruppenstrategie, wobei unterschiedliche Amino-Funktionen eines Moleküls gezielt nacheinander freigesetzt können und zur Reaktion gebracht werden.

Bei einer weiteren bevorzugten Ausführungsform finden die Schritte (B) und/oder (C) in Gegenwart eines oder mehrerer Lösungsmittel statt. Der Schritt (B) kann beispielsweise in Gegenwart von Dichlormethan (DCM), Dimethylformamid (DMF) oder Acetonitril (MeCN) durchgeführt werden. Der Schritt (C) kann beispielsweise in Gegenwart von Wasser/Dichlormethan oder Toluol/Tetrabutylammoniumiodid (TBAI) durchgeführt werden.

Einzellige oder mehrzellige Mikroorganismen können Auslöser von infektiösen Erkrankungen sein. Durch Applikation wenigstens eines Erreger-spezifischen Gegenmittels, beispielsweise Antibiotikum, Antimikotikum oder Virustatikum, kann die Anzahl der Erreger reduziert und/oder der Erreger inaktiviert werden. Die Applikation eines Erreger-spezifischen Gegenmittels kann systemisch und/oder topisch erfolgen.

Bei der systemischen Applikation wird das Erreger-spezifische Gegenmittel in das Blut- und/oder Lymphsystem des zu behandelnden Körpers übertragen und hierüber im gesamten Körper verteilt. Bei einer systemischen Aufnahme des Erreger-spezifischen Gegenmittels kann es zu einem Abbau des Gegenmittels und/oder zu Nebenwirkungen, beispielsweise durch eine biochemische Umwandlung (Metabolisierung) des Gegenmittels, kommen.

Bei der topischen Applikation des Erreger-spezifischen Gegenmittels erfolgt die Anwendung des Gegenmittels dort, wo es therapeutisch wirken soll, beispielsweise auf einer infizierten Hautpartie, während die gesunde Haut nicht belastet wird. Somit können systemische Nebenwirkungen weitgehend vermieden werden.

Oberflächliche Haut- oder Weichteilinfektionen müssen nicht notwendigerweise mit, einer systemischen Applikation eines Erreger-spezifischen Gegenmittels behandelt werden, da das Gegenmittel direkt auf die infizierte Hautpartien aufgetragen werden kann.

Die bisher bekannten Erreger-spezifischen Gegenmittel weisen sowohl bei systemischer als auch topischer Applikation teilweise starke Neben- und Wechselwirkungen auf. Darüber hinaus kann es auch bei topischer Applikation durch eine unzuverlässige Medikamenteneinnahme (Compliance) des Patienten, insbesondere bei Verwendung von Antibiotika, zur Resistenzbildung kommen.

Eine Alternative stellt hier die photodynamische Inaktivierung von Mikroorganismen dar, bei der Resistenzen gegenüber der photodynamischen Inaktivierung unbekannt sind. Unabhängig von der Art der zu bekämpfenden Mikroorganismen und der damit verbundenen infektiösen Erkrankungen wird die Anzahl der Erreger reduziert und/oder die Erreger werden abtötet. Beispielsweise können Mischungen aus verschieden Mikroorganismen, beispielsweise Pilze und Bakterien oder unterschiedliche Bakterienstämme, bekämpft werden.

Bei einer bevorzugten Ausführungsform wird wenigstens ein erfindungsgemäßes 1H-Phenalen-1-on-Derivat oder ein pharmakologisch verträgliches Salz und/oder Ester und/oder Komplex davon als Photosensibilisator bei der photodynamischen Inaktivierung von Mikroorganismen, vorzugsweise bei der photodynamischen Therapie, verwendet.

Das erfindungsgemäße 1H-Phenalen-1-on-Derivat weist nach Bestrahlung mit elektromagnetischer Strahlung geeigneter Wellenlänge und Energiedichte eine hohe Ausbeute an Singulett-Sauerstoff auf.

Vorzugsweise liegt die elektromagnetische Strahlung im sichtbaren Spektralbereich, ultravioletten und/oder infraroten Bereich. Weiter bevorzugt weist die elektromagnetische Strahlung eine Wellenlänge aus einem Bereich von 280 bis 1000 nm, weiter bevorzugt von 380 bis 1000 nm, auf.

Weiter bevorzugt weist die elektromagnetische Strahlung eine Energiedichte aus einem Bereich von 1 µW/cm² bis 1 MW/cm², weiter bevorzugt von 1 mW/cm² bis 1 kW/cm², auf.

Die Bestrahlungszeit kann in Abhängigkeit von der Art der Mikroorganismen und/oder der Schwere der Infektion variiert werden. Vorzugsweise liegt die Bestrahlungszeit in einem Bereich von 1 µs bis 1 h, weiter bevorzugt von 1 ms bis 1000 s.

Vorzugsweise wird die elektromagnetische Strahlung durch eine Strahlungsquelle erzeugt, die aus der Gruppe, die aus Sonne und künstlichen Strahlungsquellen, beispielsweise UV-Lampe, IR-Lampe, Leuchtstofflampen, Leuchtdioden, Laser oder chemisches Licht, besteht, ausgewählt wird.

Darüber hinaus haben die Erfinder überraschenderweise festgestellt, dass das erfindungsgemäße 1H-Phenalen-1-on-Derivat oder pharmakologisch verträgliche Salze und/oder Ester davon vorzugsweise eine hohe Affinität zu Mikroorganismen aufweist.

Auf Grund der Affinität kann das erfindungsgemäße 1H-Phenalen-1-on-Derivat an Mikroorganismen effektiv binden und lokal ausreichend Singulett-Sauerstoff erzeugen, um die Mikroorganismen zu inaktivieren, vorzugsweise abzutöten.

Bei dieser bevorzugten Verwendung als Photosensibilisator wird wenigstens ein erfindungsgemäßes 1H-Phenalen-1-on-Derivat von Mikroorganismen gebunden. Nach Bestrahlung mit elektromagnetischer Strahlung geeigneter Wellenlänge und Energiedichte werden die Mikroorganismen durch die entstandenen reaktiven Sauerstoffspezies (ROS), vorzugsweise Sauerstoffradikale und/oder Singulett-Sauerstoff, inaktiviert, vorzugsweise abgetötet.

Vorzugsweise erlaubt die Bindung wenigstens eines erfindungsgemäßen 1H-Phenalen-1-on-Derivates an Mikroorganismen ebenfalls eine Anfärbung oder Lokalisierung von Mikroorganismen. Dadurch kann vorzugsweise auch der Verlauf der Inaktivierung von Mikroorganismen oder der Dekolonisation verfolgt werden.

Erfindungsgemäß wird unter dem Begriff "Dekolonisation" das Entfernen, vorzugsweise vollständige Entfernen, von Mikroorganismen verstanden.

Bei einer weiteren bevorzugten Ausführungsform wird das erfindungsgemäße 1H-Phenalen-1-on-Derivat oder ein pharmakologisch verträgliches Salz und/oder Ester und/oder Komplex davon bei der Inaktivierung von einzelligen oder mehrzelligen Mikroorganismen, die vorzugsweise aus der Gruppe, die aus Viren, Archäeen, Bakterien, Bakteriensporen, Pilzen, beispielsweise Myzelpilze und Hefen, Pilzsporen, Protozoen, Algen und blutübertragbaren Parasiten besteht, ausgewählt werden, verwendet.

Vorzugsweise können Körperoberflächen, beispielsweise Haut oder Schleimhaut, von Menschen und Tieren, vorzugsweise Säugetieren, behandelt werden. Bei dieser bevorzugten Ausführungsform wird wenigstens ein erfindungsgemäßes 1H-Phenalen-1-on-Derivat oder ein pharmakologisch verträgliches Salz und/oder Ester und/oder Komplex davon, vorzugsweise in einer pharmazeutischen Zubereitung, bei der Desinfektion und/oder Dekolonisierung von Haut- oder Weichteiloberflächen verwendet, wobei vorzugsweise die Hautintegrität erhalten bleibt.

Bei einer weiteren bevorzugten Ausführungsform ist wenigstens ein erfindungsgemäßes 1H-Phenalen-1-on-Derivat oder ein pharmakologisch verträgliches Salz und/oder Ester und/oder Komplex davon in einer pharmazeutischen Zubereitung zur topischen, vorzugsweise nasalen, oralen, analen, vaginalen oder dermalen, Applikation vorhanden.

Unter topischer Applikation wird auch die Anwendung am oder im Ohr, vorzugsweise Außenohr, verstanden. Das Außenohr umfasst den Ohrknorpel, die Ohrmuschel, das Ohrläppchen und den äußeren Gehörgang oder auch Ohrkanal und die Außenseite des Trommelfells.

Unter topischer Applikation wird ebenfalls die oberflächliche Anwendung im Nasenraum, auf Schleimhäuten, auf der Haut oder jeder Körperhöhle verstanden.

Bei einer weiteren bevorzugten Ausführungsform wird wenigstens ein erfindungsgemäßes 1H-Phenalen-1-on-Derivat oder ein pharmakologisch verträgliches Salz und/oder Ester und/oder Komplex davon zur Herstellung einer pharmazeutischen Zubereitung bei der Prophylaxe und/oder Behandlung einer infektiösen, vorzugsweise viralen, bakteriellen und/oder mykotischen, Hautkrankheit, die vorzugsweise ausgewählt wird aus der Gruppe, die aus Staphylococcal scalded skin syndrome, Impetigo, Hautabszess, Furunkel, Karbunkel, Phlegmone, Cellulitis, Akute Lymphadenitis, Pilonidalzyste, Pyodermie, Dermatitis purulenta, Dermatitis septica, Dermatitis suppurativa, Erythrasma, Erysipelas, Akne vulgaris oder Pilzinfektion besteht, verwendet.

Bei einer weiteren bevorzugten Ausführungsform wird wenigstens ein erfindungsgemäßes 1H-Phenalen-1-on-Derivat oder ein pharmakologisch verträgliches Salz und/oder Ester und/oder Komplex davon zur Herstellung einer pharmazeutischen Zubereitung bei der Wundheilung, beispielsweise bei Heilungsstörungen nach chirurgischen Interventionen, verwendet.

Vorzugsweise wird wenigstens ein erfindungsgemäßes 1H-Phenalen-1-on-Derivat oder ein pharmakologisch verträgliches Salz und/oder Ester und/oder Komplex davon bzw. eine pharmazeutische Zubereitung enthaltend wenigstens ein erfindungsgemäßes 1H-Phenalen-1-on-Derivat oder ein pharmakologisch verträgliches Salz und/oder Ester und/oder Komplex davon bei der Desinfektion und/oder Reduktion der Keimzahl in infizierten Wunden verwendet.

Bei einer weiteren bevorzugten Ausführungsform wird wenigstens ein erfindungsgemäßes 1H-Phenalen-1-on-Derivat oder ein pharmakologisch verträgliches Salz und/oder Ester und/oder Komplex davon zur Herstellung einer pharmazeutischen Zubereitung bei der Prophylaxe und/oder Behandlung von infektiösen, vorzugsweise viralen, bakteriellen und/oder mykotischen, Erkrankungen des Ohrs, der oberen Luftwege, der Mundhöhle, des Rachens, des Kehlkopfes, der unteren Luftwege und/oder der Speiseröhre verwendet.

Das Vorherrschen pathogener Mikroorganismen ist beispielsweise die Hauptursache für Infektionen in der Mundhöhle. Dabei tritt das Problem auf, dass die Mikroorganismen in äußerst komplex aufgebauten Biofilmen synergetisch organisiert sind. Diese Biofilme, beispielsweise Plaque oder Zahnbelag, bestehen aus mehreren, komplex aufgebauten Schichten und enthalten Eiweiße, Kohlenhydrate, Phosphate und Mikroorganismen. Zahnbelag entsteht besonders dort, wo Zahnflächen nicht durch natürliche oder künstliche Reinigung belagfrei gehalten werden können. Dieser Umstand macht es schwierig, einen Zugang zu den im Biofilm eingebundenen Mikroorganismen zu finden.

Konventionelle Therapien, wie beispielsweise Antibiotika und Spüllösungen oder mechanische Zahnreinigung, können nur begrenzt eingesetzt werden, da sie entweder die Bakterien nicht direkt beeinflussen, beispielsweise bei der Zahnreinigung, nur schwer dosiert und appliziert werden können, beispielsweise bei Antibiotika und Spüllösungen, oder eine generelle Anwendung aufgrund von negativen Begleiterscheinungen nicht zu rechtfertigen ist.

Bei einer bevorzugten Ausführungsform wird wenigstens ein erfindungsgemäßes 1H-Phenalen-1-on-Derivat oder ein pharmakologisch verträgliches Salz und/oder Ester und/oder Komplex davon als Photosensibilisator bei der photodynamischen Inaktivierung von Mikroorganismen in der Mundhöhle verwendet.

Bei einer weiteren bevorzugten Ausführungsform wird wenigstens ein erfindungsgemäßes 1H-Phenalen-1-on-Derivat oder ein pharmakologisch verträgliches Salz und/oder Ester und/oder Komplex davon zur Herstellung einer pharmazeutischen Zubereitung bei der Behandlung und/oder Prophylaxe einer infektiösen, vorzugsweise viralen, bakteriellen und/oder mykotischen, Erkrankung des Zahngewebes, vorzugsweise Plaque, Karies oder Pulpitis, und/oder infektiösen, vorzugsweise viralen, bakteriellen und/oder mykotischen, Erkrankung des Zahnhalteapparates, vorzugsweise Gingivitis, Paradontitis, Endodontitis oder Periimplantitis, verwendet.

Bei einer weiteren bevorzugten Ausführungsform wird wenigstens ein erfindungsgemäßes 1H-Phenalen-1-on-Derivat oder ein pharmakologisch verträgliches Salz und/oder Ester und/oder Komplex davon bzw. eine pharmazeutische Zubereitung enthaltend wenigstens ein erfindungsgemäßes 1H-Phenalen-1-on-Derivat oder ein pharmakologisch verträgliches Salz und/oder Ester und/oder Komplex davon bei der Reinigung von Zähnen, Zahnersatz und/oder Zahnspangen verwendet.

Bei einer weiteren bevorzugten Ausführungsform wird das erfindungsgemäße 1H-Phenalen-1-on-Derivat oder ein pharmakologisch verträgliches Salz und/oder Ester und/oder Komplex davon bzw. eine pharmazeutische Zubereitung enthaltend wenigstens ein erfindungsgemäßes 1H-Phenalen-1-on-Derivat oder ein pharmakologisch verträgliches Salz und/oder Ester und/oder Komplex davon bei der nasalen Dekolonisierung von Mikroorganismen verwendet.

Beispielsweise besitzen Methicillin-resistente Staphylococcus aureus (MRSA)-Stämme eine monatelange Persistenz bei nasaler Kolonisierung sowie eine hohe Umweltresistenz. Daher reduziert eine nasale Dekolonisierung, d.h. Entfernung der Mikroorganismen, in der Regel auch die Kolonisation an anderen Körperstellen.

Des Weiteren betrifft die vorliegende Erfindung eine pharmazeutische Zusammensetzung enthaltend wenigstens ein erfindungsgemäßes 1H-Phenalen-1-on-Derivat oder ein pharmakologisch verträgliches Salz und/oder Ester und/oder Komplex davon.

Vorzugsweise wird die pharmazeutische Zusammensetzung durch Mischen von mindestens einer Verbindung der Formel (1) oder ein pharmakologisch verträgliches Salz und/oder Ester und/oder Komplex davon mit einem oder mehreren physiologisch annehmbaren Hilfsstoff(en) und/oder Trägerstoff(en) hergestellt und in eine geeignete Darreichungsform gebracht.

Eine geeignete Darreichungsformen der erfindungsgemäßen pharmazeutischen Zusammensetzung wird vorzugsweise aus der Gruppe, die aus Salbe, Creme, Gel, Lotion, Schüttelmixtur, Lösung, beispielsweise in Tropfen- oder Sprayform, Puder, Mikrokapsel und Paste besteht, ausgewählt.

Die erfindungsgemäße pharmazeutische Zusammensetzung kann topisch, vorzugsweise nasal, oral, anal, vaginal oder dermal, angewendet werden.

Als physiologisch annehmbare Hilfsstoffe kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage.

Bei einer weiteren bevorzugten Ausführungsform enthält die pharmazeutische Zusammensetzung eine effektive Menge wenigstens eines erfindungsgemäßen 1H-Phenalen-1-on-Derivates oder eines pharmakologisch verträglichen Salzes und/oder Esters und/oder Komplexes davon, wobei die effektive Menge von 0,01 µg bis 1000 µg pro Gramm der Zusammensetzung, vorzugsweise von 0,1 µg bis 500 µg pro Gramm der Zusammensetzung, umfasst.

Bei einer bevorzugten Ausführungsform der Erfindung umfasst die pharmazeutische Zusammensetzung wenigstens ein erfindungsgemäßes 1H-Phenalen-1-on-Derivat oder ein pharmakologisch verträgliches Salz und/oder Ester und/oder Komplex davon und wenigstens einen weiteren pharmazeutisch aktiven Bestandteil.

Vorzugsweise wird der wenigstens eine weitere pharmazeutisch aktive Bestandteil aus der Gruppe, die aus Antibiotika, Antimikotika, Virustatika, Antihistaminika, Sympathomimetika, Antihämorrhagika, Emmolientia und Hautschutzmittel, Analgetika, Desinfektionsmittel, Immunsera und Immunglobuline, Antiparasitäre Substanzen, Insektizide, Repellenzien und Corticosteroide besteht, ausgewählt.

Bei einer weiteren bevorzugten Ausführungsform wird wenigstens ein erfindungsgemäßes 1H-Phenalen-1-on-Derivat oder ein pharmakologisch verträgliches Salz und/oder Ester und/oder Komplex davon bzw. eine pharmazeutische Zubereitung enthaltend wenigstens ein erfindungsgemäßes 1H-Phenalen-1-on-Derivat oder ein pharmakologisch verträgliches Salz und/oder Ester und/oder Komplex davon durch den Anwender selbst aufgebracht und, optional, nachfolgend mit einer geeigneten Strahlenquelle, die elektromagnetische Strahlung geeigneter Wellenlänge und Energiedichte erzeugt, bestrahlt.

Bei einer weiteren bevorzugten Ausführungsform wird wenigstens ein erfindungsgemäßes 1H-Phenalen-1-on-Derivat oder ein pharmakologisch verträgliches Salz und/oder Ester und/oder Komplex davon bzw. eine Zubereitung enthaltend wenigstens ein erfindungsgemäßes 1H-Phenalen-1-on-Derivat oder ein pharmakologisch verträgliches Salz und/oder Ester und/oder Komplex davon bei der Inaktivierung von Mikroorganismen in medizinischen Blutprodukten verwendet.

Bei einer bevorzugten Ausführungsform wird wenigstens ein erfindungsgemäßes 1H-Phenalen-1-on-Derivat zur Inaktivierung von Mikroorganismen auf Oberflächen aller Art verwendet. Weiter bevorzugt wird das erfindungsgemäße 1H-Phenalen-1-on-Derivat bei der Oberflächenreinigung und/oder -beschichtung, vorzugsweise von Medizinprodukten, Lebensmittelverpackungen oder Hygieneartikeln, verwendet.

Weiter bevorzugt wird wenigstens ein erfindungsgemäßes 1H-Phenalen-1-on-Derivat auf Oberflächen aufgebracht und/oder eingebracht und, optional, nachfolgend mit einer geeigneten Strahlenquelle, die elektromagnetische Strahlung geeigneter Wellenlänge und Energiedichte erzeugt, bestrahlt. Vorzugsweise bewirkt das wenigstens eine erfindungsgemäße 1H-Phenalen-1-on-Derivat während der Bestrahlung eine "Selbstdesinfektion" der Oberfläche.

Die Bestrahlung kann dabei direkt nach der Behandlung der Oberfläche mit wenigstens einem erfindungsgemäßen 1H-Phenalen-1-on-Derivat, vorzugsweise nach dem Aufbringen des wenigstens einen erfindungsgemäßen 1H-Phenalen-1-on-Derivates auf die Oberfläche und/oder Einbringen des wenigstens einen erfindungsgemäßen 1H-Phenalen-1-on-Derivates in die Oberfläche, erfolgen und/oder zu einem späteren Zeitpunkt.

Weiter bevorzugt werden Gegenstände behandelt, die eine thermisch begrenzte Haltbarkeit aufweisen, beispielsweise Gegenstände aus thermoplastische Kunststoffen, oder von Desinfektionsmitteln angegriffen werden.

Gegenstände, die eine thermisch begrenzte Haltbarkeit aufweisen, können beispielsweise nur unzureichend sterilisiert werden, da sie bei höheren Temperaturen ihre Form verlieren oder spröde werden.

Darüber hinaus kann es bei einer unsachgemäßen und/oder übermäßigen Anwendung von Desinfektionsmitteln zu Resistenzbildung durch Selektion robuster Mikroorganismen kommen, wenn beispielsweise die Wirkstoffkonzentration und Einwirkzeit und damit die keimreduzierende Wirkung zu gering ist.

Bei einer weiter bevorzugten Ausführungsform wird wenigstens ein erfindungsgemäßes 1H-Phenalen-1-on-Derivat zur Inaktivierung von Mikroorganismen auf Oberflächen von Medizinprodukten, vorzugsweise invasiven medizinischen Hilfsmitteln wie etwa Kathetern, Hohlsonden, Schläuchen oder Nadeln, verwendet.

Vorzugsweise werden die Medizinprodukten aus Wundauflagen, Verbände, Kathetern, Hohlsonden, Schläuchen oder Nadeln ausgewählt.

Weiter bevorzugt werden unter Medizinprodukten auch zahnärztliche Abdrücke oder Zahnersatz, beispielsweise Prothesen, Kronen oder Implantate, verstanden.

Vorzugsweise wird durch eine Behandlung der Oberfläche von Medizinprodukten mit wenigstens einem 1H-Phenalen-1-on-Derivat gemäß der Erfindung und/oder Beschichtung und/oder Immobilisierung wenigstens eines erfindungsgemäßen 1H-Phenalen-1-on-Derivates auf der Oberfläche von Medizinprodukten und nachfolgender Bestrahlung mit elektromagnetische Strahlung geeigneter Wellenlänge und Energiedichte die Besiedelung von Mikroorganismen auf der behandelten Oberflächen reduziert, vorzugsweise verhindert.

Die Bestrahlung kann dabei direkt nach der Behandlung der Oberfläche mit wenigstens einem erfindungsgemäßen 1H-Phenalen-1-on-Derivat, vorzugsweise nach dem Aufbringen des wenigstens einen erfindungsgemäßen 1H-Phenalen-1-on-Derivates auf die Oberfläche und/oder Einbringen des wenigstens einen erfindungsgemäßen 1H-Phenalen-1-on-Derivates in die Oberfläche, erfolgen und/oder zu einem späteren Zeitpunkt, vor oder während der Verwendung des behandelten Medizinproduktes.

Bei einer weiter bevorzugten Verwendung des wenigstens einen erfindungsgemäßen 1H-Phenalen-1-on-Derivates oder eines pharmakologisch verträgliches Salzes und/oder Esters und/oder Komplexes davon in Wundauflagen und/oder Verbänden, beispielsweise Baumwollgaze, kann während oder nach dem Aufbringen einer Wundauflage und/oder Verbandes, die bzw. der wenigstens ein erfindungsgemäßes 1H-Phenalen-1-on-Derivat oder ein pharmakologisch verträgliches Salz und/oder Ester und/oder Komplex davon enthält, eine Bestrahlung mit elektromagnetische Strahlung geeigneter Wellenlänge und Energiedichte erfolgen wodurch es nachfolgend zu einer Reduzierung, vorzugsweise Inaktivierung, von Mikroorganismen im Wundbereich oder behandelten Hautpartien kommen.

Bei einer weiteren bevorzugten Ausführungsform umfasst die Wundauflage und/oder Verband neben wenigstens einem erfindungsgemäßen 1H-Phenalen-1-on-Derivat oder eines pharmakologisch verträgliches Salzes und/oder Esters und/oder Komplexes davon weitere Bestandteile, vorzugsweise Absorptionsmittel, beispielsweise Calciumalginat oder Polyurethanschaum, oder weitere pharmazeutisch wirksame Substanzen.

Bei einer weiteren bevorzugten Ausführungsform wird wenigstens ein erfindungsgemäßes 1H-Phenalen-1-on-Derivat zur Inaktivierung von Mikroorganismen auf Oberflächen von Lebensmittelverpackungen verwendet.

Bei einer weiteren bevorzugten Ausführungsform wird wenigstens ein erfindungsgemäßes 1H-Phenalen-1-on-Derivat zur Inaktivierung von Mikroorganismen in einer Flüssigkeit oder flüssigen, vorzugsweise wässrigen, Zubereitung, beispielsweise Dispersionsfarbe, verwendet.

Vorzugsweise handelt es sich bei der Flüssigkeit um Wasser.

Dabei kann wenigstens ein erfindungsgemäßes 1H-Phenalen-1-on-Derivat zur Aufbereitung von Wasser für die Getränke- und Lebensmittelindustrie, die Pharma-, Chemie- und Kosmetikindustrie, die Elektroindustrie verwendet werden. Ferner kann wenigstens ein erfindungsgemäßes 1H-Phenalen-1-on-Derivat bei der Trinkwasser- und Regenwasseraufbereitung, der Behandlung von Abwasser oder bei der Aufbereitung von Wasser für den Einsatz in der Klimatechnik eingesetzt werden.

Bei dieser bevorzugten Verwendung wenigstens eines erfindungsgemäßen 1H-Phenalen-1-on-Derivates kann die Flüssigkeit oder flüssige Zubereitung nachfolgend mit einer geeigneten Strahlenquelle, die elektromagnetische Strahlung geeigneter Wellenlänge und Energiedichte erzeugt, bestrahlt werden. Vorzugsweise bewirkt das erfindungsgemäße 1H-Phenalen-1-on-Derivat während der Bestrahlung eine "Selbstdesinfektion" der Flüssigkeit oder der flüssigen Zubereitung.

Bei einer weiteren bevorzugten Verwendung des wenigstens einen erfindungsgemäßen 1H-Phenalen-1-on-Derivates kann das 1H-Phenalen-1-on-Derivat an einen festen Träger gebunden vorliegen und so als Teil einer festen Matrix verwendet werden. Besonders bevorzugt wird wenigstens ein an einen festen Träger gebundenes erfindungsgemäßes 1H-Phenalen-1-on-Derivat in die zu behandelnde Flüssigkeit, vorzugsweise Wasser oder Blut, eingebracht.

Besonders bevorzugt ist als Träger ein Polymer, welches wenigstens ein erfindungsgemäßes 1H-Phenalen-1-on-Derivat daran in kovalent gebundener Weise trägt. Diese Zusammensetzung, umfassend den Träger und wenigstens ein erfindungsgemäßes 1H-Phenalen-1-on-Derivat, entwickelt eine antimikrobielle Aktivität sobald sie elektromagnetischer Strahlung geeigneter Wellenlänge und Energiedichte ausgesetzt wird.

Des Weiteren betrifft die vorliegende Erfindung einen beschichteten Gegenstand, der wenigstens ein erfindungsgemäßes 1H-Phenalen-1-on-Derivat enthält und/oder damit beschichtet ist.

Vorzugsweise weist die Oberfläche des beschichteten Gegenstand wenigstens ein erfindungsgemäßes 1H-Phenalen-1-on-Derivat auf.

Der beschichtete Gegenstand kann nachfolgend mit einer geeigneten Strahlenquelle, die elektromagnetische Strahlung geeigneter Wellenlänge und Energiedichte erzeugt, bestrahlt werden. Vorzugsweise bewirkt das erfindungsgemäße 1H-Phenalen-1-on-Derivat während der Bestrahlung eine "Selbst-desinfektion" der Oberfläche des beschichteten Gegenstandes.

Die Bestrahlung kann dabei direkt nach der Behandlung des beschichteten Gegenstandes mit wenigstens einem erfindungsgemäßen 1H-Phenalen-1-on-Derivat, vorzugsweise nach dem Aufbringen des wenigstens einen erfindungsgemäßen 1H-Phenalen-1-on-Derivates auf die Oberfläche des beschichteten Gegenstandes und/oder Einbringen des wenigstens einen erfindungsgemäßen 1H-Phenalen-1-on-Derivates in die Oberfläche des beschichteten Gegenstandes, erfolgen und/oder zu einem späteren Zeitpunkt, vorzugsweise vor oder während der Verwendung des beschichteten Gegenstandes.

Geeignete Gegenstände werden vorzugsweise aus der Gruppe, die aus Medizinprodukten, Lebensmittelverpackungen oder Hygieneartikeln, besteht, ausgewählt.

Bei einer weiteren bevorzugten Ausführungsform des beschichteten Gegenstandes handelt es sich um mit wenigstens einem erfindungsgemäßen 1-H-Phenalen-on-Derivat beschichtete Partikel, beispielsweise anorganische oder organische Partikel.

Weiter bevorzugt umfassen die Partikel wenigstens ein erfindungsgemäßes 1H-Phenalen-1-on-Derivat, das an die Partikel kovalent gebunden vorliegt.

Die Erfindung wird nachfolgend durch Figuren und Beispiele erläutert, ohne hierauf beschränkt zu sein.

### Beispiel 1) - 19) Herstellung verschiedener 1H-Phenalen-1-on-Derivate.

### Überblick über die Synthesen

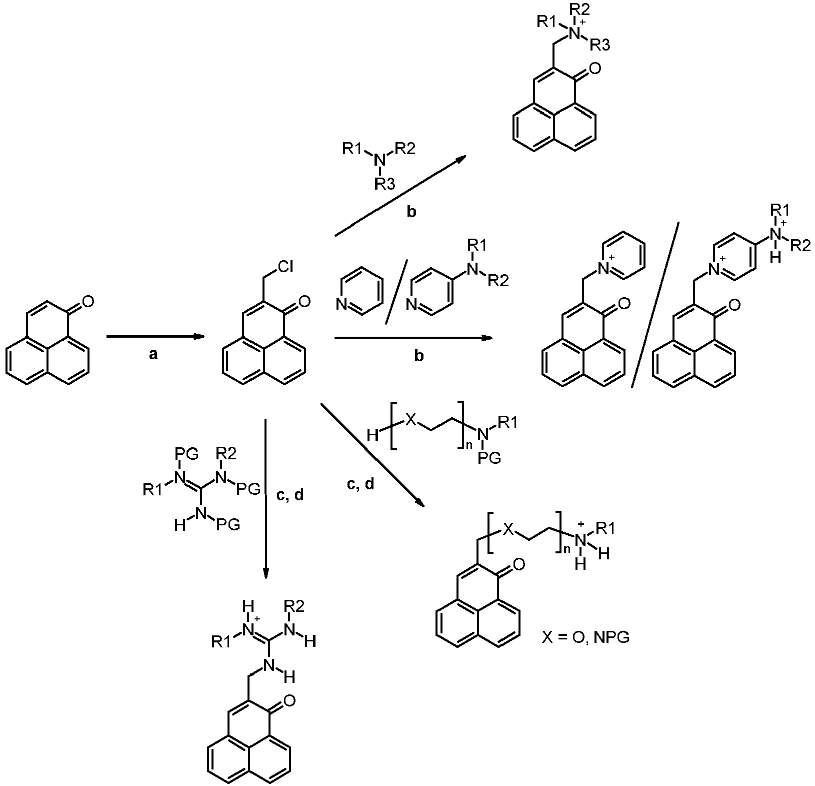

| | |
|---|---|
| Bedingungen: | *a) HCHO, HOAc, H₃PO₄, HCl, 110°C; b) DCM oder DMF oder MeCN, K₂CO₃ oder Cs₂CO₃ oder NaOH; c) NaOH, H₂O, DCM oder Toluol, TBAI; d) PG* = *Boc: HCl, RT, 3h oder PG* = *Cbz: Pd*/*C, H₂, 10bar, RT, 1d, dann HCl oder PG* = *Alloc: Tetrakis(triphenylphosphan)Pd(0), Nu⁻, 4h NPG = Stickstoffatom mit Schutzgruppe (PG)* |

### Konkrete Synthesen

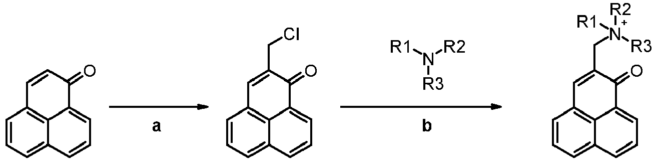

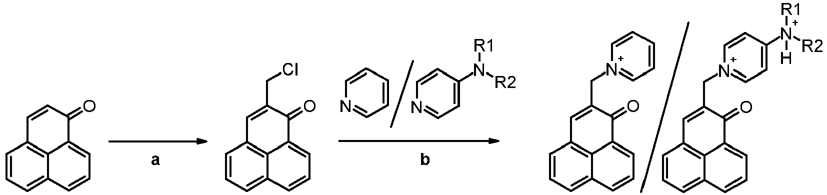

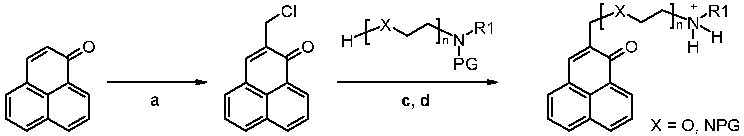

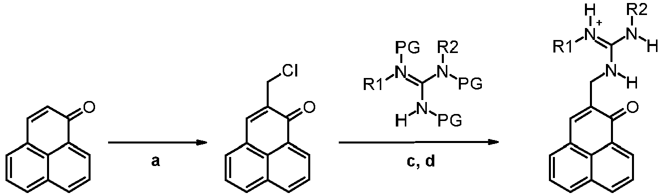

| | |
|---|---|
| Bedingungen: | *a) HCHO, HOAc, H₃PO₄, HCl, 110°C; b) DCM oder DMF oder MeCN, K₂CO₃ oder Cs*₂*CO₃ oder NaOH; c) NaOH, H₂O, DCM oder Toluol, TBAI; d) PG* = *Boc: HCl, RT, 3h oder PG* = *Cbz: Pd*/*C, H₂, 10bar, RT, 1d, dann HCl oder PG* = *Alloc: Tetrakis(triphenylphosphan)Pd(0), Nu*⁻, *4h* |

### Beispiel 1) 2-Chlormethyl-1H-phenalen-1-on

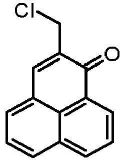

Perinaphthenon (1,725 g, 9,58 mmol) und Paraformaldehyd (5,49 g, 24 mmol) wurden in Essigsäure (40 mL) und Phosphorsäure (25 mL) bei 110 °C unter lebhaften Rühren erhitzt bis eine klare Lösung entstand. Anschließend wurde zu der gelben Lösung langsam und in kleinen Mengen wässrige HCl (32% w/w, 30 mL) zugegeben und weitere 8 h unter Rückfluss erhitzt bis eine klare braune Lösung entstand. Nach Abkühlen auf Raumtemperatur wurden 75 mL dest. Wasser zugesetzt und die Lösung mit K₂CO₃ neutralisiert. Das Reaktionsgemisch wurde mehrmals mit CH₂Cl₂ extrahiert, die organischen Phasen wurden vereinigt, über MgSO₄ getrocknet und das Lösungsmittel abgezogen. Das ölige Produkt wurde durch Säulenchromatographie (DCM/Petrolether (PE) 1:1, R_{f} = 0,3) aufgereinigt. Das Produkt kristallisiert als gelbes Pulver aus (1,54 g, 6,72 mmol, 70 % der Theorie).
**¹H-NMR** (300 MHz, CDCl₃): δ [ppm] = 4,68 (s, 2 H), 7,60 (dd, 1 H, *J* = 7 Hz, 8 Hz), 7,78 (m, 2 H), 7,92 (s, 1 H), 8,03 (d, 1 H, *J* = 8 Hz), 8,20 (d, 1 H, *J* = 7,5 Hz), 8,64 (d, 1 H, *J* = 8 Hz); - **¹³C-NMR** (75 MHz, CDCl₃): δ [ppm] = 41,5 (1 C), 126,8 (1 C), 127,2 (1 C), 127,2 (1 C), 127,3 (1 C), 129,0 (1 C), 130,9 (1 C), 132,0 (1 C), 132,1 (1 C), 132,2 (1 C), 135,2 (1 C), 135,6 (1 C), 140,4 (1 C), 183,5 (1 C); **- IR** (neat): v (cm⁻¹) = 2940 (m), 2870 (m), 1630 (m), 1570 (m), 1206 (s), 1150 (s), 1050 (m), 775 (m), 636 (m); - **MS** (ESI-MS, CH₂Cl₂/MeOH + 10 mmol NH₄OAc): e/z (%) = 229,1 (100, MH⁺), 115,0 (16, (M+2H⁺)²⁺); - **UV** (MeOH): λ (ε) = 249 (15400), 319 (2400), 360 (7300), 386 (6800);

### Beispiel 2) 2-Aminomethyl-1H-phenalen-1-on

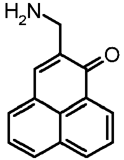

Kaliumphthalimid (93 mg, 0,5 mmol) wurde in trockenem DMF (1 mL) vorgelegt. Eine Lösung von 2-Chlormethyl-1H-Phenalen-1-on (114 mg, 0,5 mmol) in trockenem DMF (0,5 mL) wurde langsam zugetropft und das Reaktionsgemisch anschließend über Nacht bei Raumtemperatur gerührt. Nach Zugabe von 5 mL DMF wurde das Reaktionsgemisch 12 h bei 70°C erhitzt, dann wurden Ethanol (5 mL) und Hydrazin (30 µL) zugegeben und der Ansatz 2-3 h weiter unter Rückfluss gehalten (DC-Kontrolle (EtOH/Ethylacetat (EE) 3:1). Nach dem Abkühlen wurden die Lösungsmittel abgedampft und der Rückstand in wenig warmen Ethanol gelöst. Die Lösung wurde vom ungelösten Rest abfiltriert und bis zur Trockene eingedampft. Das Rohprodukt wurde säulenchromatographisch (EE/EtOH 3:1, EE/EtOH 1:1, reines EtOH) aufgereinigt. Es wurden 114 mg eines gelben, pulvrigen Feststoffes (0,335 mmol, 67 % der Theorie) erhalten.
**¹H-NMR** (300 MHz, MeOD): δ [ppm] = 4,70 (s, 2 H), 7,80 (dd, 1 H, *J* = 7 Hz, 8 Hz), 7,88 (m, 2 H), 7,97 (s, 1 H), 8,06 (d, 1 H, *J* = 8 Hz), 8,22 (d, 1 H, *J* = 8 Hz), 8,60 (d, 1 H, *J* = 8 Hz); - **IR** (neat): v (cm⁻¹) = 2946 (m), 2880 (m), 1632 (m), 1580 (m), 1208 (m), 1152 (s), 1054 (m), 776 (m), 634 (m); - **MS** (ESI-MS, CH₂Cl₂/MeOH + 10 mmol NH₄OAc): e/z (%) = 210,1 (100, MH⁺), 105,6 (8, (M+2H⁺)²⁺); - **UV** (MeOH): λ (ε) = 248 (15200), 318 (2300), 362 (7300), 384 (6600);

### Beispiel 3) Allgemeine Vorschrift zur phasentransferkatalytischen Umsetzung von 2-Chlormethyl-1H-phenalen-1-on mit Alkoholen

2-Chlormethyl-1H-phenalen-1-on (23 mg, 0,1 mmol) und ein geschützter Aminoalkohol (0,4 mmol), wie in den Beispielen 4a) und 5a) jeweils angegeben, wurden in Toluol (1 mL) gelöst. Wässrige Natronlauge (1 mL, 5 M, 5,0 mmol) wurde, gefolgt von Tetrabutylammoniumiodid (5 mg), zugegeben und der Ansatz 20 h lebhaft gerührt. Es wurde mit 10 mL DCM verdünnt, die wässrige Phase abgetrennt und die organische Phase noch zweimal mit Wasser (5 mL) gewaschen. Nach Trocknen über MgSO₄ wurde das Lösungsmittel abgezogen und der Rückstand durch Säulenchromatographie mit DCM/Ethanol 40:1 gereinigt.

Zur Entschützung der Boc-Gruppe wurde die jeweils erhaltene geschützte Stufe in DCM (0,5 mL) gelöst und mit einer gesättigten Lösung von HCl in Diethylether (0,5 mL) 2 h bei Raumtemperatur gerührt. Anschließend wurde das Produkt mit Diethylether ausgefällt, abzentrifugiert und bei vermindertem Druck getrocknet.

### Beispiel 4a) [2-(1-Oxo-1H-phenalen-2-ylmethoxy)-ethyl]-carbaminsäure-tert-butylester

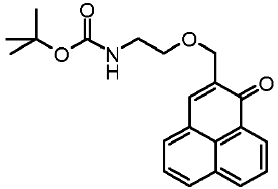

(2-Hydroxy-ethyl)-carbaminsäure-tert-butylester (64 mg, 0,4 mmol) wurde als geschützter Am inoalkohol wie in Beispiel 3) angegeben umgesetzt. Es wurden 32 mg eines gelben, zähen Feststoffes (0,091 mmol; 91 % der Theorie) erhalten.
**¹H-NMR** (300 MHz, CDCl₃): δ [ppm] = 1,43 (s, 9 H), 3,72 (d, 2 H, *J* = 6,4 Hz), 4,16 (d, 2 H, *J* = 6,4 Hz), 4,66 (s, 2 H), 5,18 (bs, 1 H), 7,62 (dd, 1 H, *J* = 7 Hz, 8 Hz), 7,76 (m, 2 H), 7,93 (s, 1 H), 8,05 (d, 1 H, *J* = 8 Hz), 8,22 (d, 1 H, *J* = 7,5 Hz), 8,62 (d, 1 H, *J* = 8 Hz); - **MS** (ESI-MS, CH₂Cl₂/MeOH + 10 mmol NH₄OAc): e/z (%) = 354,2 (100, MH⁺), 298,2 (86, MH⁺ - C₄H₉), 254,1 (53, MH⁺ - CO₂ - C₄H₉); - **UV** (MeOH): λ (ε) = 247 (15100), 319 (2400), 361 (7200), 385 (6800);

### Beispiel 4b) 2-((2-Aminoethoxy)methyl)-1H-phenalen-1-on Hydrochlorid

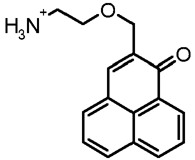

Von der in Beispiel 4a) erhaltenen geschützten Stufe wurden 32 mg (0,09 mmol) wie in Beispiel 3) angegeben entschützt. Es wurden 25 mg eines gelben Pulvers (0,086 mmol, 96 % der Theorie) erhalten.
**¹H-NMR** (300 MHz, MeOD): δ [ppm] = 3,75 (m, 2 H), 4,21 (m, 2 H), 4,69 (s, 2 H), 7,81 (dd, 1 H, *J* = 7 Hz, 8 Hz), 7,89 (m, 2 H), 7,96 (s, 1 H), 8,12 (d, 1 H, *J* = 8 Hz), 8,26 (d, 1 H, *J* = 7,5 Hz), 8,71 (d, 1 H, *J* = 8 Hz); - **MS** (ESI-MS, CH₂Cl₂/MeOH + 10 mmol NH₄OAc): e/z (%) = 254,1 (100, M⁺); - **UV** (MeOH): λ (ε) = 245 (15300), 318 (2200), 362 (7300), 383 (6800);

### Beispiel 5a) {2-[2-(1-Oxo-1H-phenalen-2-ylmethoxy)-ethoxy]-ethyl}-carbaminsäure-tert-butylester

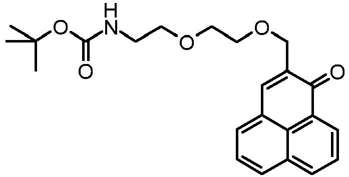

[2-(2-Hydroxy-ethoxy)-ethyl] -carbaminsäure-tert-butylester (82 mg, 0,4 mmol) wurde als geschützter Am inoalkohol wie in Beispiel 3) angegeben umgesetzt. Es wurden 35 mg eines gelben, zähen Feststoffes (0,088 mmol, 88 % der Theorie) erhalten.
**¹H-NMR** (300 MHz, CDCl₃): δ [ppm] = 1,42 (s, 9 H), 3,11 (d, 2 H, *J* = 6,4 Hz), 3,32 (d, 2 H, *J* = 6,4 Hz), 3,54 (d, 2 H, *J* = 6,4 Hz), 4,09 (d, 2 H, *J* = 6,4 Hz), 4,64 (s, 2 H), 5,14 (bs, 1 H), 7,64 (dd, 1 H, *J* = 7 Hz, 8 Hz), 7,70 (m, 2 H), 7,87 (s, 1 H), 8,02 (d, 1 H, *J* = 8 Hz), 8,31 (d, 1 H, *J* = 7,5 Hz), 8,58 (d, 1 H, *J* = 8 Hz); - **MS** (ESI-MS, CH₂Cl₂/MeOH + 10 mmol NH₄OAc): e/z (%) = 398,2 (100, MH⁺), 342,2 (71, MH⁺-C₄H₉), 298,1 (46, MH⁺ - CO₂ - C₄H₉); - **UV** (MeOH): λ (ε) = 248 (15200), 317 (2300), 362 (7400), 383 (6600);

### Beispiel 5b) 2-((2-(2-Aminoethoxy)ethoxy)methyl)-1H-phenalen-1-on Hydrochlorid

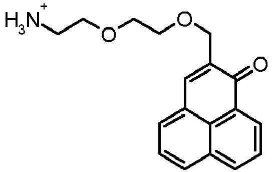

Von der in Beispiel 5a) erhaltenen geschützten Stufe wurden 32 mg (0,08 mmol) wie in Beispiel 3) angegeben entschützt. Es wurden 23 mg eines gelben Pulver (84 % der Theorie; 0,067 mmol) erhalten.
**¹H-NMR** (300 MHz, MeOD): δ [ppm] = 3,16 (m, 2 H), 3,34 (d, 2 H*, J* = 6,4 Hz), 3,58 (d, 2 H, *J* = 6,4 Hz), 4,13 (d, 2 H, *J* = 6,4 Hz), 4,65 (s, 2 H), 7,83 (dd, 1 H, *J* = 7 Hz, 8 Hz), 7,87 (m, 2 H), 7,98 (s, 1 H), 8,13 (d, 1 H, *J* = 8 Hz), 8,24 (d, 1 H, *J* = 7,5 Hz), 8,66 (d, 1 H, *J* = 8 Hz); - **MS** (ESI-MS, CH₂Cl₂/MeOH + 10 mmol NH₄OAc): e/z (%) = 298,1 (100, M⁺); - **UV** (MeOH): λ (ε) = 246 (15200), 316 (2200), 364 (7400), 386 (6800);

### Beispiel 6) Phasentransferkatalytische Umsetzung von 2-Chlormethyl-1H-phenalen-1-on mit Guanidinen

2-Chlormethyl-1H-Phenalen-1-on (23 mg, 0,1 mmol) und N,N',N"-tri-Boc-guanidin (144 mg, 0,4 mmol) wurden in Toluol (1 mL) gelöst. Wässrige Natronlauge (1 mL, 5M, 5,0 mmol) wurde, gefolgt von Tetrabutylammoniumiodid (5 mg), zugegeben und der Ansatz 20 h lebhaft gerührt. Es wurde mit 10 mL DCM verdünnt, die wässrige Phase abgetrennt und die organische Phase noch zweimal mit Wasser (5 mL) gewaschen. Nach Trocknen über MgSO₄ wurde das Lösungsmittel abgezogen und der Rückstand durch Säulenchromatographie mit DCM/Ethanol 40:1 gereinigt.

Zur Entschützung der Boc-Gruppe wurde das Reinprodukt in DCM (0,5 mL) gelöst und mit einer gesättigten Lösung von HCl in Diethylether (0,5 mL) 2 h bei Raumtemperatur gerührt. Anschließend wurde das Produkt mit Diethylether ausgefällt, abzentrifugiert und bei vermindertem Druck getrocknet.

### Beispiel 6a) N,N',N"-Tri(tert-butoxycarbonyl)-N-(1-oxo-1H-phenalen-2-ylmethyl)-guanidin

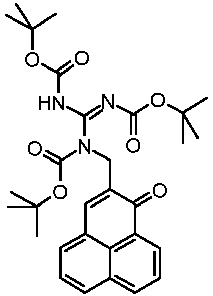

Es wurden 51 mg eines gelben, zähen Feststoffes (0,092 mmol, 92 % der Theorie) erhalten.
**¹H-NMR** (300 MHz, CDCl₃): δ [ppm] = 1,41 (s, 9 H), 1,43 (s, 9 H), 1,44 (s, 9 H), 4,62 (s, 2 H), 7,65 (dd, 1 H, *J* = 7 Hz, 8 Hz), 7,78 (m, 2 H), 7,91 (s, 1 H), 8,02 (d, 1 H, *J* = 8 Hz), 8,17 (d, 1 H, *J* = 7,5 Hz), 8,67 (d, 1 H, *J* = 8 Hz); - **MS** (ESI-MS, CH₂Cl₂/MeOH + 10 mmol NH₄OAc): e/z (%) = 552,2 (100, MH⁺), 496,2 (61, MH⁺ - C₄H₉), 452,1 (46, MH⁺ - CO₂ - C₄H₉); - **UV** (MeOH): λ (ε) = 246 (15200), 320 (2300), 360 (7300), 384 (6700);

### Beispiel 6b) N-(1-oxo-1H-phenalen-2-ylmethyl)-guanidin Hydrochlorid

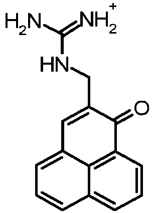

Von der in Beispiel 6a) erhaltenen geschützten Stufe wurden 50 mg (0,09 mmol) wie in Beispiel 6) angegeben entschützt. Es wurden 26 mg eines gelben Pulvers (0,091 mmol; 91 % der Theorie) erhalten.
**¹H-NMR** (300 MHz, MeOD): δ [ppm] = 4,61 (s, 2 H), 7,81 (dd, 1 H, *J* = 7 Hz, 8 Hz), 7,89 (m, 2 H), 7,96 (s, 1 H), 8,17 (d, 1 H, *J* = 8 Hz), 8,34 (d, 1 H, *J* = 7,5 Hz), 8,72 (d, 1 H, *J* = 8 Hz); - **MS** (ESI-MS, CH₂Cl₂/MeOH + 10 mmol NH₄OAc): e/z (%) = 452,1 (100, M⁺); - **UV** (MeOH): λ (ε) = 245 (15200), 321 (2300), 366 (7200), 385 (6700);

### Beispiel 7) Synthese von 2-(4-Pyridinyl)methyl)-1H-phenalen-1-on

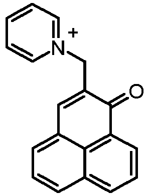

2-Chlormethyl-1H-Phenalen-1-on (182 mg, 0,8 mmol) in Ethanol (4,0 mL) wurden mit Pyridin (316 µL, 4,0 mmol) versetzt und über Nacht bei 80 °C unter Rückfluss erhitzt. Nach dem Abkühlen wurde das Lösungsmittel und überschüssiges Pyridin abgezogen. Zur weiteren Reinigung wurde das Produkt in DCM (0,5 mL) gelöst und mit Diethylether gefällt (10 mL). Es wurden 230 mg eines blassgelben Pulver (0,749 mmol; 94 % der Theorie) erhalten.
HPLC: Gradienten-Eluation in Wasser [0,0059 w% Trifluoressigsäure] (Lösung A) und Acetonitril [0,0059 w% Trifluoressigsäure] (Lösung B). [t(min), %B]: (0, 5), (30, 98): Produkt-Peak bei Raumtemperatur: 7,963 min; Reinheit: 95,8%
**¹H-NMR** (300 MHz, DMSO-d6): δ [ppm] = 5,92 (s, 2 H), 7,80 (dd, 1 H, *J* = 7 Hz, 8 Hz), 7,88 (dd, 1 H, *J* = 7,6 Hz, 8,5 Hz), 8,20 (m, 3 H), 8,30 (d, 1 H, *J* = 7,6 Hz), 8,63 (m, 3 H), 8,68 (d, 1 H, *J* = 8 Hz), 9,31 (m, 2 H); - **¹³C-NMR** (75 MHz, DMSO-d6): δ [ppm] = 59,6 (-, 1 C), 125,9 (C_{quat}, 1 C), 126,5 (C_{quat}, 1 C), 127,3 (+, 1 C), 127,5 (+, 2 C), 127,8 (+, 2 C), 127,9 (C_{quat}, 1 C), 130,4 (+, 1 C), 131,5 (C_{quat}, 1 C), 131,6 (C_{quat}, 1 C), 133,4 (+, 1 C), 133,9 (+, 1 C), 136,1 (+, 1 C), 144,1 (+, 1 C), 145,3 (+, 1 C), 145,8 (+, 1 C), 171,9 (C_{quat}, 1 C), 182,9 (C_{quat}, 1 C); - **MS** (ESI-MS, CH₂Cl₂/MeOH + 10 mmol NH₄OAc): e/z (%) = 272,1 (100, MH⁺); - **UV** (MeOH): λ (ε) = 224 (21600), 248 (17400), 317 (2600), 363 (7200), 386 (6900);

### Beispiel 8) Allgemeine Vorschrift zur Umsetzung von 2-Chlormethyl-1H-Phenalen-1-on mit Aminen

2-Chlormethyl-1H-Phenalen-1-on (23 mg, 0,1 mmol) und ein Amin (0,4 mmol), wie in den Beispielen 9) bis 14) und 15a) bis 19a) jeweils angegeben, wurden in Acetonitril (1,0 mL) gelöst. Wasser (0,02 mL) und Kaliumcarbonat (70 mg, 0,5 mmol) wurden zugegeben und der Ansatz wird 30 h bei 80 °C unter Rückfluss erhitzt. Nach dem Abkühlen wurde das Lösungsmittel und alle flüchtigen Komponenten abgezogen.

In den Beispielen 9) bis 14) wurde das Endprodukt in DCM (0,5 mL) gelöst und mit Diethylether gefällt (10 mL).

In den Beispielen 15a) bis 19a) wurde der Rückstand durch Säulenchromatographie mit DCM/Ethanol 40:1 gereinigt.

Nachfolgend wurde in den Beispielen 15a) bis 19a) zur Entschützung der Boc-Gruppe jeweils die geschützte Stufe in DCM (0,5 mL) gelöst und mit einer gesättigten Lösung von HCl in Diethylether (0,5 mL) 2 h bei Raumtemperatur gerührt. Anschließend wurde das Produkt mit Diethylether ausgefällt und abzentrifugiert. Zur weiteren Reinigung wurde das Produkt in DCM (0,5 mL) gelöst und mit Diethylether erneut gefällt (10 mL). Zuletzt wurde bei vermindertem Druck getrocknet.

### Beispiel 9) 2-((N,N-Diethyl)aminomethyl)-1H-phenalen-1-on Hydrochlorid

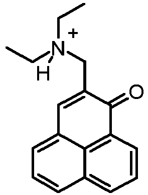

Diethylamin (29 mg, 0,4 mmol) wurde als Amin wie in Beispiel 8) angegeben umgesetzt. Das erhaltene Rohprodukt wurde durch Säulenchromatographie mit DCM/EtOH 6:1 gereinigt und mit HCl protoniert. Es wurden 18 mg eines gelben, zähen Feststoffes (0,06 mmol; 60 % der Theorie) erhalten.
**¹H-NMR** (300 MHz, MeOD): δ [ppm] = 1,42 (t, 6 H, *J* = 7,1 Hz), 3,14 (q, 4 H, *J* = 7,1 Hz), 4,68 (s, 2 H), 7,87 (dd, 1 H, *J* = 7 Hz, 8 Hz), 7,81 (dd, 1 H, *J* = 7,6 Hz, 8,5 Hz), 8,10 (m, 1 H), 8,26 (d, 1 H, *J* = 7,6 Hz), 8,62 (d, 1 H, *J* = 8 Hz), 9,31 (m, 2 H); - **MS** (ESI-MS, CH₂Cl₂/MeOH + 10 mmol NH₄OAc): e/z (%) = 267,1 (100, M⁺); - **UV** (MeOH): λ (ε) = 246 (15100), 319 (2200), 365 (7100), 388 (6700);

### Beispiel 10) 2-((N,N,N-Triethyl)aminomethyl)-1H-phenalen-1-on Hydrochlorid

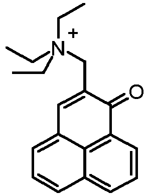

Triethylamin (40 mg, 0,4 mmol) wurde als Amin wie in Beispiel 8) angegeben umgesetzt. Es wurden 24 mg eines gelben, zähen Feststoffes (0,076 mmol; 76 % der Theorie) erhalten.
**¹H-NMR** (300 MHz, CDCl₃): δ [ppm] = 1,04 (t, 9 H, *J* = 7,1 Hz), 2,57 (q, 6 H, *J* = 7,1 Hz), 5,24 (s, 2 H), 7,72 (dd, 1 H, *J* = 7 Hz, 8 Hz), 7,83 (dd, 1 H, *J* = 7,6 Hz, 8,5 Hz), 8,14 (m, 1 H), 8,22 (d, 1 H, *J* = 7,6 Hz), 8,65 (d, 1 H, *J* = 8 Hz), 9,26 (m, 2 H); - **MS** (ESI-MS, CH₂Cl₂/MeOH + 10 mmol NH₄OAc): e/z (%) = 295,1 (100, M⁺); - **UV** (MeOH): λ (ε) = 244 (15200), 319 (2200), 360 (7200), 384 (6700);

### Beispiel 11) 2-{[Tris-(2-hydroxy-ethyl)-amino]-methyl}-phenalen-1-on Hydrochlorid

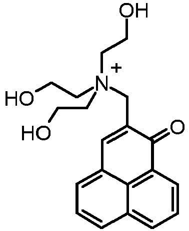

Triethanolamin (61 mg, 0,4 mmol) wurde als Amin wie in Beispiel 8) angegeben umgesetzt. Es wurden 27 mg eines gelben, zähen Feststoffes (0,071 mmol; 71 % der Theorie) erhalten.
**¹H-NMR** (300 MHz, CDCl₃): δ [ppm] = 2,54 (t, 6 H, *J* = 7,1 Hz), 3,66 (t, 6 H, *J* = 7,1 Hz), 3,76 (bs, 3 H), 4,21 (s, 2 H), 7,39 (dd, 1 H, *J* = 7 Hz, 8 Hz), 7,61 (dd, 1 H, *J* = 7,6 Hz, 8,5 Hz), 7,73 (m, 1 H), 7,92 (d, 1 H, *J* = 7,6 Hz), 8,07 (d, 1 H, *J* = 8 Hz), 8,18 (m, 1 H), 8,22 (m, 1 H); - **MS** (ESI-MS, CH₂Cl₂/MeOH + 10 mmol NH₄OAc): e/z (%) = 343,1 (100, M⁺); - **UV** (MeOH): λ (ε) = 244 (15000), 320 (2300), 362 (7300), 386 (6800);

### Beispiel 12) 2-Morpholin-4-ylmethyl-phenalen-1-on Hydrochlorid

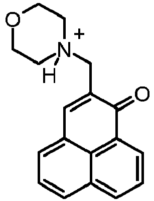

Morpholin (34 mg, 0,4 mmol) wurde als Amin wie in Beispiel 8) angegeben umgesetzt. Das Rohprodukt wurde durch Säulenchromatographie mit DCM/EtOH 6:1 gereinigt und mit HCl protoniert. Es wurden 18 mg eines gelben, zähen Feststoffes (0,081 mmol; 81 % der Theorie) erhalten.
**¹H-NMR** (300 MHz, CDCl₃): δ [ppm] = 3,16 (m, 4 H), 4,01 (m, 4 H), 4,29 (s, 2 H), 7,63 (dd, 1 H, *J* = 7 Hz, 8 Hz), 7,79 (m, 2 H), 8,02 (d, 1 H, *J* = 8 Hz), 8,13 (m, 1 H), 8,24 (dd, 1 H, *J* = 7,6 Hz, 8,5 Hz), 8,63 (d, 1 H, *J* = 8,5 Hz), 8,81 (s, 1 H); - **MS** (ESI-MS, CH₂Cl₂/MeOH + 10 mmol NH₄OAc): e/z (%) = 281,1 (100, M⁺); - **UV** (MeOH): λ (ε) = 246 (15200), 322 (2200), 363 (7200), 384 (6700);

### Beispiel 13) 2-(4-Methyl-piperazin-1-ylmethyl)-phenalen-1-on Hydrochlorid

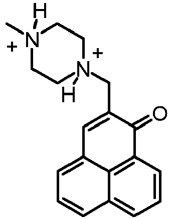

1-N-Methyl-piperazin (29 mg, 0,4 mmol) wurde als Amin wie in Beispiel 8) angegeben umgesetzt. Das Rohprodukt wurde durch Säulenchromatographie mit DCM/EtOH 6:1 gereinigt und mit HCl protoniert. Es wurden 24 mg eines gelben Feststoffes (0,064 mmol; 64 % der Theorie) erhalten.
**¹H-NMR** (300 MHz, CDCl₃): δ [ppm] = 2,33 (s, 3H), 2,78 (m, 2 H), 3,21 (m, 2 H), 5,11 (s, 2 H), 7,58 (dd, 1 H, *J* = 7 Hz, 8 Hz), 7,78 (m, 2 H), 8,02 (m, 1 H), 8,13 (d, 1 H, *J* = 8 Hz), 8,58 (dd, 1 H, *J* = 7,5 Hz, 8,5 Hz), 8,91 (m, 1 H); - **MS** (ESI-MS, CH₂Cl₂/MeOH + 10 mmol NH₄OAc): e/z (%) = 294,1 (100, M⁺); - **UV** (MeOH): λ (ε) = 246 (15100), 318 (2200), 364 (7300), 388 (6700);

### Beispiel 14) 2-(4-Dimethylamino-piperidin-1-ylmethyl)-phenalen-1-on Hydrochlorid

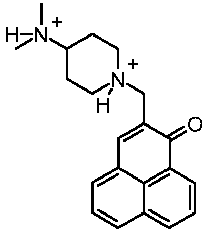

4-Dimethylaminopiperidin (42 mg, 0,4 mmol) wurde als Amin wie in Beispiel 8) angegeben umgesetzt. Das Rohprodukt wurde durch Säulenchromatographie mit DCM/EtOH 6:1 gereinigt und mit HCl protoniert. Es wurden 25 mg eines gelben Feststoffes (0,067 mmol; 67 % der Theorie) erhalten.
**¹H-NMR** (300 MHz, CDCl₃): δ [ppm] = 1,76 (m, 5H), 2,38 (s, 6 H), 2,23 (m, 2 H), 3,61 (m, 2 H), 6,32 (s, 2 H), 7,69 (dd, 1 H, *J* = 7 Hz, 8 Hz), 7,82 (dd, 1 H, *J* = 7,6 Hz, 8,5 Hz), 8,12 (d, 1 H, *J* = 8 Hz), 8,17 (d, 1 H, *J* = 8 Hz), 8,26 (d, 1 H, *J* = 7,5 Hz), 8,70 (d, 1 H, *J* = 8 Hz), 9,42 (s, 1 H); - **MS** (ESI-MS, CH₂Cl₂/MeOH + 10 mmol NH₄OAc): e/z (%) = 322,1 (100, M⁺); - **UV** (MeOH): λ (ε) = 245 (15100), 319 (2400), 363 (7200), 386 (6800);

### Beispiel 15a) ((2-tert-Butoxycarbonylamino-ethyl)-{2-[(1-oxo-1H-phenalen-2-ylmethyl)-amino]-ethyl}-amino)-essigsäure-tert-butylester

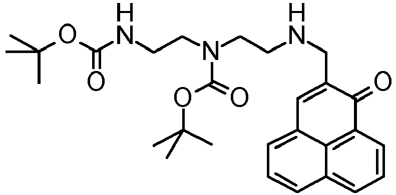

[(2-Amino-ethyl)-(2-tert-butoxycarbonylamino-ethyl)-amino]-essigsäure-tert-butylester (116 mg, 0,4 mmol) wurde als Amin wie in Beispiel 8) angegeben umgesetzt. Es wurden 26 mg eines gelben, wachsartigen Feststoffes (0,057 mmol; 57 % der Theorie) erhalten.
**¹H-NMR** (300 MHz, CDCl₃): δ [ppm] = 1,41 (s, 9 H), 1,43 (s, 9 H), 2,93 (m, 2 H), 3,21 - 3,58 (m, 8 H), 3,91 (s, 2 H), 5,58 (bs, 1 H), 6,02 (bs, 1 H), 7,58 (dd, 1 H, *J* = 7 Hz, 8 Hz), 7,81 (m, 3 H), 8,01 (d, 1 H, *J* = 8 Hz), 8,22 (d, 1 H, *J* = 7,5 Hz), 8,62 (d, 1 H, *J* = 8 Hz); - **MS** (ESI-MS, CH₂Cl₂/MeOH + 10 mmol NH₄OAc): e/z (%) = 510,2 (100, MH⁺), 454,2 (49, MH⁺ - C₄H₉), 410,1 (21, MH⁺ - CO₂ - C₄H₉); - **UV** (MeOH): λ (ε) = 245 (15200), 318 (2200), 362 (7300), 386 (6800);

### Beispiel 15b) 2-{[2-(2-Amino-ethylamino)-ethylamino]-methyl}-phenalen-1-on Hydrochlorid

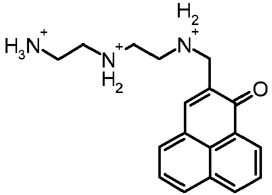

Von der in Beispiel 15a) erhaltenen geschützten Stufe wurden 26 mg (0,05 mmol) wie in Beispiel 8) angegeben entschützt. Es wurden 17 mg eines gelben Pulvers (0,045 mmol; 90 % der Theorie) erhalten.
**¹H-NMR** (300 MHz, MeOD): δ [ppm] = 2,96 (m, 2 H), 3,16 - 3,72 (m, 6 H), 4,12 (s, 2 H), 7,63 (dd, 1 H, *J* = 7 Hz, 8 Hz), 7,88 (m, 3 H), 8,06 (d, 1 H, *J* = 8 Hz), 8,17 (d, 1 H, *J* = 7,5 Hz), 8,67 (d, 1 H, *J* = 8 Hz); - **MS** (ESI-MS, CH₂Cl₂/MeOH + 10 mmol NH₄OAc): e/z (%) = 310,1 (100, M⁺); - **UV** (MeOH): λ (ε) = 242 (15400), 317 (2500), 361 (7100), 383 (6900);

### Beispiel 16a) ([2-({2-[(2-tert-Butoxycarbonylamino-ethyl)-tert-butoxycarbonylmethyl-amino]-ethyl}-tert-butoxycarbonylmethyl-amino)-ethyl]-{2-[(1-oxo-1H-phenalen-2-ylmethyl)-amino]-ethyl}-amino)-essigsäure-tert-butylester

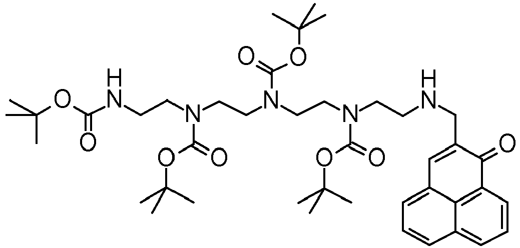

{(2-Amino-ethyl)-[2-({2-[(2-tert-butoxycarbonylamino-ethyl)-tert-butoxycarbonylmethyl-amino]-ethyl}-tert-butoxycarbonylmethyl-amino)-ethyl]-amino}-essigsäure-tert-butylester (240 mg, 0,4 mmol) wurde als Amin wie in Beispiel 8) angegeben umgesetzt. Es wurden 33 mg eines gelben, zähen Feststoffes (39 % der Theorie; 0,039 mmol) erhalten.
**¹H-NMR** (300 MHz, CDCl₃): δ [ppm] = 1,41 (s, 18 H), 1,43 (s, 18 H), 2,53 (m, 6 H), 3,21 - 3,54 (m, 10 H), 3,93 (s, 2 H), 4,46 (bs, 1 H), 5,02 (bs, 1 H), 5,31 (bs, 1 H), 5,74 (bs, 1 H), 7,56 (dd, 1 H, *J* = 7 Hz, 8 Hz), 7,83 (m, 3 H), 8,03 (d, 1 H, *J* = 8 Hz), 8,20 (d, 1 H, *J* = 7,5 Hz), 8,65 (d, 1 H, *J* = 8 Hz); - **MS** (ESI-MS, CH₂Cl₂/MeOH + 10 mmol NH₄OAc): e/z (%) = 823,2 (100, MH⁺), 767,2 (78, MH⁺ - C₄H₉), 723,1 (13, MH⁺ -CO₂ - C₄H₉); - **UV** (MeOH): λ (ε) = 2465 (15100), 322 (2400), 360 (7300), 384 (7000);

### Beispiel 16b) 2-[(2-{2-[2-(2-Amino-ethylamino)-ethylamino]-ethylamino}-ethylamino)-methyl]-phenalen-1-on Hydrochlorid

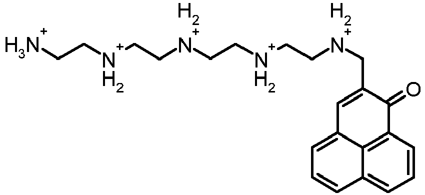

Von der in Beispiel 16a) erhaltenen geschützten Stufe wurden 27 mg (0,033 mmol) wie in Beispiel 8) angegeben entschützt. Es wurden 17 mg eines gelben Pulvers (0,029 mmol; 88 % der Theorie) erhalten.
**¹H-NMR** (300 MHz, MeOD): δ [ppm] = 2,56 (m, 6 H), 3,18 - 3,57 (m, 8 H), 3,98 (s, 2 H), 7,58 (dd, 1 H, *J* = 7 Hz, 8 Hz), 7,86 (m, 3 H), 8,07 (d, 1 H, *J =* 8 Hz), 8,18 (d, 1 H, *J* = 7,5 Hz), 8,62 (d, 1 H, *J* = 8 Hz); - **MS** (ESI-MS, CH₂Cl₂/MeOH + 10 mmol NH₄OAc): e/z (%) = 386,1 (100, M⁺); - **UV** (MeOH): λ (ε) = 245 (15500), 321 (2500), 361 (7200), 385 (6900);

### Beispiel 17a) N,N'-tert-Butoxycarbonyl-N"-{2-[(1-oxo-1H-phenalen-2-ylmethyl)-amino]-ethyl}-guanidin

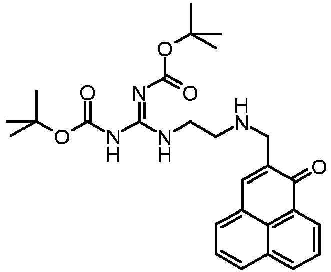

N,N'-tert-Butoxycarbonyl-N"-(2-amino-ethyl)-guanidin (106 mg, 0,4 mmol) wurde als Amin wie in Beispiel 8) angegeben umgesetzt. Es wurden 14 mg eines gelben, zähen Feststoffes (0,037 mmol; 37 % der Theorie) erhalten.
**¹H-NMR** (300 MHz, CDCl₃): δ [ppm] = 1,42 (s, 18 H), 2,62 (m, 2 H), 3,38 (m, 2 H), 4,63 (s, 2 H), 7,58 (dd, 1 H, *J* = 7 Hz, 8 Hz), 7,78 (m, 2 H), 7,94 (s, 1 H), 8,03 (d, 1 H, *J* = 8 Hz), 8,18 (d, 1 H, *J* = 7,5 Hz), 8,63 (d, 1 H, *J* = 8 Hz); - **MS** (ESI-MS, CH₂Cl₂/MeOH + 10 mmol NH₄OAc): e/z (%) = 494,2 (100, MH⁺), 438,2 (62, MH⁺ -C₄H₉), 394,1 (34, MH⁺ - CO₂ - C₄H₉); - **UV** (MeOH): λ (ε) = 244 (15100), 319 (2200), 360 (7200), 384 (6700);

### Beispiel 17b) N"-{2-[(1-Oxo-1H-phenalen-2-ylmethyl)-amino]-ethyl}-guanidin Hydrochlorid

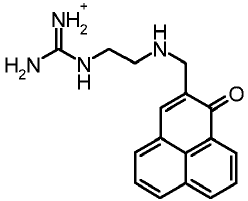

Von der in Beispiel 17a) erhaltenen geschützten Stufe wurden 12 mg (0,033 mmol) wie in Beispiel 8) angegeben entschützt. Es wurden 10 mg eines gelben Pulvers (0,027 mmol; 81 % der Theorie) erhalten.
**¹H-NMR** (300 MHz, MeOD): δ [ppm] = 2,64 (m, 2 H), 3,42 (m, 2 H), 4,62 (s, 2 H), 7,56 (dd, 1 H, *J* = 7 Hz, 8 Hz), 7,82 (m, 2 H), 7,91 (s, 1 H), 8,05 (d, 1 H, *J* = 8 Hz), 8,21 (d, 1 H, *J* = 7,5 Hz), 8,61 (d, 1 H, *J* = 8 Hz); - **MS** (ESI-MS, CH₂Cl₂/MeOH + 10 mmol NH₄OAc): e/z (%) = 295,1 (100, M⁺); - **UV** (MeOH): λ (ε) = 244 (15400), 321 (2200), 360 (7400), 386 (6800);

### Beispiel 18a) 2-{[tris-(2-tert-Butoxycarbonyl-amino-ethyl)-amino]-methyl}-phenalen-1-on Hydrochlorid

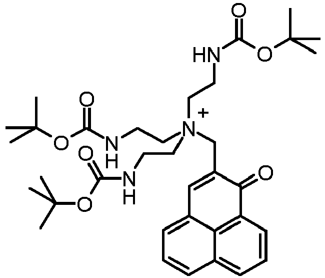

tris-(2-tert-Butoxycarbonyl-amino-ethyl)-amin (164 mg, 0,4 mmol) wurde als Amin wie in Beispiel 8) angegeben umgesetzt. Es wurden 48 mg eines gelben Feststoffes (0,073 mmol; 73 % der Theorie) erhalten.
**¹H-NMR** (300 MHz, CDCl₃): δ [ppm] = 1,42 (s, 27 H), 2,53 (m, 6 H), 3,49 (m, 6 H), 4,68 (s, 2 H), 7,65 (dd, 1 H, *J* = 7 Hz, 8 Hz), 7,77 (m, 3 H), 8,03 (d, 1 H, *J* = 8 Hz), 8,21 (d, 1 H, *J* = 7,5 Hz), 8,61 (d, 1 H, *J* = 8 Hz); - **MS** (ESI-MS, CH₂Cl₂/MeOH + 10 mmol NH₄OAc): e/z (%) = 640,1 (100, MH⁺), 584,2 (67, MH⁺ - C₄H₉), 540,1 (24, MH⁺ -CO₂ - C₄H₉); - **UV** (MeOH): λ (ε) = 246 (15300), 316 (2300), 359 (7400), 388 (7100);

### Beispiel 18b) 2-{[tris-(2-Amino-ethyl)-amino]-methyl}-phenalen-1-on Hydrochlorid

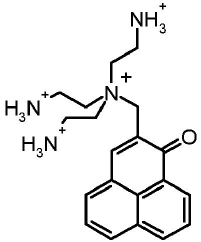

Von der in Beispiel 18a) erhaltenen geschützten Stufe wurden 46 mg (0,07 mmol) wie in Beispiel 8) angegeben entschützt. Es wurden 30 mg eines gelben Pulvers (89 % der Theorie; 0,062 mmol) erhalten.
**¹H-NMR** (300 MHz, MeOD): δ [ppm] = 2,51 (m, 6 H), 3,53 (m, 6 H), 4,66 (s, 2 H), 7,62 (dd, 1 H, *J* = 7 Hz, 8 Hz), 7,74 (m, 3 H), 8,05 (d, 1 H, *J* = 8 Hz), 8,23 (d, 1 H, *J* = 7,5 Hz), 8,67 (d, 1 H, *J* = 8 Hz); - **UV** (MeOH): λ (ε) = 246 (15500), 321 (2600), 360 (7200), 385 (6900);

### Beispiel 19a) 2-{[tris-(2-(N,N'-tert-Butoxycarbonyl-guanidino)ethyl)-amino]-methyl}-phenalen-1-on Hydrochlorid

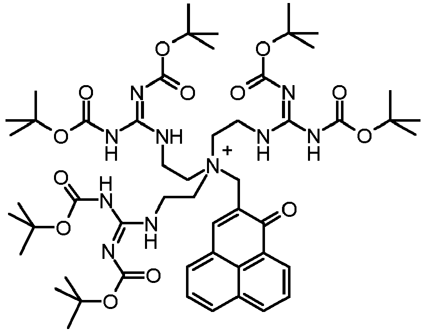

tris-(2-(N,N'-tert-Butoxycarbonyl-guanidino)ethyl)-amin (330 mg, 0,4 mmol) wurde als Amin wie in Beispiel 8) angegeben umgesetzt. Es wurden 81 mg eines gelben, zähen Feststoffes (78 % der Theorie; 0,078 mmol) erhalten.
**¹H-NMR** (300 MHz, CDCl₃): δ [ppm] = 1,41 (s, 27 H), 1,44 (s, 27 H), 2,48 (m, 6 H), 3,47 (m, 6 H), 4,71 (s, 2 H), 7,66 (dd, 1 H, *J* = 7 Hz, 8 Hz), 7,79 (m, 3 H), 8,01 (d, 1 H, *J* = 8 Hz), 8,25 (d, 1 H, *J* = 7,5 Hz), 8,57 (d, 1 H, *J* = 8 Hz); - **MS** (ESI-MS, CH₂Cl₂/MeOH + 10 mmol NH₄OAc): e/z (%) = 1066,2 (100, MH⁺), 1010,2 (58, MH⁺ -C₄H₉), 966,1 (37, MH⁺ - CO₂ - C₄H₉); - **UV** (MeOH): λ (ε) = 246 (15400), 319 (2500), 363 (7500), 384 (6900);

### Beispiel 19b) 2-{[tris-(2-Guanidino-ethyl)-amino]-methyl}-phenalen-1-on Hydrochlorid

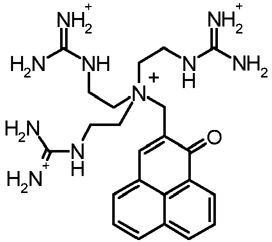

Von der in Beispiel 19a) erhaltenen geschützten Stufe wurden 73 mg (0,07 mmol) wie in Beispiel 8) angegeben entschützt. Es wurden 35 mg eines gelben Pulvers (0,058 mmol; 83 % der Theorie) erhalten.
**¹H-NMR** (300 MHz, MeOD): δ [ppm] = 2,56 (m, 6 H), 3,53 (m, 6 H), 4,66 (s, 2 H), 7,68 (dd, 1 H, *J* = 7 Hz, 8 Hz), 7,84 (m, 3 H), 8,04 (d, 1 H, *J* = 8 Hz), 8,22 (d, 1 H, *J =* 7,5 Hz), 8,60 (d, 1 H, *J* = 8 Hz); - **UV** (MeOH): λ (ε) = 245 (15100), 317 (2200), 361 (7200), 385 (6900);

### Beispiel 20) Bestimmung der photodynamischen Aktivität gegenüber Gram-positiven und Gram-negativen Bakterien.

### Herstellung der Testplatten und Bakterienstämme

Eine Probe des Bakterienstammes *Staphylococcus aureus* (ATCC-Nummer: 25923) *oder Escherichia coli* (ATCC-Nummer: 25922) wurde aus einer Cryo-Gefrierkultur entnommen, auf Müller-Hinton-Agar-Platten vereinzelt, und unter aeroben Bedingungen bei 37°C in einer Übernachtkultur kultiviert. Anschließend wurden 10 ml Müller-Hinton-Flüssigmedium mit einem Abstrich der Bakterienkultur (Einzelkolonie) beimpft und über Nacht bei 37°C bebrütet. Die so gewonnene Bakteriensuspension wurde 15 min bei 3000 Upm zentrifugiert und das erhaltene Bakterienpellet in 10 ml sterilem PBS resuspendiert. Die optische Dichte der Bakteriensupensionen für die Phototoxizitätstests betrug OD₆₀₀ₙₘ= 0.6, was einer Bakterienzahl von ∼ 8x10⁸ - 10¹² Bakterien pro ml entspricht. Die biochemische Analyse und Resistenzbestimmung der Bakterien wurde mit dem VITEK2-System gemäß den Richtlinien M100-S14 des NCCLS (National Committee for Clinical Laboratory Standards guidelines 2004) durchgeführt.

Zur Empfindlichkeitsprüfung medizinisch bedeutender Krankheitserreger gegenüber Antibiotika und Sulfonamiden wurden gemäß der NCCLS Richtlinien Müller-Hinton-Medien mit folgender Zusammensetzung verwendet (Deutsche Gesellschaft für Hygiene und Mikrobiologie (DGHM), Institut für Hygiene und Mikrobiologie, Universität Bonn, Deutschland):
a) Müller-Hinton-Bouillon (Oxoid, Wesel, Deutschland) Inhalt: 2,0 g/l Rindfleisch, getrocknete Infusion aus 300 g, 17,5 g/l Caseinhydrolysat, 1,5 g/l Stärke, pH: 7,4 + 0,2
b) Müller Hinton-Agar (Oxoid, Wesel, Deutschland) Inhalt: s. Müller-Hinton-Bouillon und 13 g/l Agaragar

### Durchführung des Phototoxizitätstests:

200 µl einer Bakteriensuspension (Bakteriendichte: 10⁸ - 10¹² /ml) wurden mit je 200 µl verschiedener Konzentrationen der in Tabelle 1 aufgeführten Photosensibilisatoren bei 37° C bei Dunkelheit 15 Minuten inkubiert. Danach wurden die Bakterien zweimal mit PBS gewaschen, in 200 µl PBS resuspendiert, das gesamte Volumen auf eine 96-well Mikrotiterplatte übertragen und anschließend bestrahlt.

Zur Sensibilisierung wurde die Lampe UV 236 (Emission 380 - 480nm, mit einem Emissionsmaxima Eₘₐₓ: 418nm) von Waldmann Medizintechnik benutzt und die auf der Lichtquelle liegenden Platten wurden von unten bestrahlt, um eine Streuung durch die Flüssigkeit zu vermeiden. Die applizierte Lichtdosis betrug 12.3 J/cm². Als Kontrollen wurden bestrahlte, nicht bestrahlte und nur mit Photosensibilisator inkubierte Bakteriensuspensionen mitgeführt, die sowohl mit als auch ohne Photosensibilisator inkubiert wurden.

Anschließend wurde der KBE-Assay zur Bestimmung der koloniebildenden Einheiten pro ml (KBE) durchgeführt. Hierfür wurden serielle Verdünnungen von 10⁻¹ bis 10⁻⁹ der entsprechenden Bakteriensuspension hergestellt. 100 µl wurden dann auf Müller-Hinton Platten ausplattiert und bei 37° C für 24 h inkubiert. Danach wurde die Anzahl der überlebenden Kolonien durch Auszählen bestimmt und graphisch als koloniebildenden Einheiten (KBE) pro ml versus der jeweils verwendeten Photosensibilisatorkonzentration aufgetragen.

### Ergebnis der Phototoxizitätsexperimente:

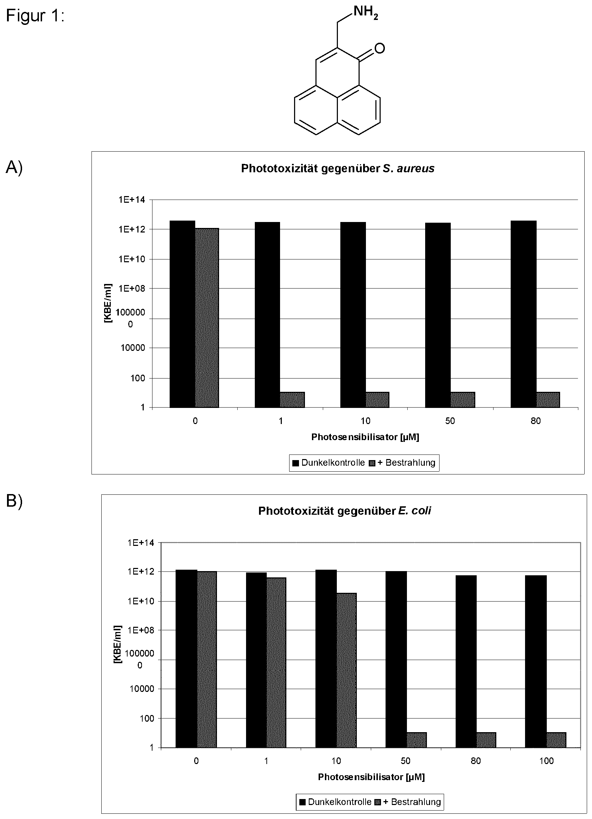

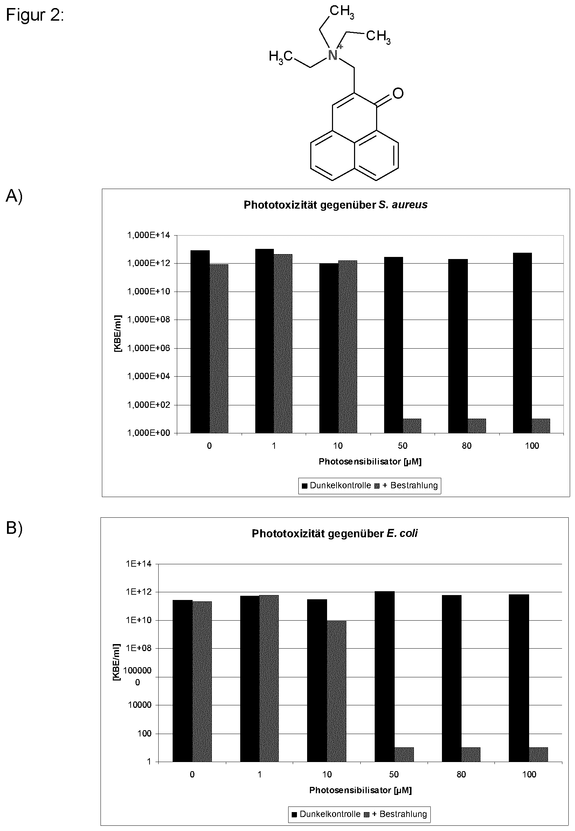

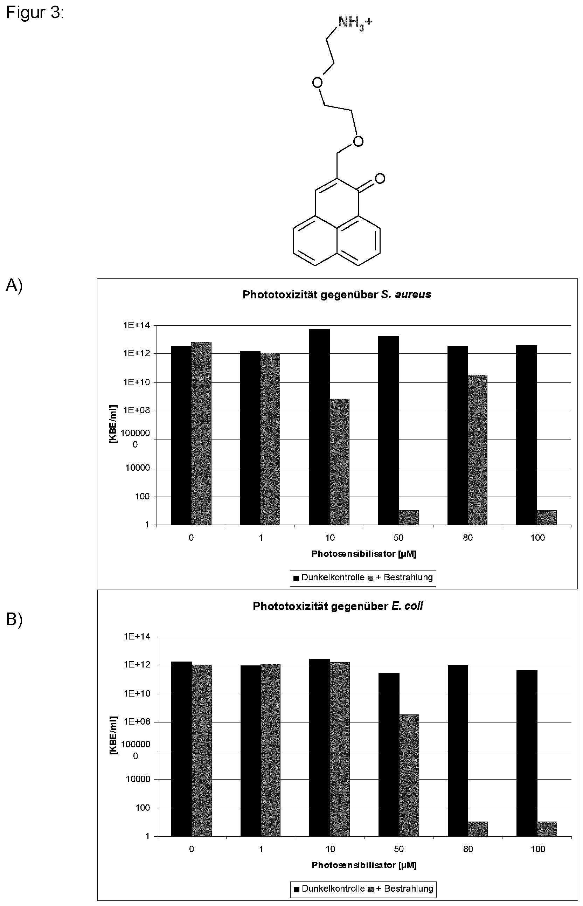

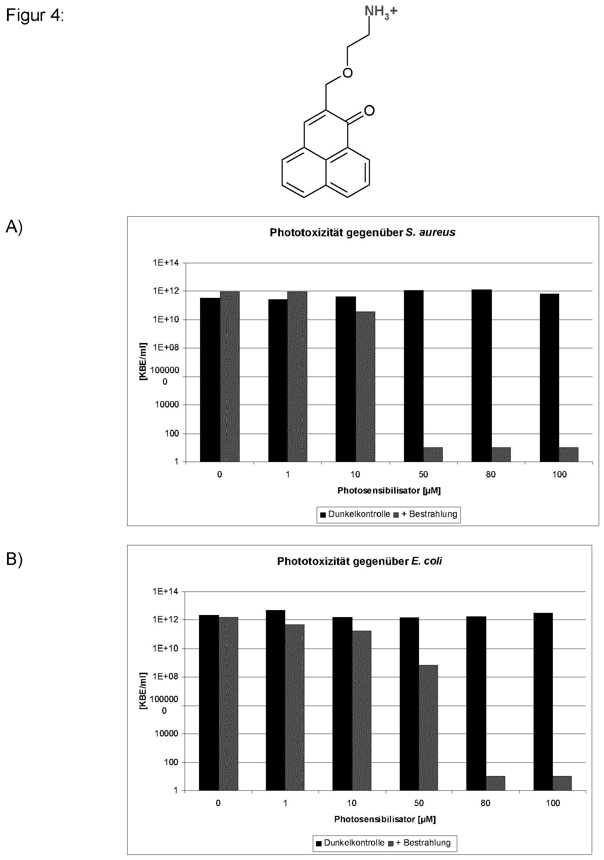

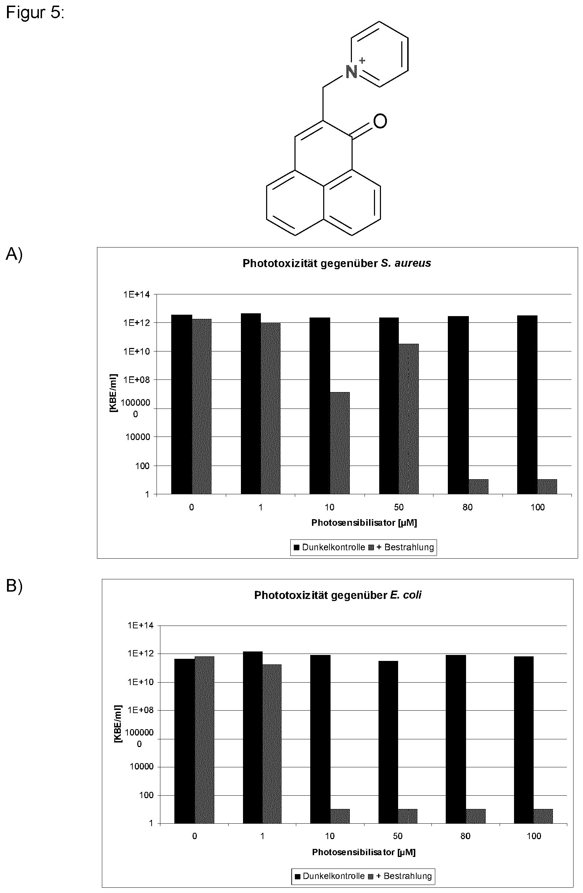

Die Ergebnisse der Phototoxizitätsexperimente sind in den Figuren 1 bis 5 dargestellt. Die Figuren 1 bis 5 zeigen die logarithmische Abnahmen der KBE/ml 24 Std nach Bestrahlung sowie die dazugehörigen Kontrollen (nur bestrahlte Bakterien; mit Photosensibilisator inkubierte Bakterien, aber nicht bestrahlt; unbehandelte Bakterien) für den jeweils verwendeten Photosensibilisator. Wie aus den Figuren 1 bis 5 ersichtlich, hat eine Bestrahlung der verwendeten Mikroorganismen *Staphylococcus aureus* (*S. aureus*) und *Escherichia coli* (*E. coli*) mit einer Lichtdosis von 12.3 J/cm² mit blauem Licht (Eₘₐₓ: 418 nm) für einen Zeitraum von 30 min in Abwesenheit eines Photosensibilisators keinen Einfluss auf die Anzahl der überlebenden Mikroorganismen im Vergleich zur unbelichteten Kontrolle (grauer Balken: 0 µM Photosensibilisator = Lichtkontrolle, nur bestrahlt vs. schwarzer Balken: 0 µM Photosensibilisator = Bakterien pur ohne Licht und ohne Photosensibilisator). Darüber hinaus zeigen die in den Figuren 1 bis 5 dargestellten Ergebnisse, dass die Inkubation (15 min) des jeweiligen Photosensibilisators mit den Mikroorganismen ohne nachfolgende Belichtung ebenfalls keinen Einfluss auf die Anzahl der überlebenden Mikroorganismen hat (schwarze Balken: 1 µM, 10 µM, 50 µM, 80 µM und 100 µM = Dunkelkontrolle, ohne Bestrahlung vs. schwarzer Balken: 0 µM = Bakterien ohne Photosensibilisator und ohne Bestrahlung).

Wie aus den Figuren 1 bis 5 ersichtlich erfolgt nach Inkubation (15 min) der Mikroorganismen in Abhängigkeit der verwendeten Konzentration der jeweiligen Photosensibilisatoren (1 µM, 10 µM, 50 µM, 80 µM und 100 µM) und nachfolgender Bestrahlung mit einer Lichtdosis von 12.3 J/cm² eine Abnahme der KBE/ml.

Die Effektivität der Phototoxizität gegenüber Bakterien nach Bestrahlung wurde nach folgenden Richtlinien für Handhygiene im Gesundheitswesen festgelegt (Boyce, J. M., and D. Pittet. 2002. Guideline for Hand Hygiene in Health-Care Settings: recommendations of the Healthcare Infection Control Practices Advisory Committee and the HICPAC/SHEA/APIC/IDSA Hand Hygiene Task Force. Infect. Control. Hosp. Epidemiol. 23, Seite 3 - 40):
- Reduktion der KBE/ml um 1 log₁₀-Stufe ≙ 90% Effektivität
- Reduktion der KBE/ml um 3 log₁₀-Stufen ≙ 99,9% Effektivität
- Reduktion der KBE/ml um 5 log₁₀-Stufen ≙ 99,999% Effektivität.

Um beispielhaft zur hygienischen Händedesinfektion zugelassen zu werden, muss ein Prüfprodukt zu einer Reduktion der KBE/ml um mindestens 5 log₁₀-Stufen führen (Gebel, J. 2002. Anforderungskatalog für die Aufnahme von chemischen Desinfektionsverfahren in die Desinfektionsmittel-Liste der DGHM. Desinfektionsmittel-Kommission der DGHM, Seiten 5 - 22.).

Als effektive Inaktivierung kann daher die Abnahme von ≥ 3 log₁₀-Stufen angenommen werden, wobei *S. aureus* und *E. coli* als Beispiele für Vertreter aus der Gruppe der Gram-positiven und Gram-negativen Bakterien ausgewählt wurden.
Die benötigte Konzentration, um jeweils eine Reduktion um ≥3log₁₀ Stufen zu erzielen, ist in Tabelle 1 dargestellt.

Wie aus Tabelle 1 ersichtlich ist, weisen die in den Beispielen 2), 4b), 5b), 7) und 10) synthetisierten Substanzen gegenüber der Ausgangssubstanz Perinaphthenon eine verbesserte antimikrobielle Wirkung auf, da eine niedrigere Konzentration dieser Verbindungen benötigt wird, um eine Reduktion der Keimzahl um 3 log₁₀ Stufen zu erreichen.

Im Vergleich zur Ausgangssubstanz Perinaphthenon wird eine um etwa 10 fach geringere Konzentration von den in den Beispielen 2), 4b), 5b), 7) und 10) synthetisierten Substanzen benötigt, um eine Reduktion um 3log₁₀ Stufen zu erreichen.

Eine Reduktion um 3 log₁₀ Stufen entspricht jeweils einer Reduktion der koloniebildenden Einheiten um 99.9%.

**Tabelle 1: Benötigte Konzentration, um eine Reduktion um ≥ 3 log₁₀ Stufen zu erzielen,**

| | **Photosensibilisator** | **Benötigte Konzentration, um eine Reduktion um ≥ 3log₁₀ Stufen zu erzielen,** |
|---|---|---|
| Vergleich | 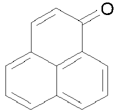 | 200 µM für *S. aureus;* 300 µM für *E. coli* |
| Beispiel 2) | 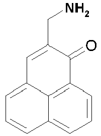 | 1 µM für *S. aureus;* 50 µM für *E. coli* |
| Beispiel 10) | 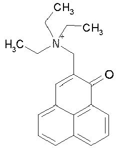 | 50 µM für *S. aureus;* 50 µM für *E. coli* |
| Beispiel 5b) | 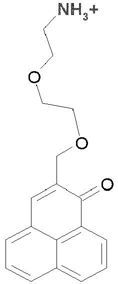 | 50 µM für *S. aureus;* 80 µM für *E. coli* |
| Beispiel 4b) | 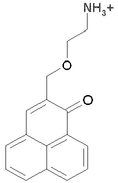 | 50 µM für *S. aureus;* 300µM für *E. coli* |
| Beispiel 7) | 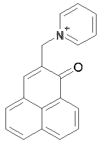 | 80 µM für *S. aureus;* 10 µM für *E. coli* |

### Beispiel 21: Verwendung verschiedener 1H-Phenalen-1-on-Derivate zur Oberflächendesinfektion von Katheterschläuchen.

5 cm lange Katheterstücke wurden mit 1 ml einer stabilen S. aureus -Lösung (1 E+12 cfu/ml) befüllt und über Nacht bei 37°C inkubiert. Nach dem Eintrocknen der Lösung wurden jeweils 2 ml einer Lösung von PBS, das eine entsprechende Konzentrationen (0, 1, 10, 50, 80 und 100 µM) der in den Beispielen 2), 4b), 5b), 7) und 10) hergestellten Verbindungen und 1H-Phenalen-1-on enthielt, besprüht. Nach einer kurzen Einwirkzeit von 5 Minuten in Dunkelheit wurden die Katheterstücke mit einer Lichtdosis von 12.3 J/cm² mit blauem Licht (Eₘₐₓ: 418nm) für einen Zeitraum von 30 min belichtet.

Als Kontrolle wurde jeweils unbelichtete Katheterstücke verwendet, auf die die entsprechende Konzentrationen (0, 1, 10, 50, 80 und 100 µM) der in Beispiel 1b) - f) hergestellten Verbindungen und 1H-Phenalen-1-on aufgebracht wurde.

Zur Bestimmung der Zellzahl wurden die belichteten und unbelichteten Katheterstücke mit 1 ml PBS befüllt und ultrageschallt. Die Waschlösung wurde in verschiedenen Verdünnungen (10⁰ bis 10⁷) mit Wachstumsmedium versetzt und auf Agarplatten ausgestrichen. Die Zellzahl wurde nach 24h Inkubation bei 37°C ausgezählt.

Im Vergleich zur Ausgangssubstanz Perinaphthenon wird eine um etwa 10 fach geringere Konzentration von den in den Beispielen 2), 4b), 5b), 7) und 10) synthetisieren Substanzen benötigt, um eine Reduktion um 3log₁₀ Stufen zu erreichen.

## Patentansprüche

1. Verbindung mit der Formel (1): 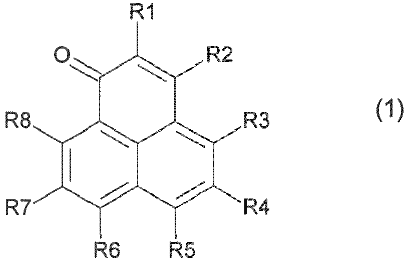 wobei R2 bis R8 Wasserstoff ist und wobei R1 den Rest -(CH₂)ₖ-X bedeutet, wobei k eine ganze Zahl von 1 bis 4 ist und wobei X ein organischer Rest, der a) wenigstens ein neutrales, protonierbares Stickstoffatom und/oder b) wenigstens ein positiv geladenes Stickstoffatom enthält, ist.

2. Verbindung nach Anspruch 1, wobei X einen Rest der Formel (2): 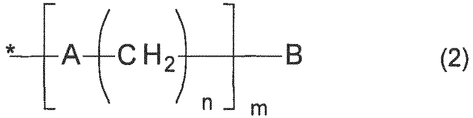 bedeutet und wobei A ein Sauerstoff- oder ein Schwefelatom oder ein Stickstoffatom ist und wobei n eine ganze Zahl von 1 bis 3 und m eine ganze Zahl von 0 bis 10 ist und wobei B aus der Gruppe, die aus den Resten der Formel (3), (4) und (5): 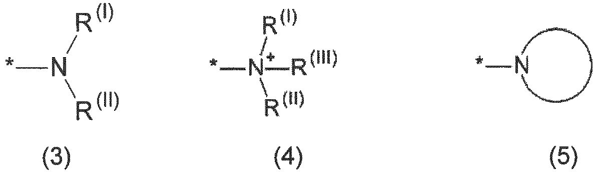 besteht, ausgewählt wird und wobei jeder der Reste R^{(I)}, R^{(II)} und R^{(III)} unabhängig voneinander ausgewählt wird aus Wasserstoff oder C1 - C20 Alkyl, das geradkettig oder verzweigt sein kann, und wobei der Rest mit der Formel (5) 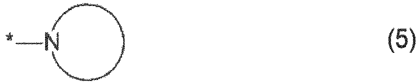 einen substituierten oder unsubstituierten heterocyclischen Rest mit 5 bis 7 Ringatomen, die mindestens ein 1 Kohlenstoffatom und 1 bis 4 Stickstoffatome sowie optional 1 oder 2 Sauerstoff- oder Schwefelatom umfassen, wobei der heterocyclische Rest gesättigt oder ungesättigt ist, darstellt.

3. Verbindung nach Anspruch 1 oder 2, wobei X jeweils aus der Gruppe, die aus den Resten der Formel (10) bis (33): 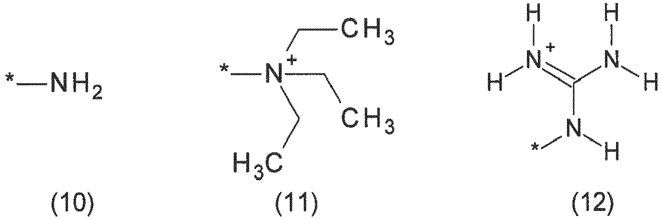 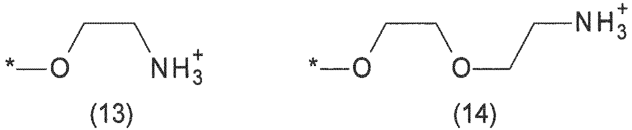 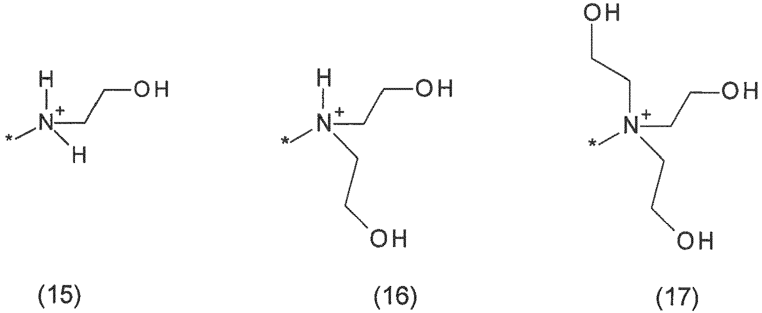 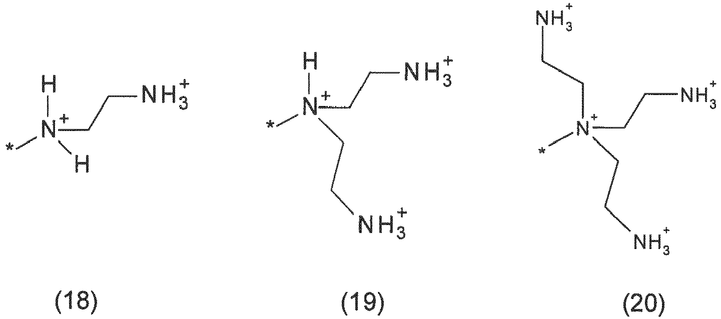 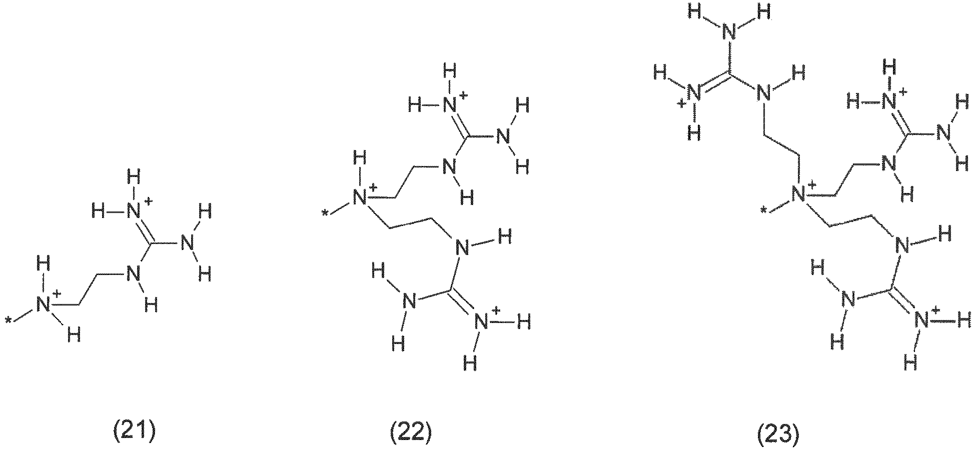 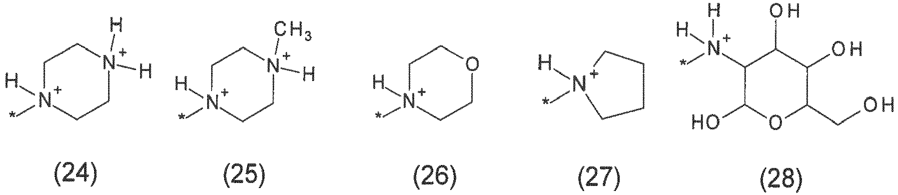 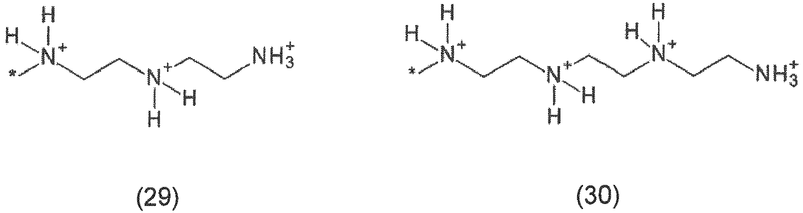 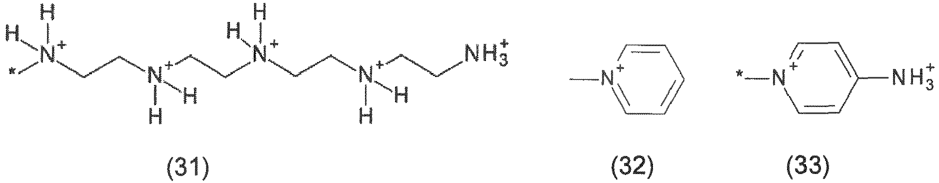 besteht, ausgewählt wird.

4. Verbindung nach einem der Ansprüche 1 bis 3zur Verwendung als Photosensibilisator, vorzugsweise bei der photodynamischen Therapie.

5. Verbindung nach einem der Ansprüche 1 bis 4 zur Verwendung bei der Inaktivierung von Mikroorganismen, die vorzugsweise aus der Gruppe, die aus Viren, Archäeen, Bakterien, Bakteriensporen, Pilzen, Pilzsporen, Protozoen, Algen und blutübertragbaren Parasiten besteht, ausgewählt werden.

6. Verbindung nach einem der Ansprüche 1 bis 5 zur Verwendung bei der Reinigung von Zähnen, Zahnersatz und/oder Zahnspangen und/oder Prophylaxe und/oder Behandlung einer Erkrankung des Zahngewebes und/oder des Zahnhalteapparates.

7. Verbindung nach einem der Ansprüche 1 bis 4, zur Verwendung bei der Prophylaxe und/oder Behandlung einer infektiösen Hautkrankheit

8. Verbindung nach einem der Ansprüche 1 bis 4, zur Verwendung bei der Oberflächenreinigung und/oder -beschichtung.

9. Verbindung nach Anspruch 8, zur Verwendung bei der Oberflächenreinigung und/oder -beschichtung von Medizinprodukten, Lebensmittelverpackungen oder Hygieneartikeln.

10. Pharmazeutische Zusammensetzung enthaltend wenigstens eine Verbindung nach einem der Ansprüche 1 bis 3oder einem pharmakologisch verträglichen Salz davon.

11. Beschichteter Gegenstand **dadurch gekennzeichnet dass** die Oberfläche des Gegenstandes wenigstens eine Verbindung nach einem der Ansprüche 1 bis 3 aufweist.

12. Verfahren zur Herstellung einer Verbindung nach Anspruch 1, wobei das Verfahren folgende Schritte umfasst:
(A1) Umsetzen von 1H-Phenalen-1-on mit wenigstens einem Alkylierungsagens der Formel Y-(CH₂)ₕ-Z, wobei Y aus der Gruppe, die aus H, Cl, Br, I, p-Toluolsulfonyl (OTs), Methansulfonyl (OMs), OH und Alkyl₂S⁺ besteht, wobei Alkyl gleich oder unabhängig voneinander verschieden sein kann und vorzugsweise Methyl, Ethyl, Propyl oder Butyl bedeutet, ausgewählt wird, optional in Gegenwart eines Katalysators, unter Erhalt einer Verbindung mit der Formel (58): 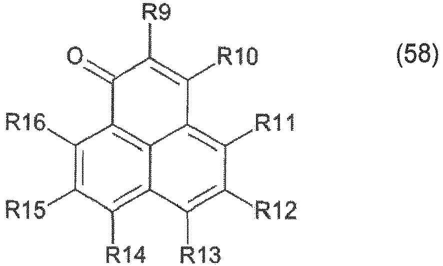 wobei R10 bis R16 jeweils Wasserstoff bedeuten und wobei R9 den Rest -(CH₂)ₕ-Z, wobei h eine ganze Zahl von 1 bis 4 bedeutet und wobei Z aus der Gruppe, die aus Cl, Br, I, p-Toluolsulfonyl (OTs), Methansulfonyl (OMs), OH und Alkyl₂S⁺ besteht, wobei Alkyl gleich oder unabhängig voneinander verschieden sein kann und vorzugsweise Methyl, Ethyl, Propyl oder Butyl bedeutet, ausgewählt wird, bedeutet, oder
(A2) Umsetzen von 1H-Phenalen-1-on mit Formaldehyd und wenigstens einer Halogenwasserstoffsäure, optional in Gegenwart eines Katalysators, unter Erhalt einer Verbindung mit der Formel (58): 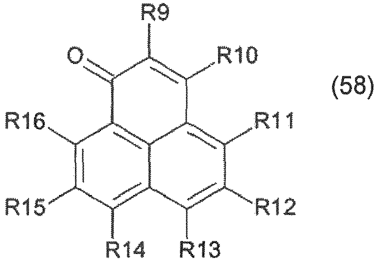 wobei R10 bis R16 jeweils Wasserstoff bedeuten und wobei R9 den Rest -CH₂-W bedeutet und wobei W aus der Gruppe, die aus Cl, Br und I besteht, ausgewählt wird,
(B) Umsetzen der in Schritt (A1) oder (A2) erhaltenen Verbindung mit der Formel (58) mit einer organischen Verbindung, die a) wenigstens ein neutrales, protonierbares Stickstoffatom und/oder b) wenigstens ein positiv geladenes Stickstoffatom enthält, optional in Gegenwart einer Base, und optional
(C) Entfernen vorhandener Aminoschutzgruppen unter Erhalt des erfindungsgemäßen 1H-Phenalen-1-on-Derivats der Formel (1).

## Claims

1. Compound having formula (1): 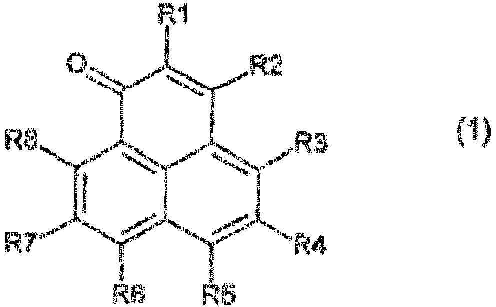 wherein R2 to R8 is hydrogen and wherein R1 denotes the -(CH₂)ₖ-X radical, wherein k is an integer from 1 to 4 and wherein X is an organic radical that contains a) at least one neutral, protonatable nitrogen atom and/or b) at least one positively charged nitrogen atom.

2. Compound according to claim 1, wherein X denotes a radical of formula (2): 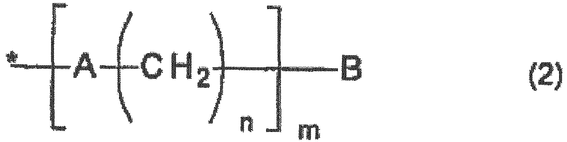 and wherein A is an oxygen or sulphur atom or a nitrogen atom, and wherein n is an integer from 1 to 3 and m is an integer from 0 to 10, and wherein B is selected from the group consisting of the radicals of formulae (3), (4) and (5): 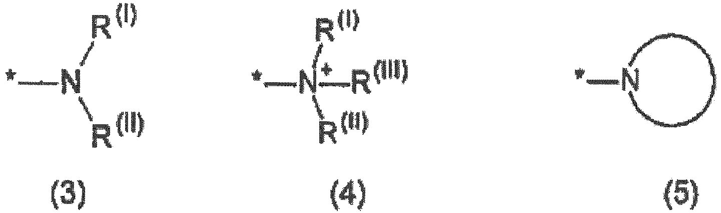 and wherein each of radicals R^{(I)}, R^{(II)} and R^{(III)} are selected independently from one another from hydrogen or C1-C20 alkyl, which can be linear or branched, and wherein the radical having formula (5) 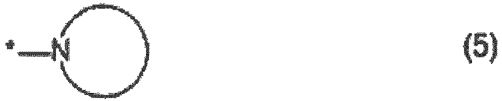 represents a substituted or unsubstituted, heterocyclic radical having 5 to 7 ring atoms that comprise at least one carbon atom and 1 to 4 nitrogen atoms, and optionally 1 or 2 oxygen or sulphur atoms, wherein the heterocyclic radical is saturated or unsaturated.

3. Compound according to claim 1 or 2, wherein X is selected respectively from the group consisting of the radicals of formulae (10) to (33):

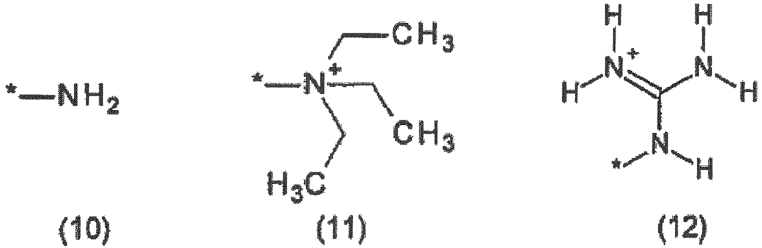

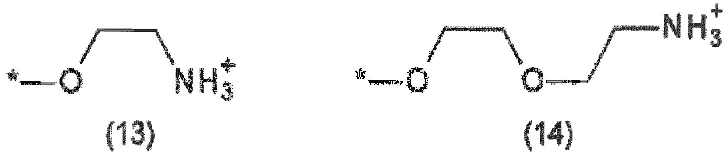

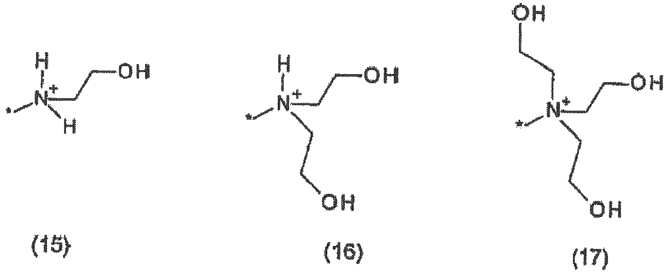

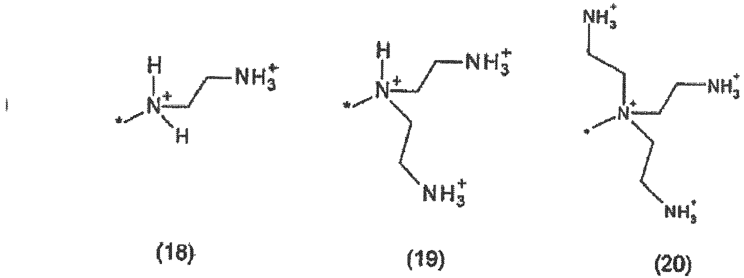

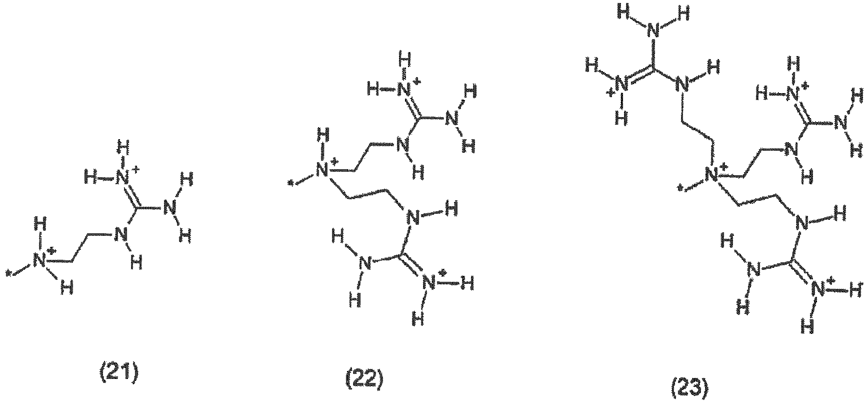

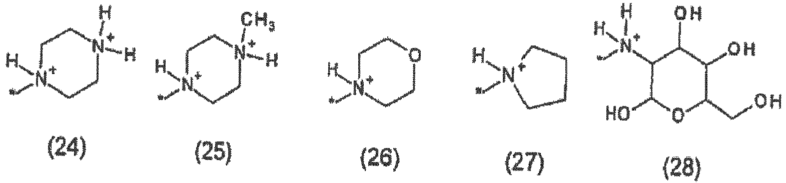

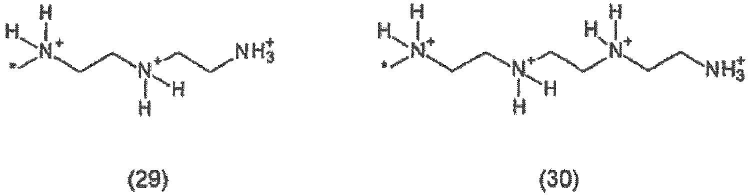

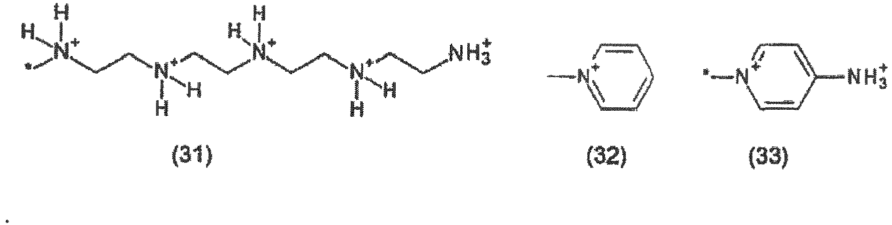

4. Compound according to one of claims 1 to 3 for use as a photosensitiser, preferably in photodynamic therapy.

5. Compound according to one of claims 1 to 4 for use during deactivation of microorganisms that are preferably selected from the group consisting of viruses, archaea, bacteria, bacterial spores, fungi, fungal spores, protozoa, algae and blood-borne parasites.

6. Compound according to one of claims 1 to 5 for use during cleaning of teeth, false teeth and/or braces and/or prophylaxis and/or treatment of a dental tissue disease and/or periodontal disease.

7. Compound according to one of claims 1 to 4 for use during prophylaxis and/or treatment of an infectious skin disease.

8. Compound according to one of claims 1 to 4 for use in surface cleaning and/or coating.

9. Compound according to claim 8 for use in surface cleaning and/or coating of medical products, food packaging or sanitary products.

10. Pharmaceutical composition containing at least one compound according to one of claims 1 to 3 or a pharmaceutically acceptable salt thereof.

11. Coated object, **characterised in that** the surface of the object has at least one compound according to one of claims 1 to 3.

12. Method for the production of a compound according to claim 1, wherein the method comprises the following steps:
(A1) Reacting 1H-phenalen-1-one with at least one alkylation agent of formula Y-(CH₂)ₕ-Z, wherein Y is selected from the group consisting of H, Cl, Br, I, p-toluenesulphonyl (OTs), methanesulphonyl (OMs), OH and alkyl²S⁺, wherein alkyl can be the same as or independently different from one another, and preferably denotes methyl, ethyl, propyl or butyl, optionally in the presence of a catalyst, to obtain a compound having formula (58): 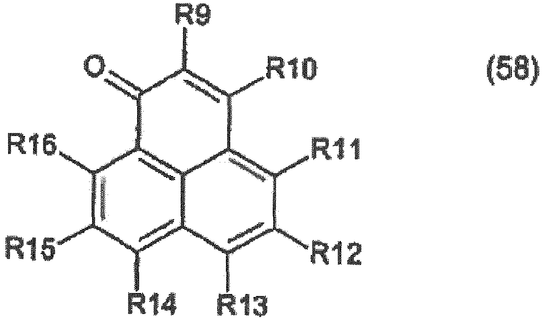 wherein R10 to R16 each denote hydrogen and wherein R9 denotes the radical -(CH₂)ₕ-Z, wherein h denotes an integer from 1 to 4, and wherein Z is selected from the group consisting of Cl, Br, I, p-toluenesulphonyl (OTs), methanesulphonyl (OMs), OH and alkyl²S⁺, wherein alkyl can be the same as or independently different from one another and preferably denotes methyl, ethyl, propyl or butyl, or
(A2) Reacting 1H-phenalen-1-one with formaldehyde and at least one hydrohalic acid, optionally in the presence of a catalyst, to obtain a compound having formula (58): 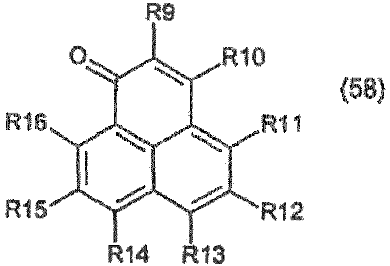 wherein R10 to R16 each denote hydrogen, and wherein R9 denotes the radical -CH₂-W, and wherein W is selected from the group consisting of Cl, Br and I,
(B) Reacting the compound having formula (58) obtained in step (A1) or (A2) with an organic compound containing a) at least one neutral, protonatable nitrogen atom and/or b) at least one positively charged nitrogen atom, optionally in the presence of a base, and optionally
(C) Removing any amino protecting groups present in order to obtain the 1H-phenalen-1-one derivative of formula (1) according to the invention.

## Revendications

1. Composé de formule (1) : 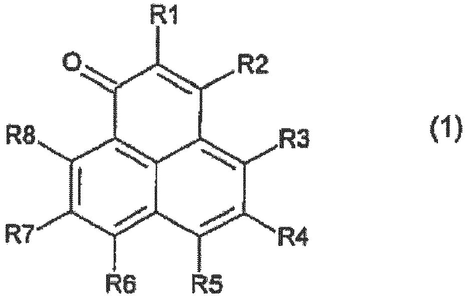 dans laquelle R2 à R8 représentent des atomes d'hydrogène et dans lequel R1 représente le radical -(CH₂)ₖ-X, dans lequel k est un nombre entier de 1 à 4 et dans lequel X est un radical organique, qui contient a) au moins un atome d'azote protonable neutre et/ou b) au moins un atome d'azote positivement chargé.

2. Composé selon la revendication 1, dans lequel X représente un radical de formule (2) : 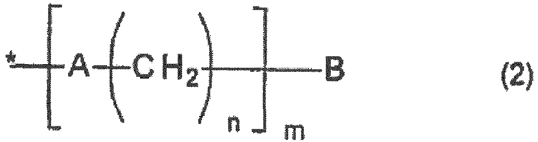 et dans lequel A représente un atome d'oxygène ou un atome de soufre, ou un atome d'azote, et dans lequel n est un nombre entier de 1 à 3, et m est un nombre entier de 0 à 10, et dans lequel B est choisi dans un groupe constitué des radicaux de formules (3), (4) et (5) : 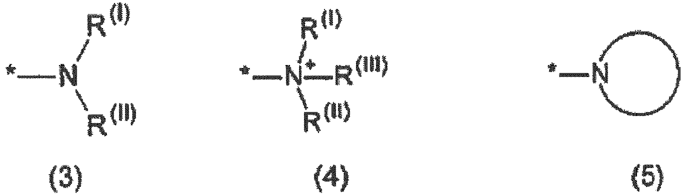 et dans lequel chacun des radicaux R^{(I)}, R^{(II)} et R^{(III)} est choisi indépendamment des autres parmi l'hydrogène ou un radical alkyle en C1-C20 qui peut être linéaire ou ramifié, et le radical de formule (5) 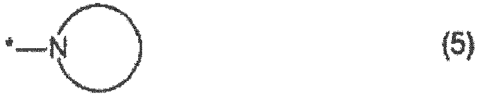 représentant un radical hétérocyclique substitué ou non-substitué ayant 5 à 7 atomes nucléaires, qui comprennent au moins un atome de carbone et 1 à 4 atomes d'azote ainsi qu'en option 1 ou 2 atomes d'oxygène ou de soufre, dans lequel le radical hétérocyclique est saturé ou insaturé.

3. Composé selon la revendication 1 ou 2, dans lequel chaque X est choisi dans le groupe qui est constitué des radicaux de formules (10) à (33) :

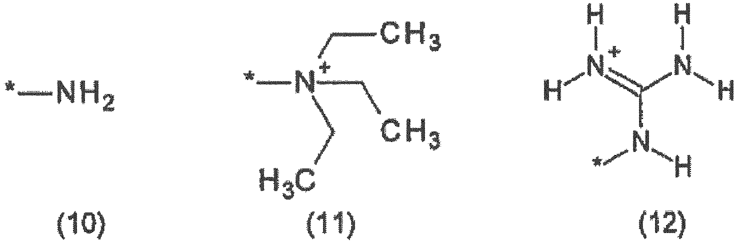

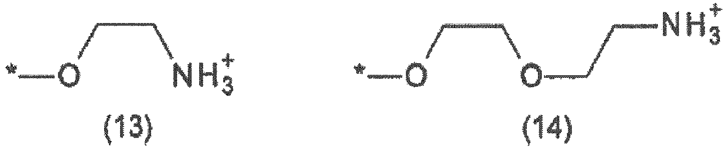

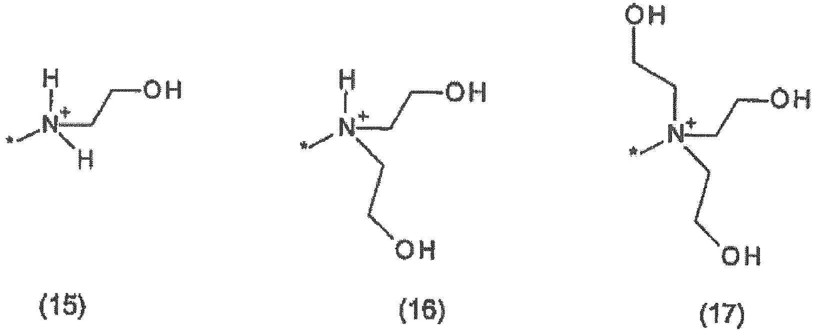

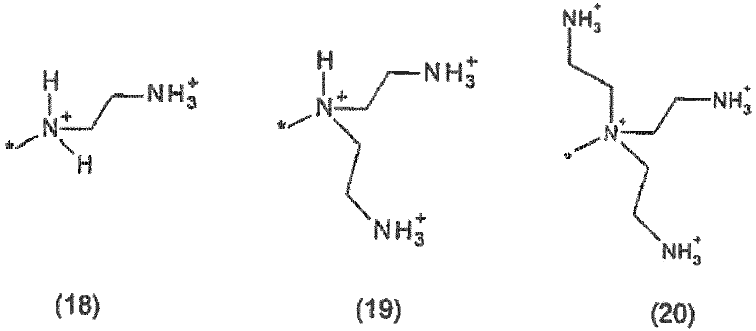

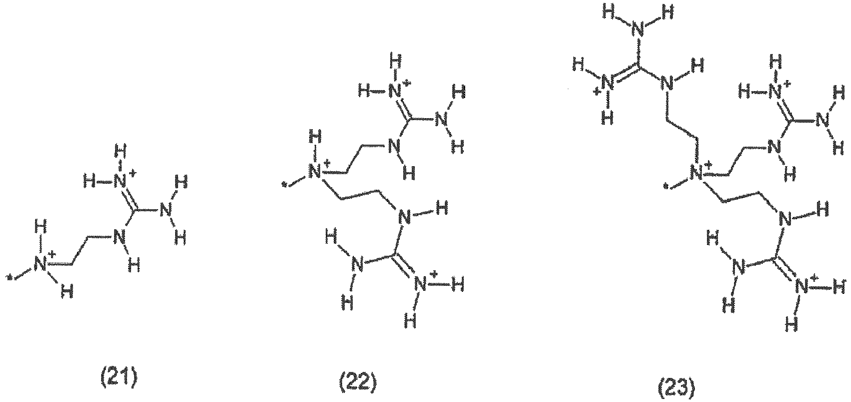

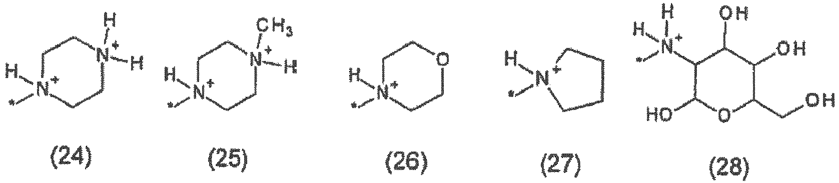

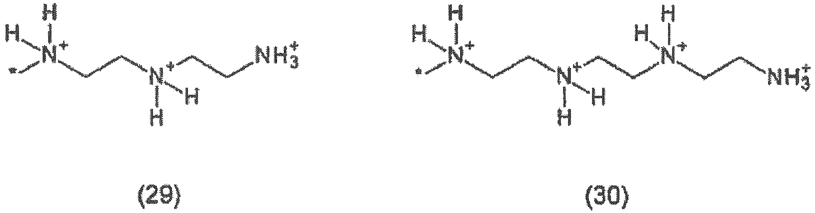

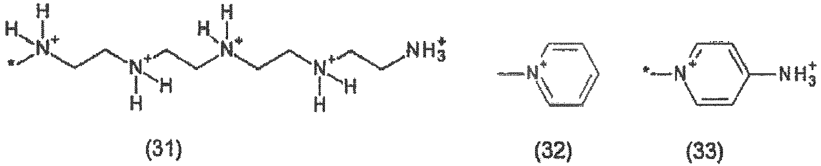

4. Composé selon l'une des revendications 1 à 3, pour une utilisation en tant que photosensibilisant, de préférence dans le cadre d'une thérapie photodynamique.

5. Composé selon l'une des revendications 1 à 4, pour une utilisation lors de l'inactivation de microorganismes, qui de préférence sont choisis dans le groupe constitué des virus, des archées, des bactéries, des spores bactériennes, des champignons, des spores fongiques, des protozoaires, des algues et des parasites transmissibles par le sang.

6. Composé selon l'une des revendications 1 à 5, pour une utilisation lors du nettoyage des dents, d'une prothèse dentaire et/ou d'appareils d'orthodontie et/ou lors de la prophylaxie et/ou du traitement d'une maladie du tissu dentaire et/ou de l'appareil de soutien des dents.

7. Composé selon l'une des revendications 1 à 4, pour une utilisation lors de la prophylaxie et/ou du traitement d'une maladie cutanée infectieuse.

8. Composé selon l'une des revendications 1 à 4, pour une utilisation lors du nettoyage et/ou du revêtement de surfaces.

9. Composé selon la revendication 8, pour une utilisation lors du nettoyage et/ou du revêtement de la surface de produits médicaux, d'emballages pour produits alimentaires ou d'articles d'hygiène.

10. Composition pharmaceutique contenant au moins un composé selon l'une des revendications 1 à 3 ou un sel pharmacologiquement compatible de ce dernier.

11. Objet revêtu, **caractérisé en ce que** la surface de l'objet présente au moins un composé selon l'une des revendications 1 à 3.

12. Procédé de préparation d'un composé selon la revendication 1, dans lequel le procédé comprend les étapes suivantes :
(A1) réaction de 1H-phénalèn-1-one avec au moins un agent d'alkylation de formule Y-(CH₂)ₕ-Z, Y étant choisi dans le groupe constitué des radicaux H, Cl, Br, I, p-toluènesulfonyle (OTs), méthanesulfonyle (OMs), OH et alkyle₂S⁺, les radicaux alkyle pouvant être identiques l'un à l'autre ou d'une manière indépendante différents l'un de l'autre, et représentant de préférence chacun un groupe méthyle, éthyle, propyle ou butyle, en option en présence d'un catalyseur, avec obtention d'un composé de formule (58) 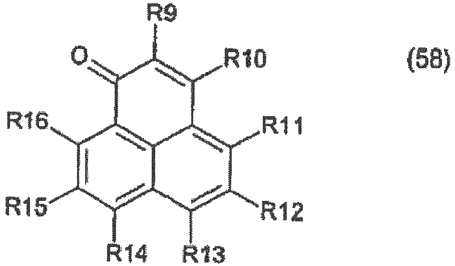 dans laquelle R10 à R16 représentent chacun un atome d'hydrogène et R9 représente le radical -(CH₂)ₕ-Z, h étant un nombre entier de 1 à 4 et Z étant choisi dans le groupe qui est constitué des radicaux Cl, Br, I, p-toluènesulfonyle (OTs), méthanesulfonyle (OMs), OH et alkyle₂S⁺, les radicaux alkyle pouvant être identiques l'un à l'autre ou d'une manière indépendante différents l'un de l'autre, et représentant chacun de préférence un groupe méthyle, éthyle, propyle ou butyle, ou
(A2) réaction de 1H-phénalèn-1-one avec du formaldéhyde et au moins un acide halogènhydrique, en option en présence d'un catalyseur, avec obtention d'un composé de formule (58) : 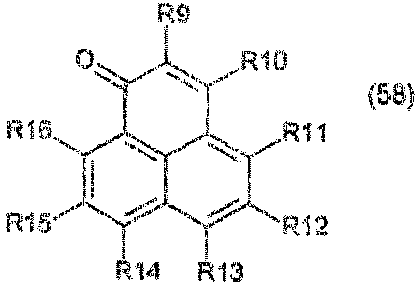 dans laquelle R10 à R16 représentent chacun un atome d'hydrogène, et R9 représente le radical -CH₂-W, W étant choisi dans le groupe qui est constitué de Cl, Br et I,
(B) réaction du composé obtenu dans l'étape (A1) ou (A2), de formule (58), avec un composé organique qui contient a) au moins un atome d'azote protonable neutre et/ou b) au moins un atome d'azote positivement chargé, en option en présence d'une base, et en option
(C) élimination des groupes protecteurs amino présents, avec obtention du dérivé de 1H-phénalèn-1-one de formule (1) selon l'invention.
